# EUROPEAN PATENT APPLICATION

(11) **EP 3 868 760 A1**
(43) Date of publication of application: **25.08.2021**
(21) Application number: 20158619.5
(22) Date of filing: 20.02.2020
(51) Int. Cl.: C07D 471/04, A61P 35/00, A61K 31/437

(54) **2,3,5-SUBSTITUTED PYRROLO[2,3-B]PYRIDINES AS ERBB MODULATORS USEFUL FOR TREATING CANCER**

(71) Applicant: PearlRiver Bio GmbH, 44227 Dortmund (DE)
(72) Inventor: The designation of the inventor has not yet been filed
(74) Representative: Arth, Hans-Lothar

(57) **Abstract**

The present invention relates to 2,3,5-substituted 1*H*-pyrrolo[2,3-*b*]pyridine compounds of general formula (I) with aromatic 5-membered heterocyclic substituents at position 5 as well as pharmaceutically acceptable salts thereof.

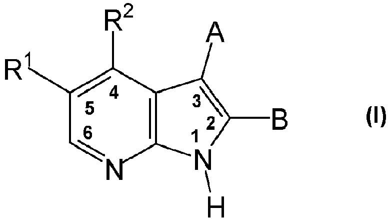

These compounds are used for the treatment or prevention of a disease or medical condition mediated through certain mutated forms of ErbB receptor, especially of Exon20 Her2 and EGFR mutations.

## Description

### Field of the Invention

The present invention relates to 2,3,5-substituted 1*H*-pyrrolo[2,3-*b*]pyridine compounds of general formula (I) with aromatic 5-membered heterocyclic substituents at position 5 as well as pharmaceutically acceptable salts thereof.

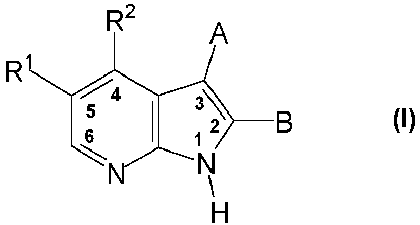

These compounds are used for the treatment or prevention of a disease or medical condition mediated through certain mutated forms of ErbB receptor, especially of Exon20 Her2 and EGFR mutations.

### Background of the Invention

Receptor tyrosine kinases (RTKs) are cell surface receptors that transmit signals from the extracellular environment to control growth, differentiation and survival of cells. Deregulated expression of protein kinases by gene deletion, -mutation or -amplification has been found to be important for tumor initiation and -progression, involving cancer cell proliferation, -survival, -motility and -invasivity as well tumor angiogenesis and chemotherapy resistance. Because of the advanced understanding of their critical role, protein kinases are important targets for novel therapies, especially for cancer.

In humans the receptor tyrosine kinase family ErbB comprises four members: EGFR (Her1), ErbB2 (Her2), ErbB3 (Her3) and ErbB4 (Her4). The binding of a ligand induces conformational change in receptors to form homo- and heterodimerization. The extracellular domain of Her2 is already fixed in a conformation without ligand binding that resembles the other ligand-activated ErbB members and hereby acts as a preferred dimerization partner for other ligand-bound ErbBs. The dimerization of receptors activates the intrinsic kinase activity and hereby yielding to the phosphorylation of its substrates, resulting in activation of multiple downstream pathways within the cell, including the anti-apoptotic/survival PI3K-AKT-mTOR and the mitogenic RAS-RAF-MEK-ERK-MAPK pathways (Chong et al. Nature Med. 2013; 19 (11):1389-1400). There is strong precedent for the involvement of ErbB kinase family in human cancer as overexpression and/or mutations of ErbB is commonly found in cancers (for example breast, lung, head, neck and bladder). First generation small molecule EGFR inhibitors like Tarceva (Erlotinib) and Iressa (Gefitinib), both binding reversibly to EGFR, are currently first-line therapy for non-small cell lung cancer patients with tumors harbouring EGFR mutations in exon 19 and 21 (like L858R and delE746-A750). Second and third generation small molecule EGFR inhibitors have been designed as irreversible EGFR inhibitors. These compounds (for example Afatinib, HKI-272, CI-1033, EKB-569, WZ-4002, AZ9291, CO-1686) bind irreversibly to EGFR, preferably to cysteine 797.

Approximately 10% of patients with NSCLC in the United States are reported to have tumor-associated EGFR mutations (Lynch et al. N Engl J Med. 2004, 350 (21): 2129-39). The EGFR mutations mostly occur within EGFR Exon 18-21 (Yasuda et al. Sci Transl Med. 2013, 18, 5(216): 216ra177; Leduc et al. Annals of Oncology 2017, 28, 11: 2715-2724; Arcila et al. Mol Cancer Ther. 2013, 12 (2): 220-229). These mutations increase the kinase activity of EGFR, leading to hyperactivation of downstream pro-survival signalling pathways (Pao et al. Nat Rev Cancer 2010, 10: 760-774). EGFR Exon 20 insertions reportedly comprise approximately 4-9.2 % of all EGFR mutant lung tumors (Arcila et al. Mol Cancer Ther. 2013, 12 (2): 220-9; Oxnard et al. J Thorac Oncol. 2013, 8(2): 179-184; Yasuda et al. Lancet Oncol. 2012, 13 (1): e23-31). Most EGFR Exon 20 insertions occur in the region encoding amino acids 767 through 774 of exon 20 within the loop that follows the C-helix of the kinase donmain of EGFR. Analysis of patients with tumors harbouring EGFR Exon 20 insertion mutations mostly displayed progressive disease in the course of treatment with Gefitinib, Erlotinib or Afatinib (Yasuda et al. Lancet Oncol. 2012, 13 (1): e23-31; Yasuda et al. Sci Transl Med. 2013, 18, 5(216): 216ra177).

Her2 mutations are reportedly present in ∼2-4% of NSCLC (Buttitta et al. Int J Cancer. 2006, 119: 2586-2591). The most common mutation is an in-frame insertion within Exon 20. In 83% of patients having Her2 associated NSCLC, a four amino acid YVMA insertion mutation occurs at codon 775 in Exon 20 of Her2 (Arcila et al. Clin Cancer Res 2012, 18: 4910-4918). The Her2 Exon 20 insertion results in increased kinase activity and enhanced signalling through downstream pathways, resulting in increased survival, invasiveness, and tumorgenicity (Wang et al. Cancer Cell 2006; 10: 25-38). Tumors harbouring the Her2 YVMA mutation are largely resistant to known EGFR inhibitors (Arcila et al. Clin Cancer Res 2012, 18: 4910-4918).

It is the objective of the present invention to provide compounds and pharmaceutic compositions comprising these compounds as mutant-selective ErbB inhibitors, especially for the Exon 20 EGFR/Her2 mutations, which can be used as pharmaceutically active agents, especially for prophylaxis and/or treatment of cell proliferative diseases such as cancer.

The present invention provides novel 2,3,5-substituted pyrrolo[2,3-*b*]pyridines being mutant-selective ErbB inhibitors, especially for the Exon 20 EGFR/Her2 mutations, and additionally being inhibitors for other mutants like EGFR T790ML858R mutation.

Thus, the objective of the present invention is solved by the teachings of the independent claims. Further advantageous features, aspects and details of the invention are evident from the dependent claims, the description, the figures, and the examples of the present application.

### Description of the invention

The present invention is directed to compounds of the general formula (I) 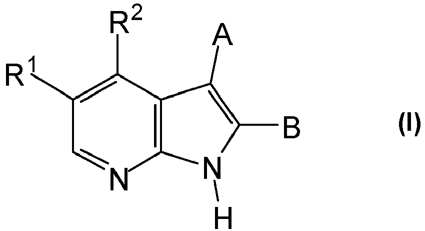 wherein
**A** represents 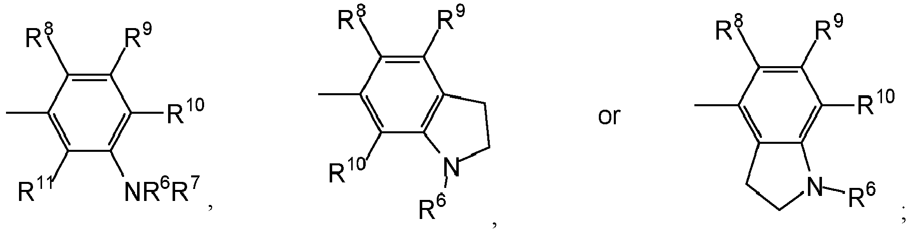
**B** represents 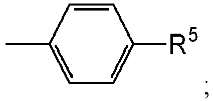
**R¹** represents 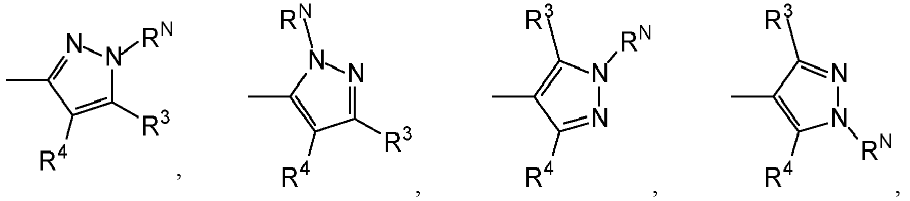 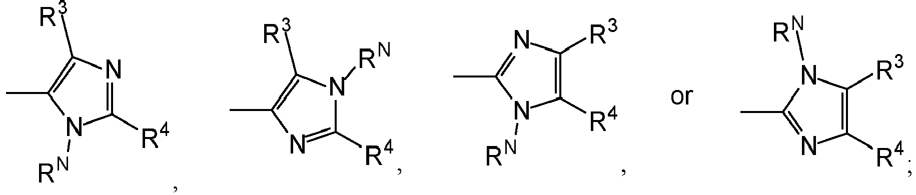
**R²** represents -H, -F, -CI, -Br, -CN, -CH₃, -C₂H₅, -C₃H₇, -OCH₃, -OC₂H₅, or -OC₃H₇;
**R³** and **R⁴** represent independently of each other -H, -F, -CI, -CN, -CF₃, -OCH₃, -OC₂H₅, -CH₂OCH₃, -CH₃, -C₂H₅, or -C₃H₇;
**R⁵** represents 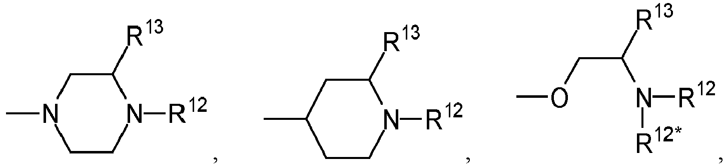 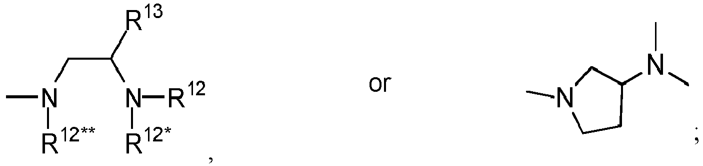
**R^{N}** represent -H, -CH₃, -CF₃, -C₂H₅, -C₃H₇, -CH(CH₃)₂, -CH₂CH(CH₃)₂, -C₄H₉, -cyclo-C₃H₅, -cyclo-C₄H₇, -CH₂CH₂OH, -CH₂CH(CH₃)OH, -CH₂CH₂OCH₃, -CH₂CH₂CH₂OH, -CH₂CH₂CH₂OCH₃, or -CH₂CH₂N(CH₃)₂;
**R⁶** represents 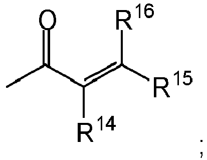
**R⁷** represents -H, -CH₃, or -C₂H₅;
**R⁸** - **R¹¹** represent independently of each other -H, -F, -CI, -CN, -OCH₃, -OC₂H₅, -CH₃, -CF₃, or -C₂H₅;
**R¹², R^{12*}, R^{12**},** and **R¹³** represent independently of each other -H, -CH₃, or -C₂H₅;
**R¹⁴, R¹⁵,** and **R¹⁶** represent independently of each other -H, -CH₃, -CF₃, -CN, -NO₂, -COCH₃, or -COC₂H₅;
or an enantiomer, a mixture of enantiomers, a diastereomer, a mixture of diastereomers, a tautomer, a hydrate, a solvate, or pharmaceutically acceptable salts thereof.

Preferred are compounds of general formula (I) wherein **B** represents 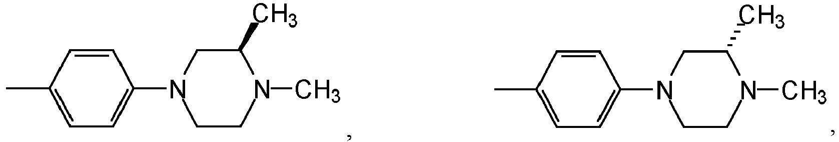 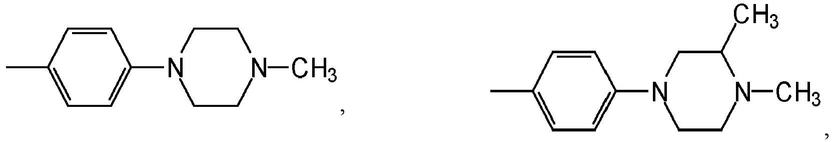 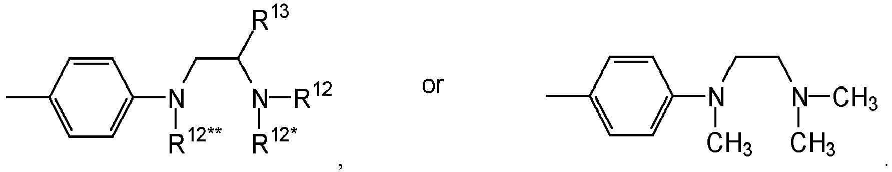 and **R¹², R^{12*}, R^{12**}**, and **R¹³** have the meanings as defined herein.

Also preferred are compounds of general formula (I) wherein A represents 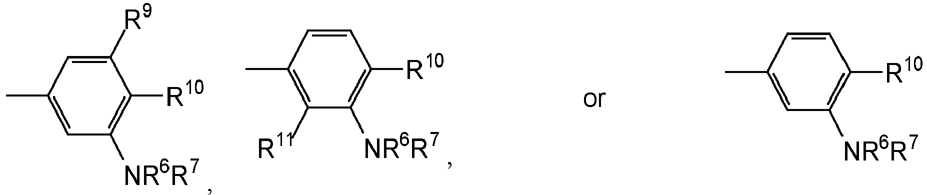 and **R⁶, R⁷** and **R⁹ - R¹¹** have the meanings as defined herein.

Moreover, **R¹** represents preferably 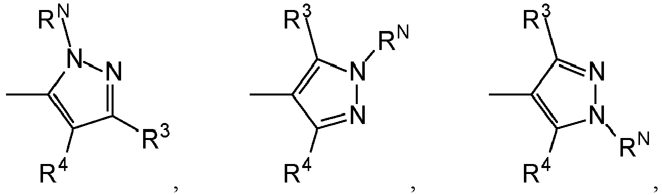 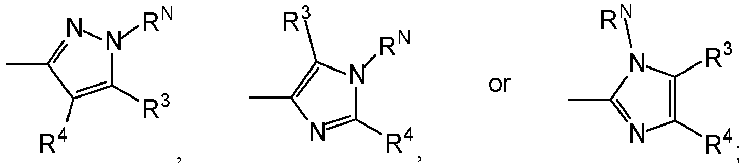 **R³, R⁴,** and **R^{N}** have the meanings as defined in Claim 1.

Furthermore, compounds of general formula (I) are preferred wherein **R⁶** represents

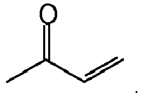

More preferred are compounds of general formula (I) wherein
A represents 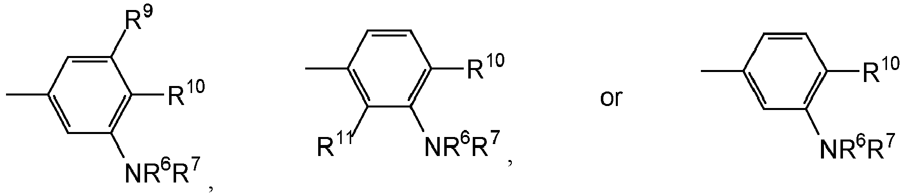 B represents 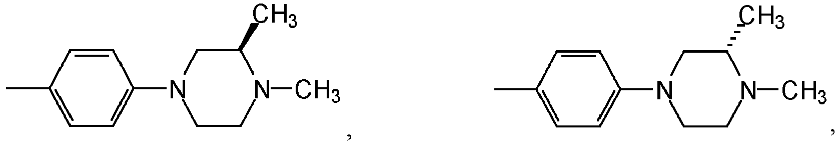 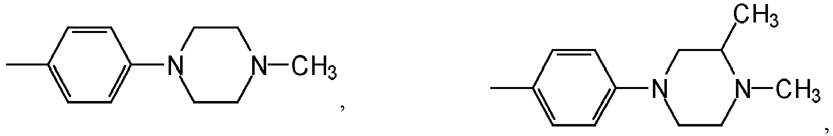 or 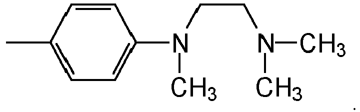 **R¹** represents 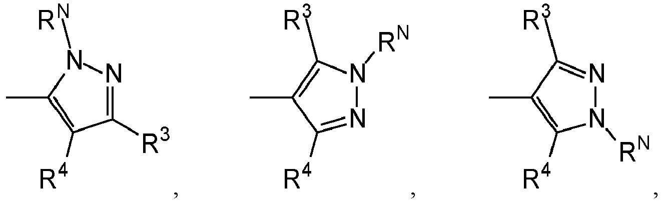 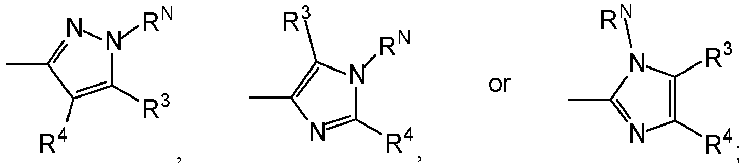 **R²** represents -H, -F, -CI, -Br, -CN, -CH₃, -C₂H₅, -C₃H₇, -OCH₃, -OC₂H₅, or -OC₃H₇;
**R³** and **R⁴** represent independently of each other -H, -F, -CI, -CN, -CF₃, -OCH₃, -OC₂H₅, -CH₂OCH₃, -CH₃, -C₂H₅, or -C₃H₇;
**R⁵** represents 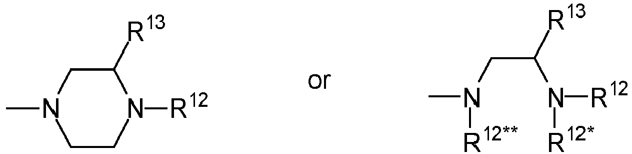 **R^{N}** represent -H, -CH₃, -CF₃, -C₂H₅, -C₃H₇, -CH(CH₃)₂, -CH₂CH(CH₃)₂, -C₄H₉, -cyclo-C₃H₅, -cyclo-C₄H₇, -CH₂CH₂OH, -CH₂CH(CH₃)OH, -CH₂CH₂OCH₃, -CH₂CH₂CH₂OH, -CH₂CH₂CH₂OCH₃, or -CH₂CH₂N(CH₃)₂ ; **R⁶** represents 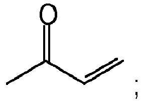 **R⁷** represents -H, -CH₃, or -C₂H₅;
**R⁸** - **R¹¹** represent independently of each other -H, -F, -CI, -CN, -OCH₃, -OC₂H₅, -CH₃, -CF₃, or -C₂H₅;
**R¹², R^{12*}, R^{12**},** and **R¹³** represent independently of each other -H or -CH₃;
or an enantiomer, a mixture of enantiomers, a diastereomer, a mixture of diastereomers, a tautomer, a hydrate, a solvate, or pharmaceutically acceptable salts thereof.

In regard to all general formula disclosed herein, **A** represents preferably 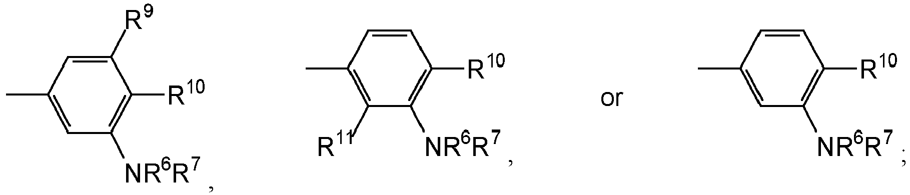 more preferably 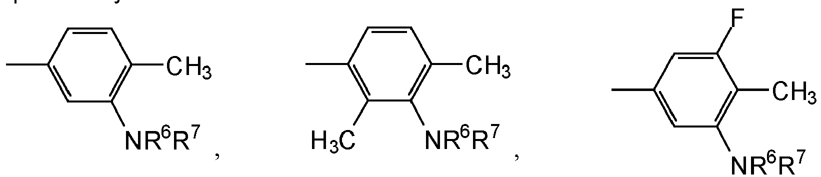 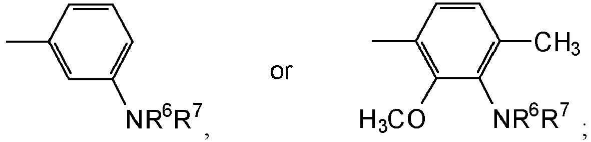 and most preferably 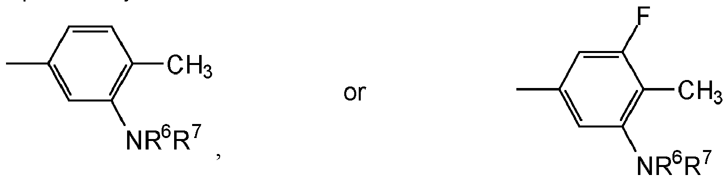 and **R⁶, R⁷, R⁹, R¹⁰,** and **R¹¹** have the meanings as defined herein.

In regard to all general formula disclosed herein **R¹** represents preferably 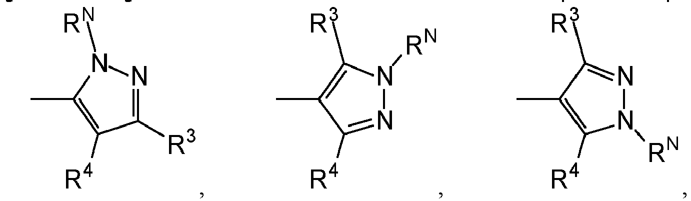 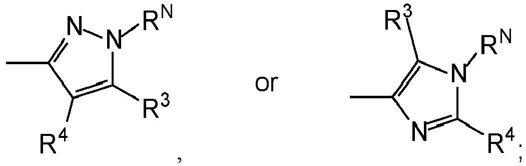 and more preferably 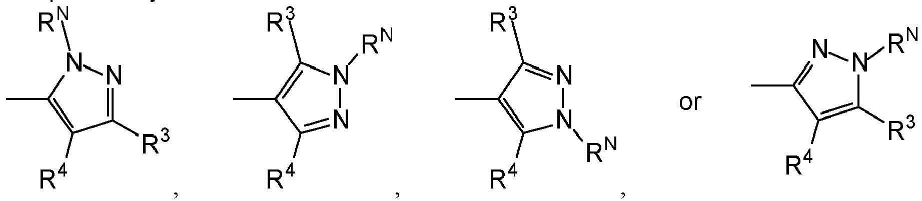 and **R³, R⁴,** and **R^{N}** have the meanings as defined herein.

In regard to all general formula disclosed herein, **R²** represents preferably -H, -F, -Cl, -CN, -CH₃, -C₂H₅, -OCH₃, or -OC₂H₅, more preferably -H, -CI, -CN, -CH₃, -C₂H₅, or -OCH₃, still more preferably -H, -CI, -CH₃, -C₂H₅, or -OCH₃, still more preferably -H, -CI, -CH₃, or -OCH₃, still more preferably -H, -CI, or -CH₃, still more preferably -H or -CH₃, and most preferably -CH₃.

In regard to all general formula disclosed herein, **R³** and **R⁴** preferably represent independently of each other -H, -F, -CI, -CN, -CF₃, -OCH₃, -OC₂H₅, -OC₃H₇, -CH₂OCH₃, -CH₃, -C₂H₅, or -C₃H₇, -CH(CH₃)₂, -C₄H₉, -cyclo-C₃H₅, more preferably -H, -CI, -CN, -CF₃, -OCH₃, -OC₂H₅, -CH₃, or -C₂H₅, still more preferably -H, -CN, -OCH₃, -CH₃, or -C₂H₅, still more preferably -H, -CN, -OCH₃, or -CH₃, still more preferably -H, -CN, or -CH₃, and most preferably -H or -CH₃.

In regard to all general formula disclosed herein, **R^{N}** represent preferably -H, -CH₃, -CF₃, -C₂H₅, -C₃H₇, -CH(CH₃)₂, -CH₂CH(CH₃)₂, -C₄H₉, -cyclo-C₃H₅, -cyclo-C₄H₇, -CH₂CH₂OH, -CH₂CH(CH₃)OH, -CH₂CH₂OCH₃, -CH₂CH₂CH₂OH, or -CH₂CH₂CH₂OCH₃; more preferably -H, -CH₃, -CF₃, -C₂H₅, -C₃H₇, -CH(CH₃)₂, -cyclo-C₃H₅, -CH₂CH₂OH, -CH₂CH(CH₃)OH, -CH₂CH₂OCH₃, -CH₂CH₂CH₂OH, or -CH₂CH₂CH₂OCH₃; still more preferably -H, -CH₃, -CF₃, -C₂H₅, -C₃H₇, -CH(CH₃)₂, -cyclo-C₃H₅, -CH₂CH₂OH, -CH₂CH(CH₃)OH, or -CH₂CH₂OCH₃.

**R⁵** is preferably

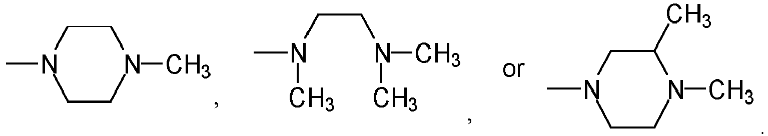

In regard to all general formula disclosed herein, **R⁷** represent preferably -H or -CH₃, and more preferably -H.

In regard to all general formula disclosed herein, **R⁸** - **R¹¹** preferably represent independently of each other -H, -F, -CI, -CN, -OCH₃, -OC₂H₅, -OC₃H₇, -CH₂OCH₃, -CH₃, -CF₃, -C₂H₅, or -C₃H₇; more preferably -H, -F, -CN, -OCH₃, -OC₂H₅, -CH₃, or -C₂H₅; still more preferably -H, -F, or -OCH₃, -CH₃; most preferably -CH₃.
More preferably **R⁸** represents -H.

Thus, in regard to all general formula disclosed herein, **R⁹** - **R¹¹** preferably represent independently of each other -H, -F, -CI, -CN, -OCH₃, -OC₂H₅, -OC₃H₇, -CH₂OCH₃, -CH₃, -CF₃, -C₂H₅, or -C₃H₇; more preferably -H, -F, -CN, -OCH₃, -OC₂H₅, -CH₃, or -C₂H₅; still more preferably -H, -F, or -OCH₃, -CH₃; most preferably -CH₃.

More preferably **R⁹** represents -H, -F, -CI, -CF₃, or -CN, and still more preferably -H or -F.

Thus, in regard to all general formula disclosed herein, **R¹⁰** and **R¹¹** preferably represent independently of each other -H, -OCH₃, -OC₂H₅, -OC₃H₇, -CH₂OCH₃, -CH₃, -C₂H₅, or -C₃H₇; more preferably -H, -OCH₃, -OC₂H₅, -CH₃, or -C₂H₅; still more preferably -H, -OCH₃, or -CH₃;
In regard to all general formula disclosed herein, **R¹⁰** represent preferably -CH₂OCH₃, -CH₃, -C₂H₅, or -C₃H₇; more preferably -CH₃, -C₂H₅, or -C₃H₇; still more preferably -CH₃ or -C₂H₅; and most preferably -CH₃.

In regard to all general formula disclosed herein, **R¹⁴, R¹⁵,** and **R¹⁶** preferably represent independently of each other -H, -CH₃, -CF₃, -CN, -NO₂, or -COCH₃; more preferably other -H, -CF₃, -CN, or -COCH₃; more preferably -H, -CN, or -COCH₃; more preferably -H, -COCH₃, and most preferably -H.

Also preferred are compounds of general formula (I) wherein
**A** represents 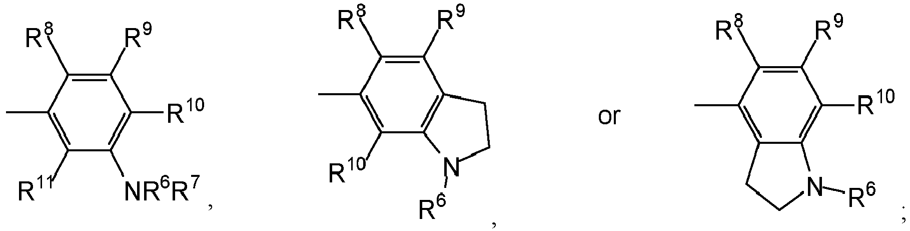 **B** represents 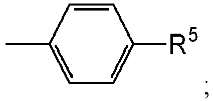 **R¹** represents 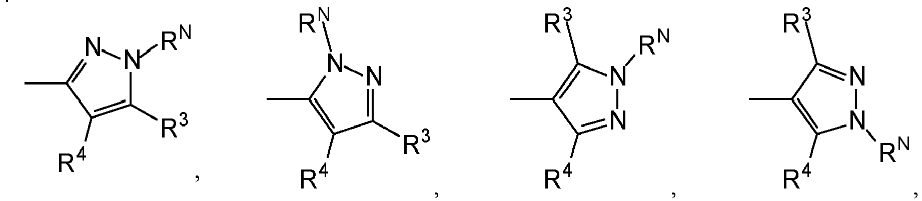 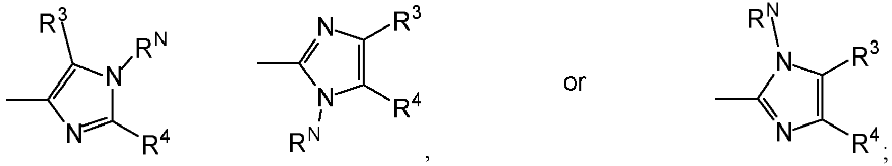 **R²** represents -H, -CI, -CH₃, -C₂H₅, -OCH₃;
**R³** and **R⁴** represent independently of each other -H, -CN, or -CH₃;
**R⁵** represents 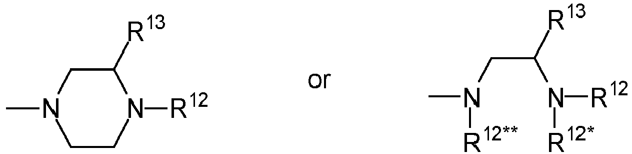 **R^{N}** represent -H, -CH₃, -CF₃, -C₂H₅, -CH(CH₃)₂, -cyclo-C₃H₅, -CH₂CH₂OH, -CH₂CH(CH₃)OH, or -CH₂CH₂OCH₃;
**R⁶** represents 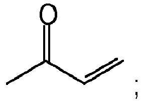 **R⁷** represents -H or -CH₃;
**R⁸** - **R¹¹** represent independently of each other -H, -F, -OCH₃, or -CH₃;
**R¹², R^{12*}, R^{12**},** and **R¹³** represent independently of each other -H or -CH₃;
or an enantiomer, a mixture of enantiomers, a diastereomer, a mixture of diastereomers, a tautomer, a hydrate, a solvate, or pharmaceutically acceptable salts thereof.

The present invention provides 2,3,5-substituted 1*H*-pyrrolo[2,3-*b*]pyridine compounds with an aromatic 5-membered heterocyclic substituent at position 5 which exhibit inhibition of HER2 Exon20 A775_G776insYVMA (and similar mutations) and/or EGFR Exon20 H773_V774insNPH (and similar mutations). The present invention provides these compounds as mutant-selective ErbB inhibitors, especially for the Exon20 EGFR/Her2 mutations, and additionally as inhibitors for other mutants like EGFR T790ML858R mutation. In certain embodiments the current invention is directed towards 2,3,5-substituted 1*H*-pyrrolo[2,3-*b*]pyridine compounds which are mutant-specific for Exon20 of EGFR and Her2. In one aspect, the invention provides 2,3,5-substituted 1*H*-pyrrolo[2,3-*b*]pyridine compounds as irreversible kinase inhibitors. The 2,3,5-substituted 1*H*-pyrrolo[2,3-*b*]pyridine compounds covalently modify the cysteine 797/805 in EGFR/Her2.

The 2,3,5-substituted 1*H*-pyrrolo[2,3-*b*]pyridine compounds with an aromatic 5-membered heterocyclic substituent at position 5 and/or the pharmaceutically acceptable salts thereof are used as pharmaceutical active ingredients for the treatment and/or prophylxis of a cell proliferative disease such as cancer.

The cell proliferative disease is selected from breast cancer, colon cancer, prostate cancer, lung cancer, gastric cancer, ovarian cancer, endometrial cancer, renal cancer, hepatocellular cancer, thyroid cancer, uterine cancer, esophagus cancer, squamous cell cancer, leukemia, osteosarcoma, melanoma, glioblastoma and neuroblastoma. In an especially preferred embodiment, the disorders are selected from breast cancer, glioblastoma, renal cancer, non-small cell lung cancer (NSCLC), and melanoma. The compounds are also suitable for the prevention and/or treatment of other hyperproliferative disorders, particulary benign hyperproliferative disorders such as benign prostate hyperplasia.

Especially preferred compounds according to the present invention include compounds presented by **Table 1.**

**Table 1**

| **Example** | **Structure** | **Nomenclature** |
|---|---|---|
| **1** | 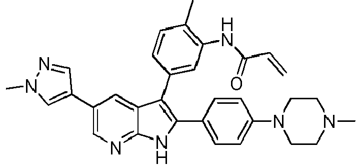 | N-(2-methyl-5-(5-(1-methyl-1H-pyrazol-4-yl)-2-(4-(4-methylpiperazin-1-yl)phenyl)-1H-pyrrolo[2,3-b]pyridin-3-yl)phenyl)acrylamide |
| **2** | 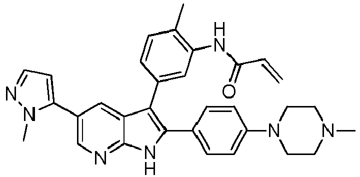 | N-(2-methyl-5-(5-(1-methyl-1H-pyrazol-5-yl)-2-(4-(4-methylpiperazin-1-yl)phenyl)-1H-pyrrolo[2,3-b]pyridin-3-yl)phenyl)acrylamide |
| **3** | 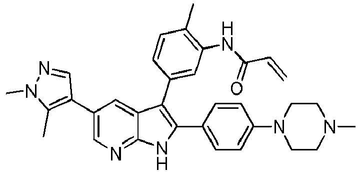 | N-(5-(5-(1,5-dimethyl-1H-pyrazol-4-yl)-2-(4-(4-methylpiperazin-1-yl)phenyl)-1H-pyrrolo[2,3-b]pyridin-3-yl)-2-methylphenyl)acrylamide |
| **4** | 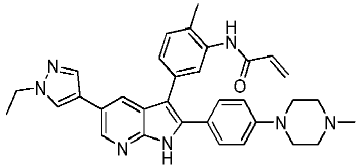 | N-(5-(5-(1-ethyl-1H-pyrazol-4-yl)-2-(4-(4-methylpiperazin-1-yl)phenyl)-1 H-pyrrolo[2,3-b]pyridin-3-yl)-2-methylphenyl)acrylamide |
| **5** | 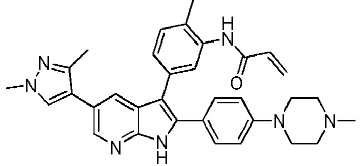 | N-(5-(5-(1,3-dimethyl-1H-pyrazol-4-yl)-2-(4-(4-methylpiperazin-1-yl)phenyl)-1H-pyrrolo[2,3-b]pyridin-3-yl)-2-methylphenyl)acrylamide |
| **6** | 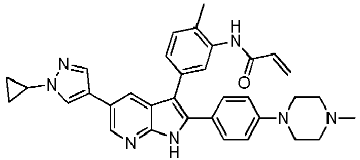 | N-(5-(5-(1-cyclopropyl-1H-pyrazol-4-yl)-2-(4-(4-methylpiperazin-1-yl)phenyl)-1H-pyrrolo[2,3-b]pyridin-3-yl)-2-methylphenyl)acrylamide |
| **7** | 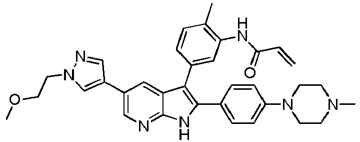 | N-(5-(5-(1-(2-methoxyethyl)-1H-pyrazol-4-yl)-2-(4-(4-methylpiperazin-1-yl)phenyl)-1H-pyrrolo[2,3-b]pyridin-3-yl)-2-methylphenyl)acrylamide |
| **8** | 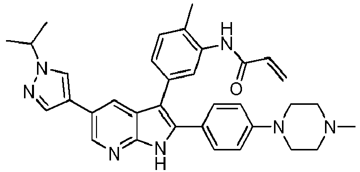 | N-(5-(5-(1-isopropyl-1H-pyrazol-4-yl)-2-(4-(4-methylpiperazin-1-yl)phenyl)-1H-pyrrolo[2,3-b]pyridin-3-yl)-2-methylphenyl)acrylamide |
| **9** | 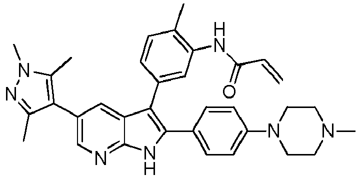 | N-(2-methyl-5-(2-(4-(4-methylpiperazin-1-yl)phenyl)-5-(1,3,5-trimethyl-1H-pyrazol-4-yl)-1H-pyrrolo[2,3-b]pyridin-3-yl)phenyl)acrylamide |
| **10** | 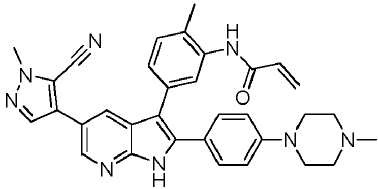 | N-(5-(5-(5-cyano-1-methyl-1H-pyrazol-4-yl)-2-(4-(4-methylpiperazin-1-yl)phenyl)-1H-pyrrolo[2,3-b]pyridin-3-yl)-2-methylphenyl)acrylamide |
| **11** | 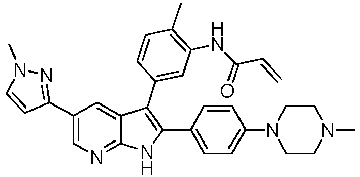 | N-(2-methyl-5-(5-(1-methyl-1H-pyrazol-3-yl)-2-(4-(4-methylpiperazin-1-yl)phenyl)-1H-pyrrolo[2,3-b]pyridin-3-yl)phenyl)acrylamide |
| **12** | 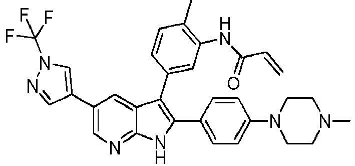 | N-(2-methyl-5-(2-(4-(4-methylpiperazin-1-yl)phenyl)-5-(1-(trifluoromethyl)-1H-pyrazol-4-yl)-1H-pyrrolo[2,3-b]pyridin-3-yl)phenyl)acrylamide |
| **13** | 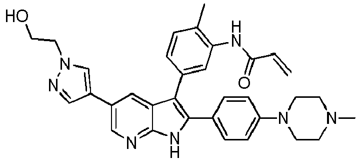 | N-(5-(5-(1-(2-hydroxyethyl)-1H-pyrazol-4-yl)-2-(4-(4-methylpiperazin-1-yl)phenyl)-1H-pyrrolo[2,3-b]pyridin-3-yl)-2-methylphenyl)acrylamide |
| **14** | 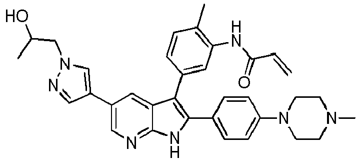 | N-(5-(5-(1-(2-hydroxypropyl)-1H-pyrazol-4-yl)-2-(4-(4-methylpiperazin-1-yl)phenyl)-1H-pyrrolo[2,3-b]pyridin-3-yl)-2-methylphenyl)acrylamide |
| **15** | 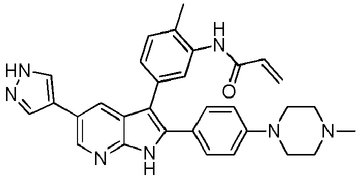 | N-(2-methyl-5-(2-(4-(4-methylpiperazin-1-yl)phenyl)-5-(1 H-pyrazol-4-yl)-1H-pyrrolo[2,3-b]pyridin-3-yl)phenyl)acrylamide |
| **16** | 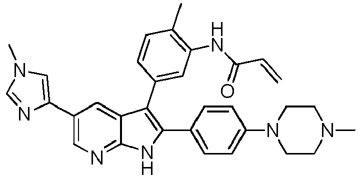 | N-(2-methyl-5-(5-(1-methyl-1H-imidazol-4-yl)-2-(4-(4-methylpiperazin-1-yl)phenyl)-1H-pyrrolo[2,3-b]pyridin-3-yl)phenyl)acrylamide |
| **17** | 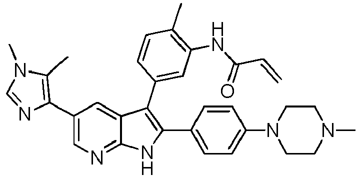 | N-(5-(5-(1,5-dimethyl-1H-imidazol-4-yl)-2-(4-(4-methylpiperazin-1-yl)phenyl)-1H-pyrrolo[2,3-b]pyridin-3-yl)-2-methylphenyl)acrylamide |
| **18** | 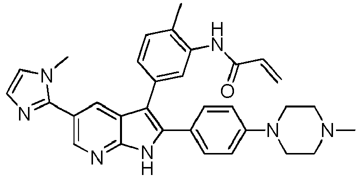 | N-(2-methyl-5-(5-(1-methyl-1H-imidazol-2-yl)-2-(4-(4-methylpiperazin-1-yl)phenyl)-1H-pyrrolo[2,3-b]pyridin-3-yl)phenyl)acrylamide |
| **19** | 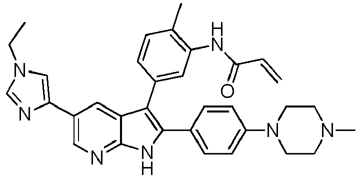 | N-(5-(5-(1-ethyl-1H-imidazol-4-yl)-2-(4-(4-methylpiperazin-1-yl)phenyl)-1 H-pyrrolo[2,3-b]pyridin-3-yl)-2-methylphenyl)acrylamide |
| **20** | 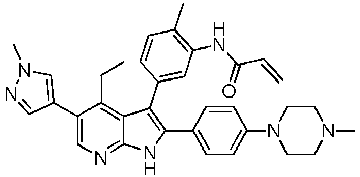 | N-(5-(4-ethyl-5-(1-methyl-1H-pyrazol-4-yl)-2-(4-(4-methylpiperazin-1-yl)phenyl)-1H-pyrrolo[2,3-b]pyridin-3-yl)-2-methylphenyl)acrylamide |
| **21** | 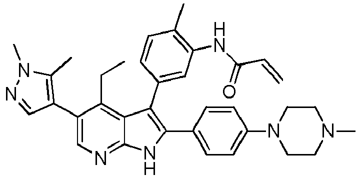 | N-(5-(5-(1,5-dimethyl-1H-pyrazol-4-yl)-4-ethyl-2-(4-(4-methylpiperazin-1-yl)phenyl)-1H-pyrrolo[2,3-b]pyridin-3-yl)-2-methylphenyl)acrylamide |
| **22** | 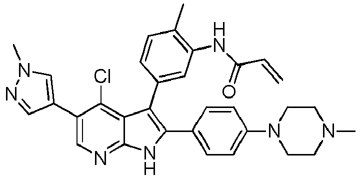 | N-(5-(4-chloro-5-(1-methyl-1H-pyrazol-4-yl)-2-(4-(4-methylpiperazin-1-yl)phenyl)-1H-pyrrolo[2,3-b]pyridin-3-yl)-2-methylphenyl)acrylamide |
| **23** | 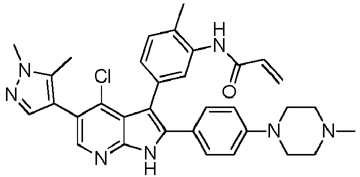 | N-(5-(4-chloro-5-(1,5-dimethyl-1H-pyrazol-4-yl)-2-(4-(4-methylpiperazin-1-yl)phenyl)-1H-pyrrolo[2,3-b]pyridin-3-yl)-2-methylphenyl)acrylamide |
| **24** | 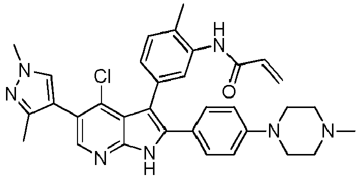 | N-(5-(4-chloro-5-(1,3-dimethyl-1H-pyrazol-4-yl)-2-(4-(4-methylpiperazin-1-yl)phenyl)-1H-pyrrolo[2,3-b]pyridin-3-yl)-2-methylphenyl)acrylamide |
| **25** | 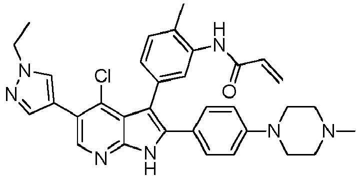 | N-(5-(4-chloro-5-(1-ethyl-1H-pyrazol-4-yl)-2-(4-(4-methylpiperazin-1-yl)phenyl)-1H-pyrrolo[2,3-b]pyridin-3-yl)-2-methylphenyl)acrylamide |
| **26** | 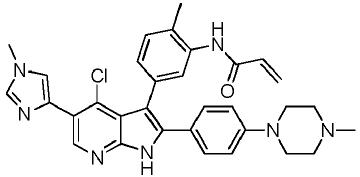 | N-(5-(4-chloro-5-(1-methyl-1H-imidazol-4-yl)-2-(4-(4-methylpiperazin-1-yl)phenyl)-1H-pyrrolo[2,3-b]pyridin-3-yl)-2-methylphenyl)acrylamide |
| **27** | 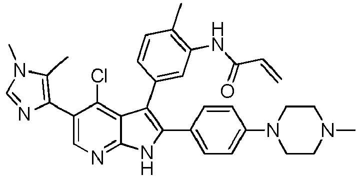 | N-(5-(4-chloro-5-(1,5-dimethyl-1H-imidazol-4-yl)-2-(4-(4-methylpiperazin-1-yl)phenyl)-1H-pyrrolo[2,3-b]pyridin-3-yl)-2-methylphenyl)acrylamide |
| **28** | 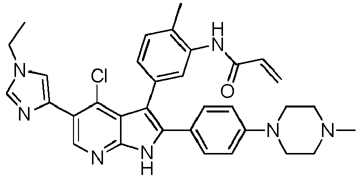 | N-(5-(4-chloro-5-(1-ethyl-1H-imidazol-4-yl)-2-(4-(4-methylpiperazin-1-yl)phenyl)-1H-pyrrolo[2,3-b]pyridin-3-yl)-2-methylphenyl)acrylamide |
| **29** | 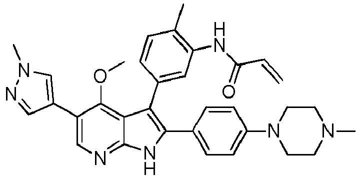 | N-(5-(4-methoxy-5-(1-methyl-1H-pyrazol-4-yl)-2-(4-(4-methylpiperazin-1-yl)phenyl)-1H-pyrrolo[2,3-b]pyridin-3-yl)-2-methylphenyl)acrylamide |
| **30** | 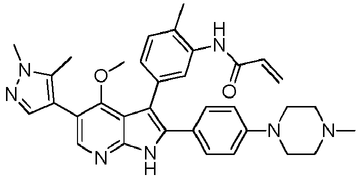 | N-(5-(5-(1,5-dimethyl-1H-pyrazol-4-yl)-4-methoxy-2-(4-(4-methylpiperazin-1-yl)phenyl)-1 H-pyrrolo[2,3-b]pyridin-3-yl)-2-methylphenyl)acrylamide |
| **31** | 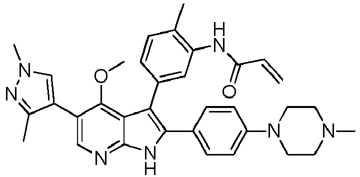 | N-(5-(5-(1,3-dimethyl-1H-pyrazol-4-yl)-4-methoxy-2-(4-(4-methylpiperazin-1-yl)phenyl)-1 H-pyrrolo[2,3-b]pyridin-3-yl)-2-methylphenyl)acrylamide |
| **32** | 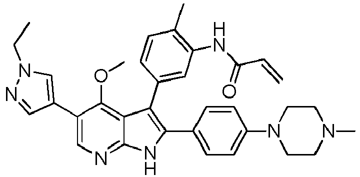 | N-(5-(5-(1-ethyl-1H-pyrazol-4-yl)-4-methoxy-2-(4-(4-methylpiperazin-1-yl)phenyl)-1H-pyrrolo[2,3-b]pyridin-3-yl)-2-methylphenyl)acrylamide |
| **33** | 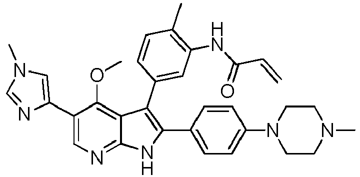 | N-(5-(4-methoxy-5-(1-methyl-1H-imidazol-4-yl)-2-(4-(4-methylpiperazin-1-yl)phenyl)-1H-pyrrolo[2,3-b]pyridin-3-yl)-2-methylphenyl)acrylamide |
| **34** | 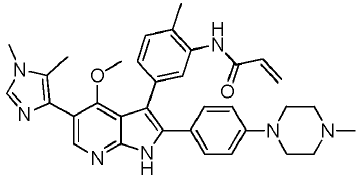 | N-(5-(5-(1,5-dimethyl-1H-imidazol-4-yl)-4-methoxy-2-(4-(4-methylpiperazin-1-yl)phenyl)-1 H-pyrrolo[2,3-b]pyridin-3-yl)-2-methylphenyl)acrylamide |
| **35** | 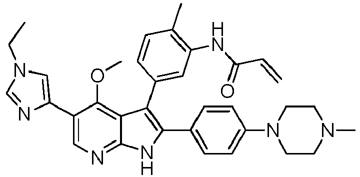 | N-(5-(5-(1-ethyl-1H-imidazol-4-yl)-4-methoxy-2-(4-(4-methylpiperazin-1-yl)phenyl)-1H-pyrrolo[2,3-b]pyridin-3-yl)-2-methylphenyl)acrylamide |
| **36** | 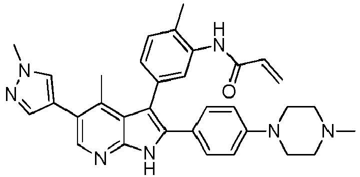 | N-(2-methyl-5-(4-methyl-5-(1-1-methyl-1 H-pyrazol-4-yl)-2-(4-(4-methylpiperazin-1-yl)phenyl)-1 H-pyrrolo[2,3-b]pyridin-3-yl)phenyl)acrylamide |
| **37** | 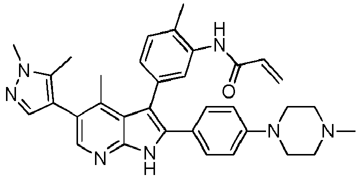 | N-(5-(5-(1,5-dimethyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide |
| **38** | 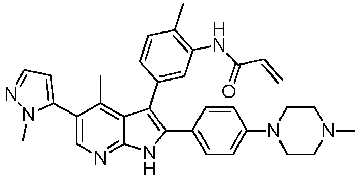 | N-(2-methyl-5-(4-methyl-5-(1-1-methyl-1 H-pyrazol-5-yl)-2-(4-(4-methylpiperazin-1-yl)phenyl)-1 H-pyrrolo[2,3-b]pyridin-3-yl)phenyl)acrylamide |
| **39** | 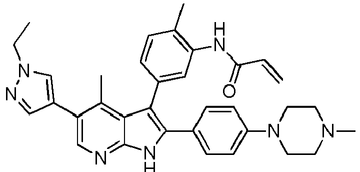 | N-(5-(5-(1-ethyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide |
| **40** | 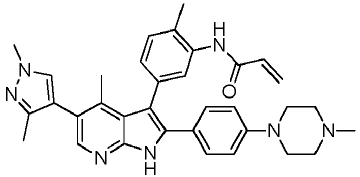 | N-(5-(5-(1,3-dimethyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide |
| **41** | 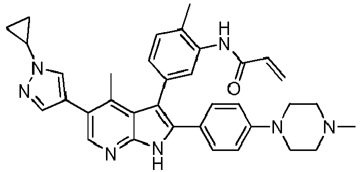 | N-(5-(5-(1-cyclopropyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide |
| **42** | 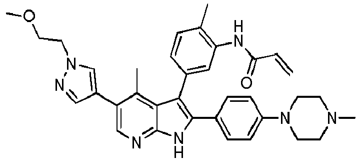 | N-(5-(5-(1-(2-methoxyethyl)-1 H-pyrazol-4-yl)-4-methyl-2-(4-(4-methylpiperazin-1-yl)phenyl)-1 H-pyrrolo[2,3-b]pyridin-3-yl)-2-methylphenyl)acrylamide |
| **43** | 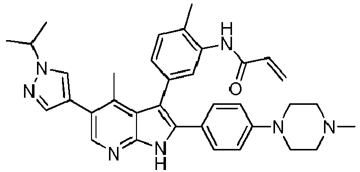 | N-(5-(5-(1-isopropyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide |
| **44** | 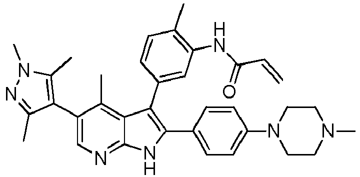 | N-(2-methyl-5-(4-methyl-2-(4-(4-methylpiperazin-1-yl)phenyl)-5-(1,3,5-trimethyl-1H-pyrazol-4-yl)-1H-pyrrolo[2,3-b]pyridin-3-yl)phenyl)acrylamide |
| **45** | 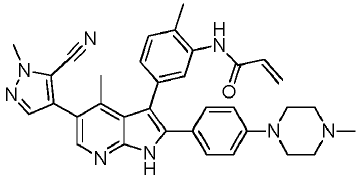 | N-(5-(5-(5-cyano-1-methyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide |
| **46** | 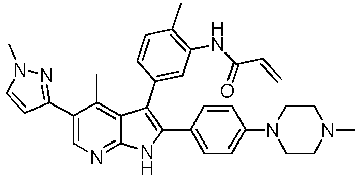 | N-(2-methyl-5-(4-methyl-5-( 1-methyl-1 H-pyrazol-3-yl)-2-(4-(4-methylpiperazin-1-yl)phenyl)-1 H-pyrrolo[2,3-b]pyridin-3-yl)phenyl)acrylamide |
| **47** | 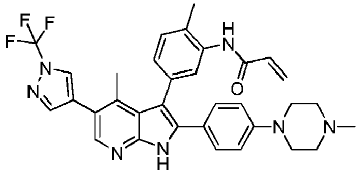 | N-(2-methyl-5-(4-methyl-2-(4-(4-methylpiperazin-1-yl)phenyl)-5-(1-(trifluoromethyl)-1H-pyrazol-4-yl)-1H-pyrrolo[2,3-b]pyridin-3-yl)phenyl)acrylamide |
| **48** | 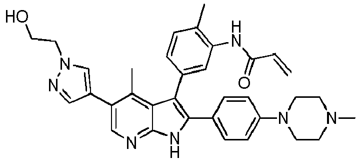 | N-(5-(5-(1-(2-hydroxyethyl)-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide |
| **49** | 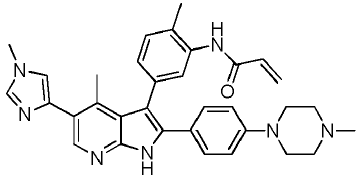 | N-(2-methyl-5-(4-methyl-5-( 1-methyl-1 H-imidazol-4-yl)-2-(4-(4-methylpiperazin-1-yl)phenyl)-1 H-pyrrolo[2,3-b]pyridin-3-yl)phenyl)acrylamide |
| **50** | 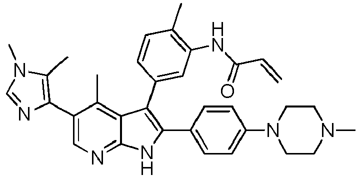 | N-(5-(5-(1,5-dimethyl-1H-imidazol-4-yl)-4-methyl-2-(4-(4-methylpiperazin-1-yl)phenyl)-1H-pyrrolo[2,3-b]pyridin-3-yl)-2-methylphenyl)acrylamide |
| **51** | 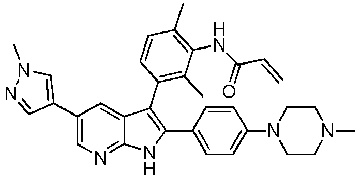 | N-(2,6-dimethyl-3-(5-(1-methyl-1H-pyrazol-4-yl)-2-(4-(4-methylpiperazin-1-yl)phenyl)-1H-pyrrolo[2,3-b]pyridin-3-yl)phenyl)acrylamide |
| **52** | 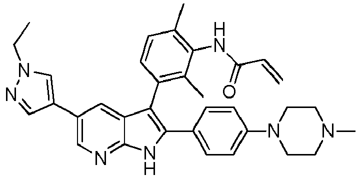 | N-(3-(5-(1-ethyl-1H-pyrazol-4-yl)-2-(4-(4-methylpiperazin-1-yl)phenyl)-1 H-pyrrolo[2,3-b]pyridin-3-yl)-2,6-dimethylphenyl)acrylam ide |
| **53** | 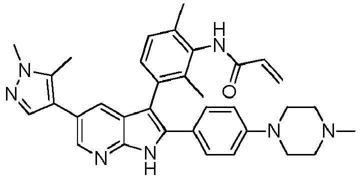 | N-(3-(5-(1,5-dimethyl-1H-pyrazol-4-yl)-2-(4-(4-methylpiperazin-1-yl)phenyl)-1H-pyrrolo[2,3-b]pyridin-3-yl)-2,6-dimethylphenyl)acrylamide |
| **54** | 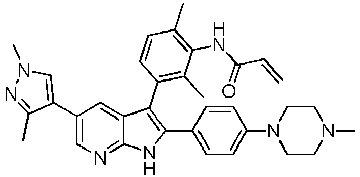 | N-(3-(5-(1,3-dimethyl-1H-pyrazol-4-yl)-2-(4-(4-methylpiperazin-1-yl)phenyl)-1H-pyrrolo[2,3-b]pyridin-3-yl)-2,6-dimethylphenyl)acrylamide |
| **55** | 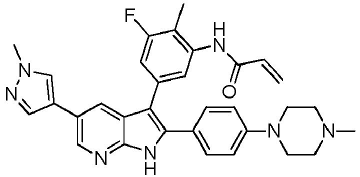 | N-(3-fluoro-2-methyl-5-(5-(1-methyl-1 H-pyrazol-4-yl)-2-(4-(4-methylpiperazin-1-yl)phenyl)-1 H-pyrrolo[2,3-b]pyridin-3-yl)phenyl)acrylamide |
| **56** | 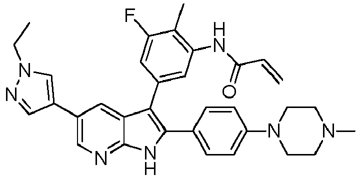 | N-(5-(5-(1-ethyl-1H-pyrazol-4-yl)-2-(4-(4-methylpiperazin-1-yl)phenyl)-1 H-pyrrolo[2,3-b]pyridin-3-yl)-3-fluoro-2-methylphenyl)acrylamide |
| **57** | 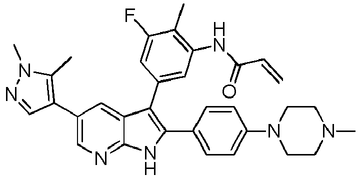 | N-(5-(5-(1,5-dimethyl-1H-pyrazol-4-yl)-2-(4-(4-methylpiperazin-1-yl)phenyl)-1 H-pyrrolo[2,3-b]pyridin-3-yl)-3-fluoro-2-methylphenyl)acrylamide |
| **58** | 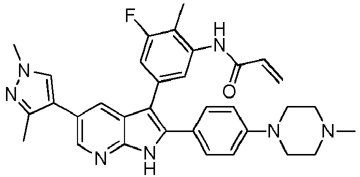 | N-(5-(5-(1,3-dimethyl-1H-pyrazol-4-yl)-2-(4-(4-methylpiperazin-1-yl)phenyl)-1H-pyrrolo[2,3-b]pyridin-3-yl)-3-fluoro-2-methylphenyl)acrylamide |
| **59** | 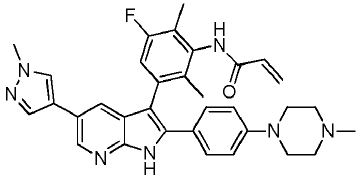 | N-(3-fluoro-2,6-dimethyl-5-(5-(1-methyl-1 H-pyrazol-4-yl)-2-(4-(4-methylpiperazin-1-yl)phenyl)-1 H-pyrrolo[2,3-b]pyridin-3-yl)phenyl)acrylamide |
| **60** | 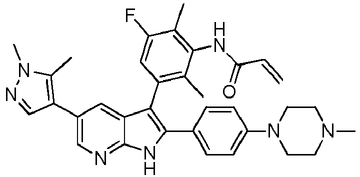 | N-(3-(5-(1,5-dimethyl-1H-pyrazol-4-yl)-2-(4-(4-methylpiperazin-1-yl)phenyl)-1 H-pyrrolo[2,3-b]pyridin-3-yl)-5-fluoro-2,6-dimethylphenyl)acrylam ide |
| **61** | 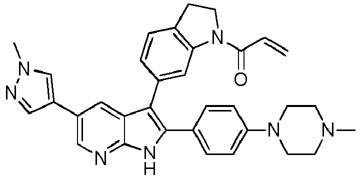 | 1-(6-(5-(1-methyl-1 H-pyrazol-4-yl)-2-(4-(4-methylpiperazin-1-yl)phenyl)-1 H-pyrrolo[2,3-b]pyridin-3-yl)indolin-1-yl)prop-2-en-1-one |
| **62** | 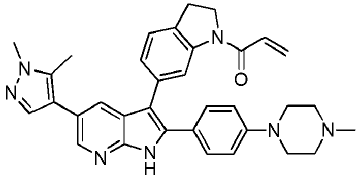 | 1-(6-(5-(1,5-dimethyl-1H-pyrazol-4-yl)-2-(4-(4-methylpiperazin-1-yl)phenyl)-1H-pyrrolo[2,3-b]pyridin-3-yl)indolin-1-yl)prop-2-en-1-one |
| **63** | 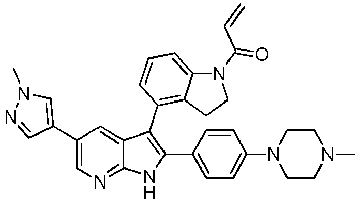 | 1-(4-(5-(1-methyl-1 H-pyrazol-4-yl)-2-(4-(4-methylpiperazin-1-yl)phenyl)-1 H-pyrrolo[2,3-b]pyridin-3-yl)indolin-1-yl)prop-2-en-1-one |
| **64** | 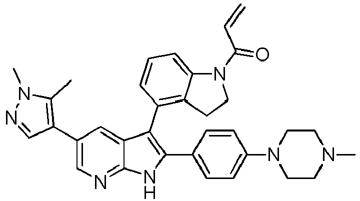 | 1-(4-(5-(1,5-dimethyl-1H-pyrazol-4-yl)-2-(4-(4-methylpiperazin-1-yl)phenyl)-1H-pyrrolo[2,3-b]pyridin-3-yl)indolin-1-yl)prop-2-en-1-one |
| **65** | 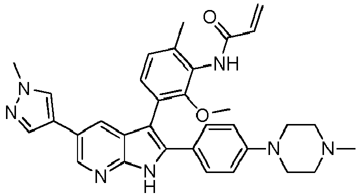 | N-(2-methoxy-6-methyl-3-(5-(1-methyl-1 H-pyrazol-4-yl)-2-(4-(4-methylpiperazin-1-yl)phenyl)-1 H-pyrrolo[2,3-b]pyridin-3-yl)phenyl)acrylamide |
| **66** | 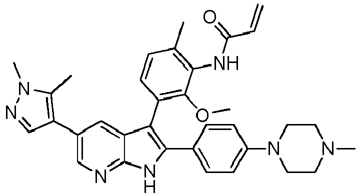 | N-(3-(5-(1,5-dimethyl-1H-pyrazol-4-yl)-2-(4-(4-methylpiperazin-1-yl)phenyl)-1 H-pyrrolo[2,3-b]pyridin-3-yl)-2-methoxy-6-methylphenyl)acrylamide |
| **67** | 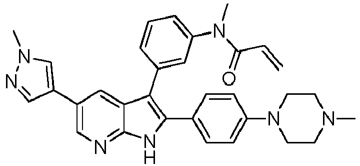 | N-methyl-N-(3-(5-(1-methyl-1H-pyrazol-4-yl)-2-(4-(4-methylpiperazin-1-yl)phenyl)-1H-pyrrolo[2,3-b]pyridin-3-yl)phenyl)acrylamide |
| **68** | 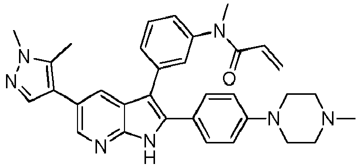 | N-(3-(5-(1,5-dimethyl-1H-pyrazol-4-yl)-2-(4-(4-methylpiperazin-1-yl)phenyl)-1H-pyrrolo[2,3-b]pyridin-3-yl)phenyl)-N-methylacrylamide |
| **69** | 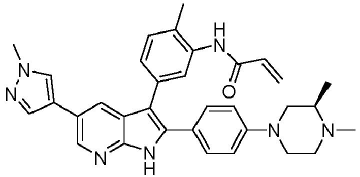 | (R)-N-(5-(2-(4-(3,4-dimethylpiperazin-1-yl)phenyl)-5-(1-methyl-1 H-pyrazol-4-yl)-1 H-pyrrolo[2,3-b]pyridin-3-yl)-2-methylphenyl)acrylamide |
| **70** | 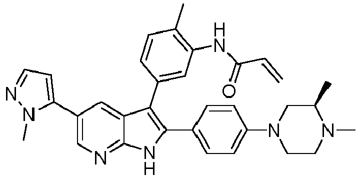 | (R)-N-(5-(2-(4-(3,4-dimethylpiperazin-1-yl)phenyl)-5-(1-methyl-1 H-pyrazol-5-yl)-1 H-pyrrolo[2,3-b]pyridin-3-yl)-2-methylphenyl)acrylamide |
| **71** | 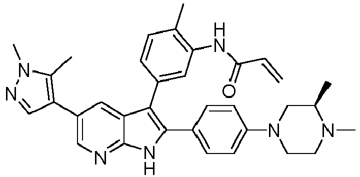 | (R)-N-(5-(5-(1,5-dimethyl-1 H-pyrazol-4-yl)-2-(4-(3,4-dimethylpiperazin-1-yl)phenyl)-1 H-pyrrolo[2,3-b]pyridin-3-yl)-2-methylphenyl)acrylamide |
| **72** | 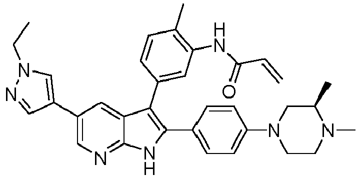 | (R)-N-(5-(2-(4-(3,4-dimethylpiperazin-1-yl)phenyl)-5-(1-ethyl-1 H-pyrazol-4-yl)-1 H-pyrrolo[2,3-b]pyridin-3-yl)-2-methylphenyl)acrylamide |
| **73** | 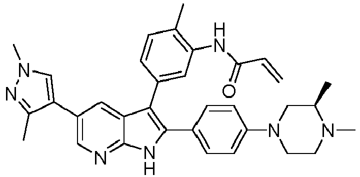 | (R)-N-(5-(5-(1,3-dimethyl-1 H-pyrazol-4-yl)-2-(4-(3,4-dimethylpiperazin-1-yl)phenyl)-1 H-pyrrolo[2,3-b]pyridin-3-yl)-2-methylphenyl)acrylamide |
| **74** | 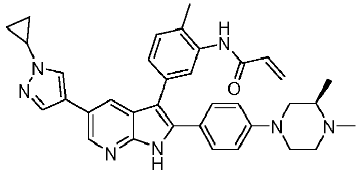 | (R)-N-(5-(5-(1-cyclopropyl-1 H-pyrazol-4-yl)-2-(4-(3,4-dimethylpiperazin-1-yl)phenyl)-1 H-pyrrolo[2,3-b]pyridin-3-yl)-2-methylphenyl)acrylamide |
| **75** | 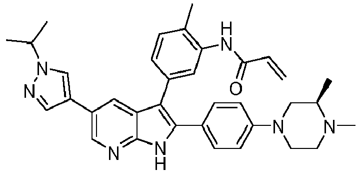 | (R)-N-(5-(2-(4-(3,4-dimethylpiperazin-1-yl)phenyl)-5-(1-isopropyl-1 H-pyrazol-4-yl)-1 H-pyrrolo[2,3-b]pyridin-3-yl)-2-methylphenyl)acrylamide |
| **76** | 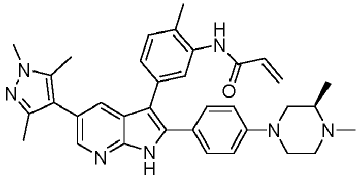 | (R)-N-(5-(2-(4-(3,4-dimethylpiperazin-1-yl)phenyl)-5-(1,3,5-trimethyl-1 H-pyrazol-4-yl)-1 H-pyrrolo[2,3-b]pyridin-3-yl)-2-methylphenyl)acrylamide |
| **77** | 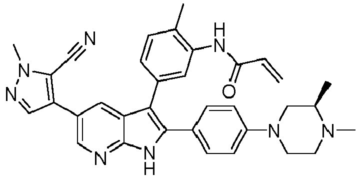 | (R)-N-(5-(5-(5-cyano-1-methyl-1 H-pyrazol-4-yl)-2-(4-(3,4-dimethylpiperazin-1-yl)phenyl)-1 H-pyrrolo[2,3-b]pyridin-3-yl)-2-methylphenyl)acrylamide |
| **78** | 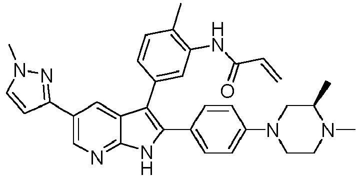 | (R)-N-(5-(2-(4-(3,4-dimethylpiperazin-1-yl)phenyl)-5-(1-methyl-1 H-pyrazol-3-yl)-1 H-pyrrolo[2,3-b]pyridin-3-yl)-2-methylphenyl)acrylamide |
| **79** | 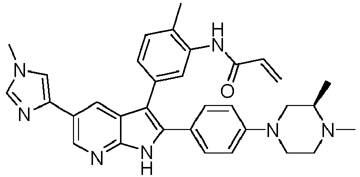 | (R)-N-(5-(2-(4-(3,4-dimethylpiperazin-1-yl)phenyl)-5-(1-methyl-1 H-imidazol-4-yl)-1 H-pyrrolo[2,3-b]pyridin-3-yl)-2-methylphenyl)acrylamide |
| **80** | 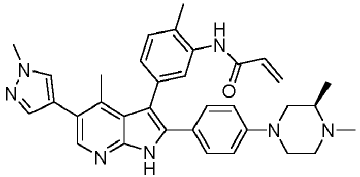 | (R)-N-(5-(2-(4-(3,4-dimethylpiperazin-1-yl)phenyl)-4-methyl-5-(1-methyl-1 H-pyrazol-4-yl)-1 H-pyrrolo[2,3-b]pyridin-3-yl)-2-methylphenyl)acrylamide |
| **81** | 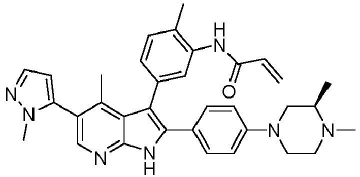 | (R)-N-(5-(2-(4-(3,4-dimethylpiperazin-1-yl)phenyl)-4-methyl-5-(1-methyl-1 H-pyrazol-5-yl)-1 H-pyrrolo[2,3-b]pyridin-3-yl)-2-methylphenyl)acrylamide |
| **82** | 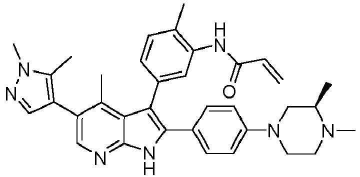 | (R)-N-(5-(5-(1,5-dimethyl-1 H-pyrazol-4-yl)-2-(4-(3,4-dimethylpiperazin-1-yl)phenyl)-4-methyl-1 H-pyrrolo[2,3-b]pyridin-3-yl)-2-methylphenyl)acrylamide |
| **83** | 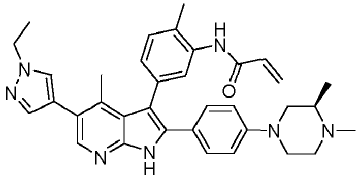 | (R)-N-(5-(2-(4-(3,4-dimethylpiperazin-1-yl)phenyl)-5-(1-ethyl-1 H-pyrazol-4-yl)-4-methyl-1 H-pyrrolo[2,3-b]pyridin-3-yl)-2-methylphenyl)acrylamide |
| **84** | 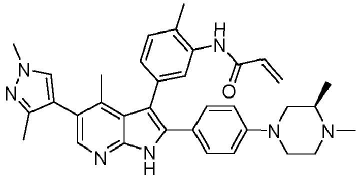 | (R)-N-(5-(5-(1,3-dimethyl-1 H-pyrazol-4-yl)-2-(4-(3,4-dimethylpiperazin-1-yl)phenyl)-4-methyl-1 H-pyrrolo[2,3-b]pyridin-3-yl)-2-methylphenyl)acrylamide |
| **85** | 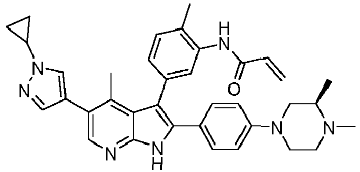 | (R)-N-(5-(5-(1-cyclopropyl-1 H-pyrazol-4-yl)-2-(4-(3,4-dimethylpiperazin-1-yl)phenyl)-4-methyl-1 H-pyrrolo[2,3-b]pyridin-3-yl)-2-methylphenyl)acrylamide |
| **86** | 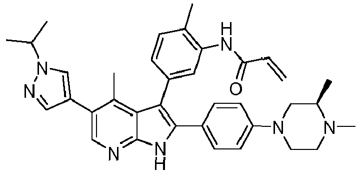 | (R)-N-(5-(2-(4-(3,4-dimethylpiperazin-1-yl)phenyl)-5-(1-isopropyl-1 H-pyrazol-4-yl)-4-methyl-1 H-pyrrolo[2,3-b]pyridin-3-yl)-2-methylphenyl)acrylamide |
| **87** | 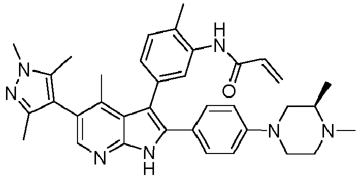 | (R)-N-(5-(2-(4-(3,4-dimethylpiperazin-1-yl)phenyl)-4-methyl-5-(1,3,5-trimethyl-1 H-pyrazol-4-yl)-1 H-pyrrolo[2,3-b]pyridin-3-yl)-2-methylphenyl)acrylamide |
| **88** | 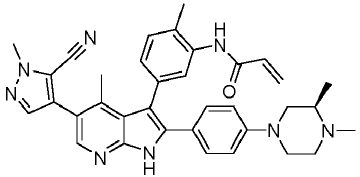 | (R)-N-(5-(5-(5-cyano-1-methyl-1 H-pyrazol-4-yl)-2-(4-(3,4-dimethylpiperazin-1-yl)phenyl)-4-methyl-1 H-pyrrolo[2,3-b]pyridin-3-yl)-2-methylphenyl)acrylamide |
| **89** | 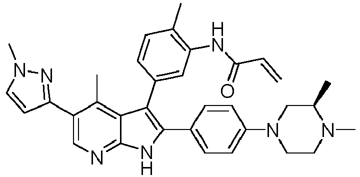 | (R)-N-(5-(2-(4-(3,4-dimethylpiperazin-1-yl)phenyl)-4-methyl-5-(1-methyl-1 H-pyrazol-3-yl)-1 H-pyrrolo[2,3-b]pyridin-3-yl)-2-methylphenyl)acrylamide |
| **90** | 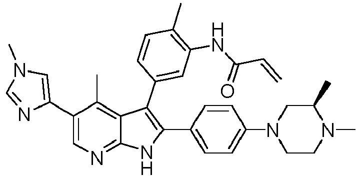 | (R)-N-(5-(2-(4-(3,4-dimethylpiperazin-1-yl)phenyl)-4-methyl-5-(1-methyl-1 H-imidazol-4-yl)-1 H-pyrrolo[2,3-b]pyridin-3-yl)-2-methylphenyl)acrylamide |
| **91** | 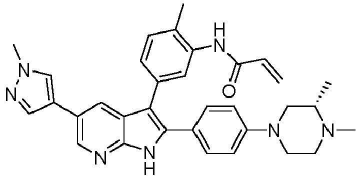 | (S)-N-(5-(2-(4-(3,4-dimethylpiperazin-1-yl)phenyl)-5-(1-methyl-1 H-pyrazol-4-yl)-1 H-pyrrolo[2,3-b]pyridin-3-yl)-2-methylphenyl)acrylamide |
| **92** | 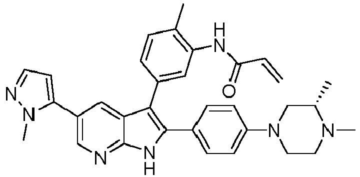 | (S)-N-(5-(2-(4-(3,4-dimethylpiperazin-1-yl)phenyl)-5-(1-methyl-1 H-pyrazol-5-yl)-1 H-pyrrolo[2,3-b]pyridin-3-yl)-2-methylphenyl)acrylamide |
| **93** | 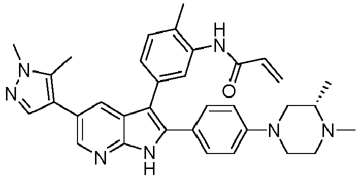 | (S)-N-(5-(5-(1,5-dimethyl-1 H-pyrazol-4-yl)-2-(4-(3,4-dimethylpiperazin-1-yl)phenyl)-1 H-pyrrolo[2,3-b]pyridin-3-yl)-2-methylphenyl)acrylamide |
| **94** | 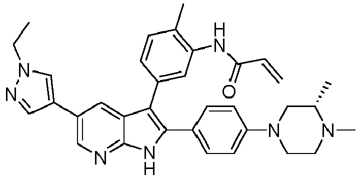 | (S)-N-(5-(2-(4-(3,4-dimethylpiperazin-1-yl)phenyl)-5-(1-ethyl-1 H-pyrazol-4-yl)-1 H-pyrrolo[2,3-b]pyridin-3-yl)-2-methylphenyl)acrylamide |
| **95** | 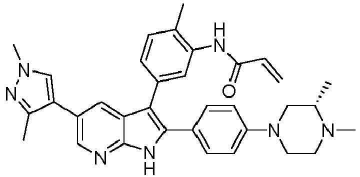 | (S)-N-(5-(5-(1,3-dimethyl-1 H-pyrazol-4-yl)-2-(4-(3,4-dimethylpiperazin-1-yl)phenyl)-1 H-pyrrolo[2,3-b]pyridin-3-yl)-2-methylphenyl)acrylamide |
| **96** | 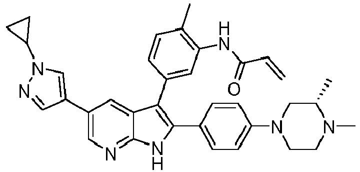 | (S)-N-(5-(5-(1-cyclopropyl-1 H-pyrazol-4-yl)-2-(4-(3,4-dimethylpiperazin-1-yl)phenyl)-1 H-pyrrolo[2,3-b]pyridin-3-yl)-2-methylphenyl)acrylamide |
| **97** | 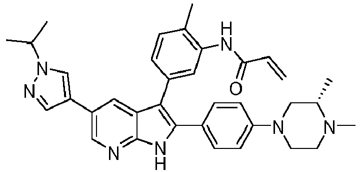 | (S)-N-(5-(2-(4-(3,4-dimethylpiperazin-1-yl)phenyl)-5-(1-isopropyl-1 H-pyrazol-4-yl)-1 H-pyrrolo[2,3-b]pyridin-3-yl)-2-methylphenyl)acrylamide |
| **98** | 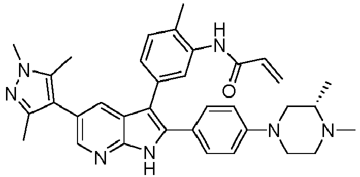 | (S)-N-(5-(2-(4-(3,4-dimethylpiperazin-1-yl)phenyl)-5-(1,3,5-trimethyl-1 H-pyrazol-4-yl)-1 H-pyrrolo[2,3-b]pyridin-3-yl)-2-methylphenyl)acrylamide |
| **99** | 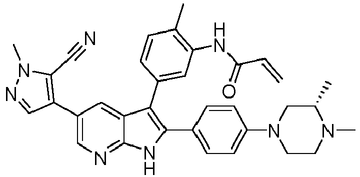 | (S)-N-(5-(5-(5-cyano-1-methyl-1 H-pyrazol-4-yl)-2-(4-(3,4-dimethylpiperazin-1-yl)phenyl)-1 H-pyrrolo[2,3-b]pyridin-3-yl)-2-methylphenyl)acrylamide |
| **100** | 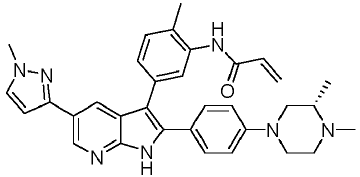 | (S)-N-(5-(2-(4-(3,4-dimethylpiperazin-1-yl)phenyl)-5-(1-methyl-1H-pyrazol-3-yl)-1H-pyrrolo[2,3-b]pyridin-3-yl)-2-methylphenyl)acrylamide |
| **101** | 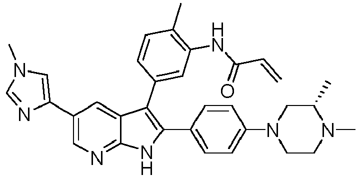 | (S)-N-(5-(2-(4-(3,4-dimethylpiperazin-1-yl)phenyl)-5-(1-methyl-1H-imidazol-4-yl)-1H-pyrrolo[2,3-b]pyridin-3-yl)-2-methylphenyl)acrylamide |
| **102** | 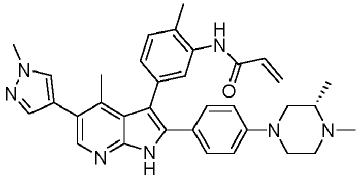 | (S)-N-(5-(2-(4-(3,4-dimethylpiperazin-1-yl)phenyl)-4-methyl-5-(1-methyl-1 H-pyrazol-4-yl)-1 H-pyrrolo[2,3-b]pyridin-3-yl)-2-methylphenyl)acrylamide |
| **103** | 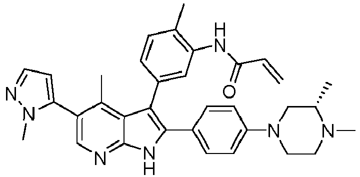 | (S)-N-(5-(2-(4-(3,4-dimethylpiperazin-1-yl)phenyl)-4-methyl-5-(1-methyl-1H-pyrazol-5-yl)-1H-pyrrolo[2,3-b]pyridin-3-yl)-2-methylphenyl)acrylamide |
| **104** | 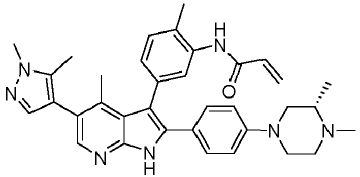 | (S)-N-(5-(5-(1,5-dimethyl-1 H-pyrazol-4-yl)-2-(4-(3,4-dimethylpiperazin-1-yl)phenyl)-4-methyl-1 H-pyrrolo[2,3-b]pyridin-3-yl)-2-methylphenyl)acrylamide |
| **105** | 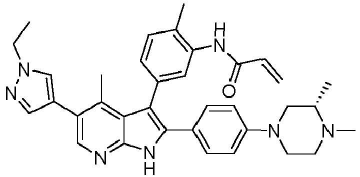 | (S)-N-(5-(2-(4-(3,4-dimethylpiperazin-1-yl)phenyl)-5-(1-ethyl-1 H-pyrazol-4-yl)-4-methyl-1 H-pyrrolo[2,3-b]pyridin-3-yl)-2-methylphenyl)acrylamide |
| **106** | 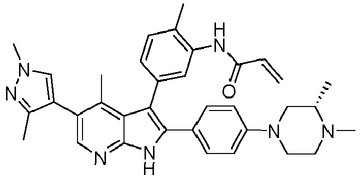 | (S)-N-(5-(5-(1,3-dimethyl-1 H-pyrazol-4-yl)-2-(4-(3,4-dimethylpiperazin-1-yl)phenyl)-4-methyl-1 H-pyrrolo[2,3-b]pyridin-3-yl)-2-methylphenyl)acrylamide |
| **107** | 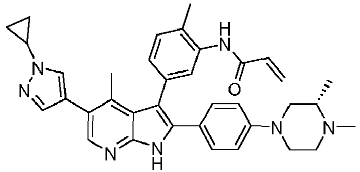 | (S)-N-(5-(5-(1-cyclopropyl-1 H-pyrazol-4-yl)-2-(4-(3,4-dimethylpiperazin-1-yl)phenyl)-4-methyl-1 H-pyrrolo[2,3-b]pyridin-3-yl)-2-methylphenyl)acrylamide |
| **108** | 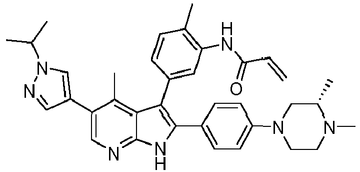 | (S)-N-(5-(2-(4-(3,4-dimethylpiperazin-1-yl)phenyl)-5-(1-isopropyl-1H-pyrazol-4-yl)-4-methyl-1H-pyrrolo[2,3-b]pyridin-3-yl)-2-methylphenyl)acrylamide |
| **109** | 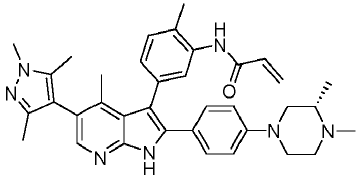 | (S)-N-(5-(2-(4-(3,4-dimethylpiperazin-1-yl)phenyl)-4-methyl-5-(1,3,5-trimethyl-1H-pyrazol-4-yl)-1H-pyrrolo[2,3-b]pyridin-3-yl)-2-methylphenyl)acrylamide |
| **110** | 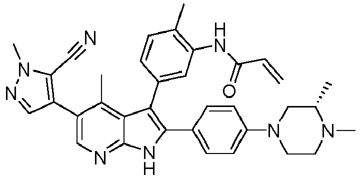 | (S)-N-(5-(5-(5-cyano-1-methyl-1H-pyrazol-4-yl)-2-(4-(3,4-dimethylpiperazin-1-yl)phenyl)-4-methyl-1H-pyrrolo[2,3-b]pyridin-3-yl)-2-methylphenyl)acrylamide |
| **111** | 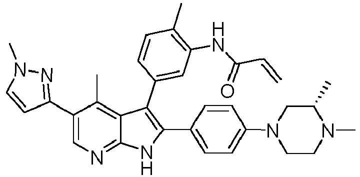 | (S)-N-(5-(2-(4-(3,4-dimethylpiperazin-1-yl)phenyl)-4-methyl-5-(1-methyl-1H-pyrazol-3-yl)-1H-pyrrolo[2,3-b]pyridin-3-yl)-2-methylphenyl)acrylamide |
| **112** | 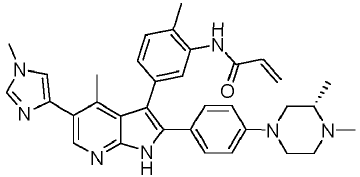 | (S)-N-(5-(2-(4-(3,4-dimethylpiperazin-1-yl)phenyl)-4-methyl-5-(1-methyl-1 H-imidazol-4-yl)-1 H-pyrrolo[2,3-b]pyridin-3-yl)-2-methylphenyl)acrylamide |
| **113** | 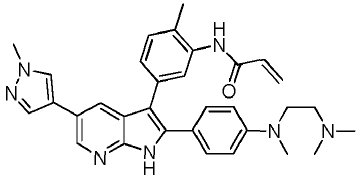 | N-(5-(2-(4-((2-(dimethylamino)ethyl)(methyl)a mino)phenyl)-5-(1-methyl-1H-pyrazol-4-yl)-1H-pyrrolo[2,3-b]pyridin-3-yl)-2-methylphenyl)acrylamide |
| **114** | 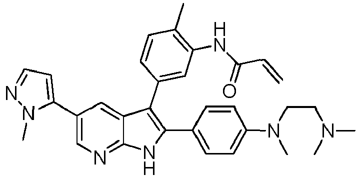 | N-(5-(2-(4-((2-(dimethylamino)ethyl)(methyl)a mino)phenyl)-5-(1-methyl-1H-pyrazol-5-yl)-1H-pyrrolo[2,3-b]pyridin-3-yl)-2-methylphenyl)acrylamide |
| **115** | 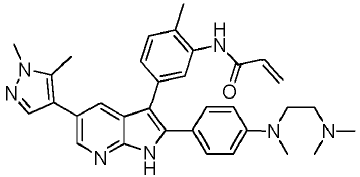 | N-(5-(5-(1,5-dimethyl-1H-pyrazol-4-yl)-2-(4-((2-(dimethylamino)ethyl)(methyl)a mino)phenyl)-1H-pyrrolo[2,3-b]pyridin-3-yl)-2-methylphenyl)acrylamide |
| **116** | 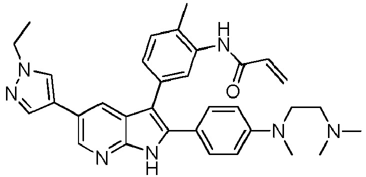 | N-(5-(2-(4-((2-(dimethylamino)ethyl)(methyl)a mino)phenyl)-5-(1-ethyl-1H-pyrazol-4-yl)-1H-pyrrolo[2,3-b]pyridin-3-yl)-2-methylphenyl)acrylamide |
| **117** | 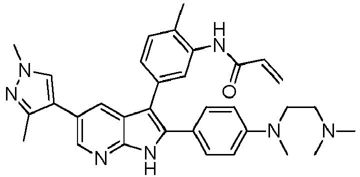 | N-(5-(5-(1,3-dimethyl-1H-pyrazol-4-yl)-2-(4-((2-(dimethylamino)ethyl)(methyl)a mino)phenyl)-1H-pyrrolo[2,3-b]pyridin-3-yl)-2-methylphenyl)acrylamide |
| **118** | 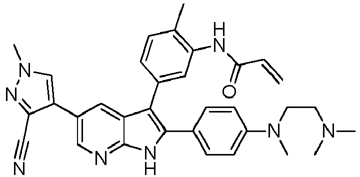 | N-(5-(5-(3-cyano-1-methyl-1H-pyrazol-4-yl)-2-(4-((2-(dimethylamino)ethyl)(methyl)a mino)phenyl)-1H-pyrrolo[2,3-b]pyridin-3-yl)-2-methylphenyl)acrylamide |
| **119** | 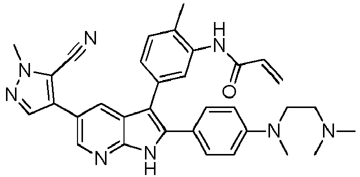 | N-(5-(5-(5-cyano-1-methyl-1H-pyrazol-4-yl)-2-(4-((2-(dimethylamino)ethyl)(methyl)a mino)phenyl)-1 H-pyrrolo[2,3-b]pyridin-3-yl)-2-methylphenyl)acrylamide |
| **120** | 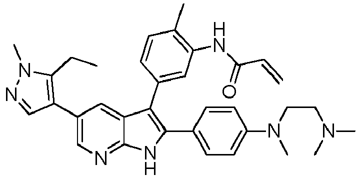 | N-(5-(2-(4-((2-(dimethylamino)ethyl)(methyl)a mino)phenyl)-5-(5-ethyl-1-methyl-1H-pyrazol-4-yl)-1H-pyrrolo[2,3-b]pyridin-3-yl)-2-methylphenyl)acrylamide |

or an enantiomer, a mixture of enantiomers, a diastereomer, a mixture of diastereomers, a tautomer, a prodrug, a hydrate, a solvate of the above mentioned compounds, or pharmaceutically acceptable salts thereof.

The expression prodrug is defined as a substance, which is applied in an inactive or significantly less active form. Once applied and incorporated, the prodrug is metabolized in the body *in vivo* into the active compound. Conventional procedures for the selection and preparation of suitable prodrug derivatives are described, for example in "Design of Prodrugs", ed. H. B. Bundgaard, Elsevier, 1985.

The present invention also includes within its scope N-oxides of the compounds of formula (I) above. In general, such N-oxides may be formed by conventional means, such as reacting the compound of formula (I) with oxone in the presence of wet alumina.

The expression tautomer is defined as an organic compound that is interconvertible by a chemical reaction called tautomerization. Tautomerization can be catalyzed preferably by bases or acids or other suitable compounds.

The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, D-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form of the compounds of formula (I) with a sufficient amount of the desired acid to produce a salt in the conventional manner well known to those skilled in the art.

The inventive compounds may exist in a number of different polymorphic forms.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

The inventors found that preferred inhibitors should have a substituent at the 2-position of the phenyl ring next to the warhead. The warhead is most preferably a but-2-enamide. The position 2 on the phenyl ring next to the warhead should be different from hydrogen and is preferably an alkyl or alkylenyl residue such as a methyl group or a fluoro substituent (like in compounds No. 50 - 60) or can be part of a ring system preferably containing the nitrogen atom of the warhead (like in compounds No. 61 - 64). Moreover it was found that an aromatic nitrogen heterocyclic 5-membered ring should be present at position 5 of the pyrrolo[2,3-b]pyridine in order to achieve the desired inhibitory activity. This N-heterocyclic aromatic ring should most preferably contain two nitrogen atoms. The nitrogen atom not attached to the carbon atom linked to the pyrrolo[2,3-b]pyridine moiety should preferably be substituted in order to obtain quite potent inhibitors. Especially preferred is the pyrazol ring and even more preferred a pyrazol-4-yl ring at position 5 of the pyrrolo[2,3-b]pyridine moiety.

### Syntheses of the compounds

The compound of formula (I) is prepared by reference to the methods illustrated by the following reaction protocols. The compound of formula (I) is produced as outlined below by the suitable selection of reagents with appropriate substitution. Solvents, temperatures, pressures, and other reaction conditions may readily be selected by one of ordinary skill in the art. Starting materials are commercially available or readily prepared by one of ordinary skill in the art.

### Procedure A

The present invention is also directed to a method for producing the compounds of formula **(I),** the method comprises the following steps in the row as described below:
**Step A1:** perfoming a first cross coupling reaction of pyridine compound **1*** with alkyne compound **2a*** 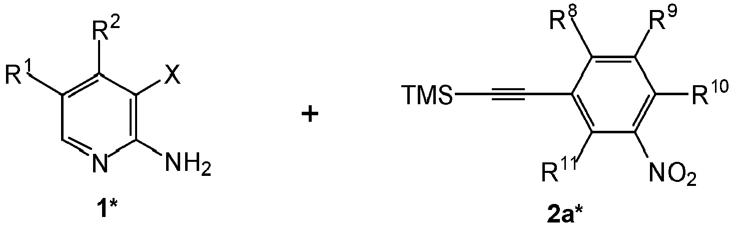 wherein the substituents **R¹, R²** and **R⁸** - **R¹¹** have the meanings as defined in claim 1 and X is a leaving group and represents Cl, Br, I, or OTf,
   to obtain a compound **3*** 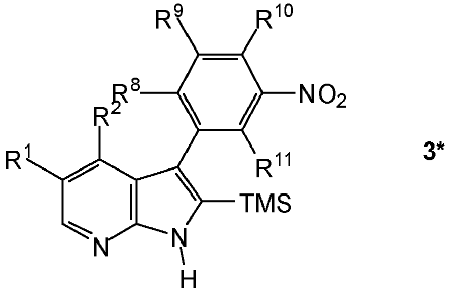 in the presence of a first palladium catalyst, and a first base;
**Step B1:** converting a trimethylsilyl group of the compound **3*** to a halide like an iodide to obtain a compound **4*** 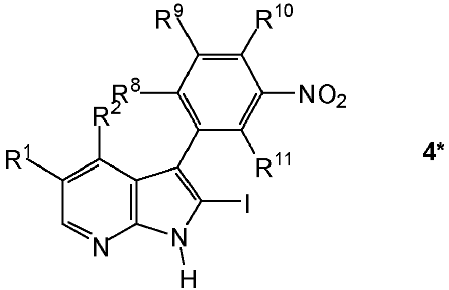
**Step C1:** perfoming a second cross coupling reaction of **4*** with a compound **5*** 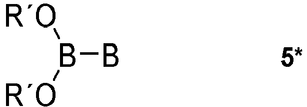 wherein R' is H or an alkyl chain with 1-10 carbon atoms or a cycloalkyl chain with 3 to 12 carbon atoms or both residues R' represent together a residue derived from pinacol, in the presence of a second palladium catalyst, and a second base to obtain a compound **6*** 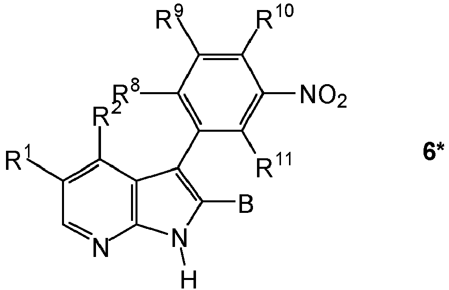
**Step D1:** reducing nitro (NO₂) group of the compound **6*** to a primary amine (NH₂) group to obtain a compound **7*;** and
**Step E1:** perfoming a coupling reaction of the compound **7*** 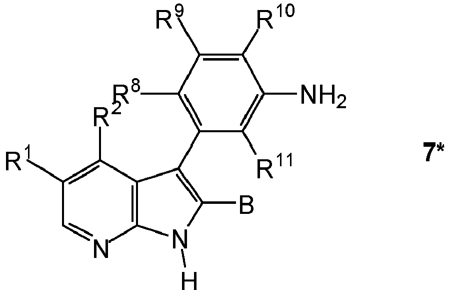
with a compound HO-**R⁶** or AG-**R⁶**, wherein the substituent **R⁶** has the meanings as defined in claim 1 and AG is an activating group of a carboxylic acid,
to obtain a product compound of the general formula **(I)**

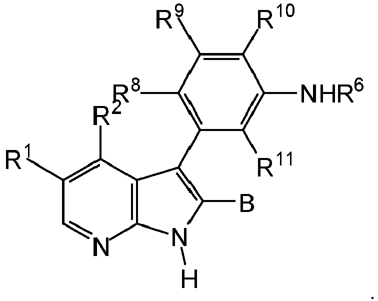

Thus, Step A (e.g. Step A1, Step A3 or A4) comprises performing the Sonogashira reaction followed by cyclisation with, for instance, Pd⁰, (CuI), base and enhanced temperature. The iodination in Step B (e.g. Step B1, Step B2, Step B3 or B4) is carried out by means of NIS (N-iodosuccinimide) and Step C (e.g. Step C1, Step C2, Step C3 or C4) is a Suzuki-coupling with compound 5*, Pd⁰, base and enhanced temperature. Reduction of the nitro group to the amino group in Step D (e.g. Step D1 or D2) is achieved by use of Fe or H₂ + Pd/C and finally Step E (e.g. Step E1, Step E2, Step E3 or E4) is a coupling reaction with HO-**R⁶** or AG-**R⁶** and base.

### Procedure B

Furthermore, the present invention is directed to a method for producing the compounds of formula (I), the method comprises the following steps in the row as described below:
**Step A1:** perfoming a first cross coupling reaction of pyridine compound **1*** with alkyne compound **2a*** 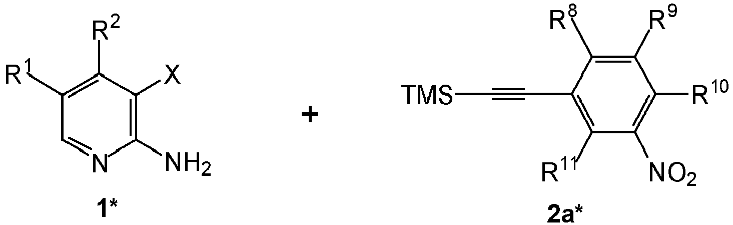 wherein the substituents **R¹, R²** and **R⁸** - **R¹¹** have the meanings as defined in claim 1 and X is a leaving group and represents Cl, Br, I, or OTf,
   to obtain a compound **3*** 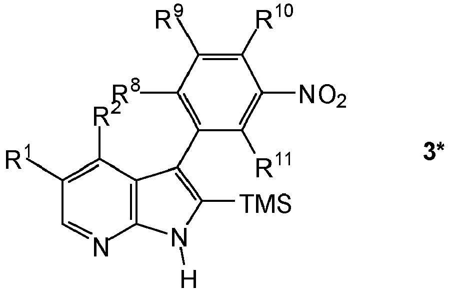 in the presence of a first palladium catalyst, and a first base;
**Step D2:** reducing nitro (NO₂) group of the compound **3*** to a primary amine (NH₂) group to obtain a compound **10*** 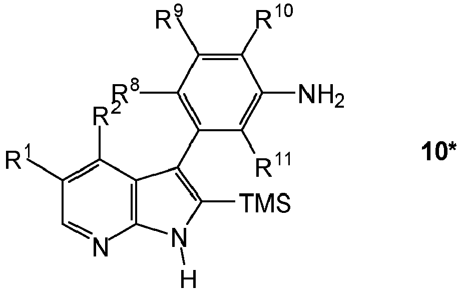
**Step E2:** perfoming a coupling reaction of the compound **10*** with a compound HO-**R⁶** or AG-**R⁶**, wherein the substituent **R⁶** has the meanings as defined in claim 1 and AG is an activating group of a carboxylic acid,
   to obtain a compound **11*** 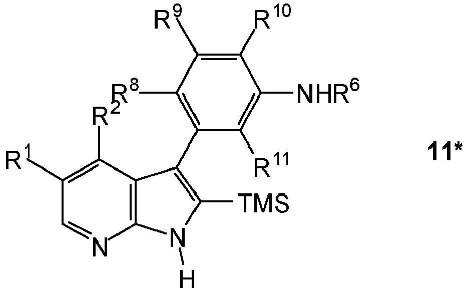
**Step B2:** converting a trimethylsilyl group of the compound **11*** to a halide like an iodide to obtain a compound **12*** 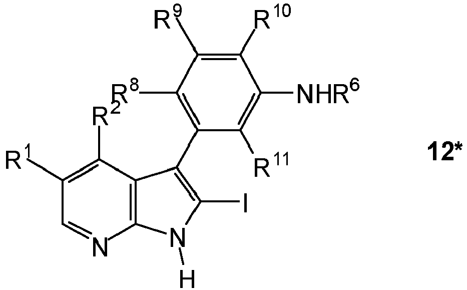
**Step C2:** perfoming a second cross coupling reaction of the compound **12*** with a compound **5*** 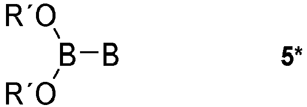 wherein R' is H or an alkyl chain with 1-10 carbon atoms or a cycloalkyl chain with 3 to 12 carbon atoms or both residues R' represent together a residue derived from pinacol, in the presence of a second palladium catalyst, and a second base to obtain a product compound of the formula (**I**)

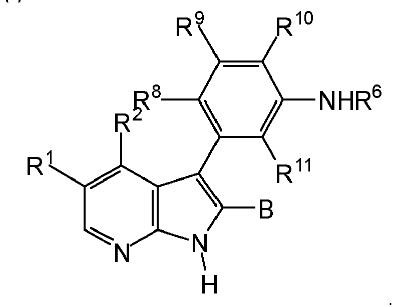

### Procedure C

Furthermore, the present invention is directed to a method for producing the compounds of formula **(I),** the method comprises the following steps in the row as described below:
**Step A3:**
   i) perfoming a first cross coupling reaction of pyridine compound **1*** with alkyne compound **2b*** 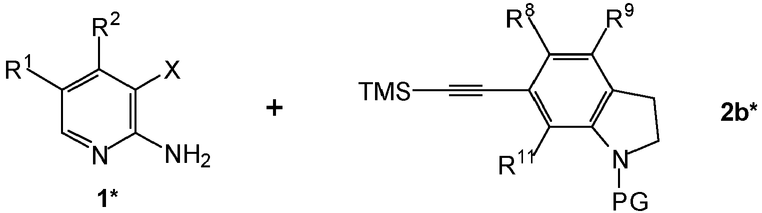 wherein the substituents **R¹, R², R⁸, R⁹, R¹¹,** and **R^{a}** - **R^{d}** have the meanings as defined in claim 1 and X is a leaving group and represents Cl, Br, I, or OTf and PG is an amino protecting group,
      in the presence of a first palladium catalyst, and a first base; and
   ii) removing a protecting group PG of a resulting compound after the step i) to obtain a compound **3b*** 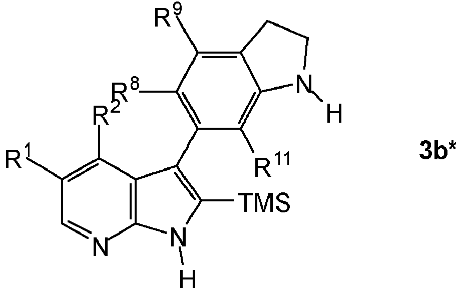
**Step E3:** perfoming a coupling reaction of the compound **3b*** with a compound HO-**R⁶** or AG-**R⁶**, wherein the substituent **R⁶** has the meanings as defined in claim 1 and AG is an activating group of a carboxylic acid,
   to obtain a compound **11b*** 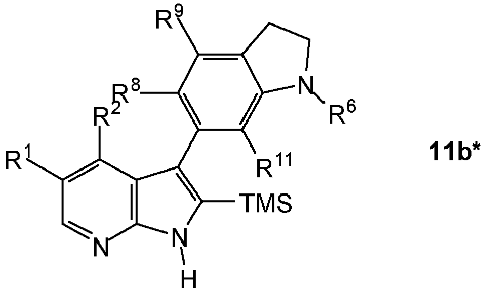
**Step B3:** converting a trimethylsilyl group of the compound **11b*** to a halide like an iodide to obtain a compound **12b*** 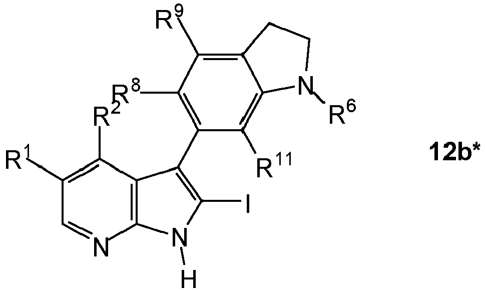
**Step C3:** perfoming a second cross coupling reaction of the compound **12b*** with a compound **5*** 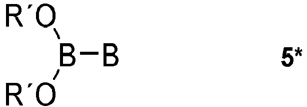 wherein R' is H or an alkyl chain with 1-10 carbon atoms or a cycloalkyl chain with 3 to 12 carbon atoms or both residues R' represent together a residue derived from pinacol, in the presence of a second palladium catalyst, and a second base to obtain a product compound of the formula **(I)**

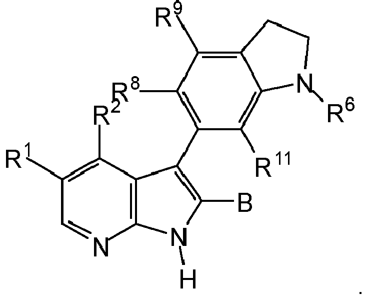

### Procedure D

Furthermore, the present invention is directed to a method for producing the compounds of formula **(I),** the method comprises the following steps in the row as described below:
**Step A4:**
   i) perfoming a first cross coupling reaction of pyridine compound **1*** with alkyne compound **2c*** 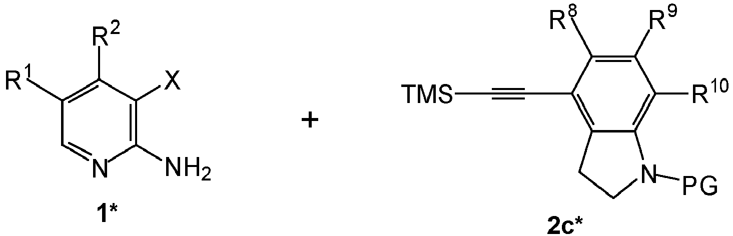 wherein the substituents **R¹, R², R⁸, R⁹, R¹¹,** and **R^{a}** - **R^{d}** have the meanings as defined in claim 1 and X is a leaving group and represents Cl, Br, I, or OTf and PG is an amino protecting group,
      in the presence of a first palladium catalyst, and a first base; and
   ii) removing a protecting group PG of a resulting compound after the step i) to obtain a compound **3c*** 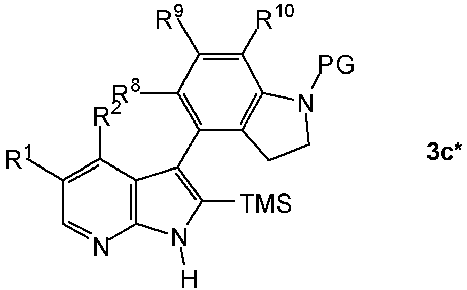
**Step E4:** perfoming a coupling reaction of the compound **3c*** with a compound HO-**R⁶** or AG-**R⁶**, wherein the substituent **R⁶** has the meanings as defined in claim 1 and AG is an activating group of a carboxylic acid,
   to obtain a compound **11c*** 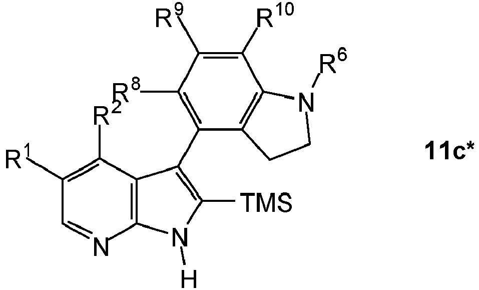
**Step** B4: converting a trimethylsilyl group of the compound **11c^{*}** to a halide like an iodide to obtain a compound **12c*** 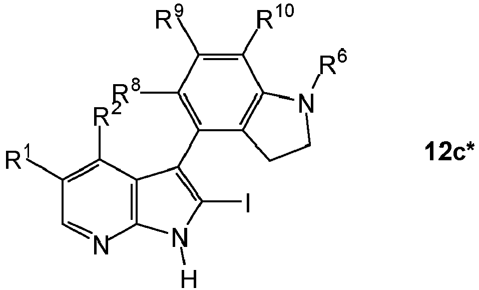
**Step C4:** perfoming a second cross coupling reaction of the compound **12c*** with a compound **5*** 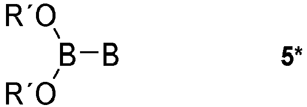 wherein R' is H or an alkyl chain with 1-10 carbon atoms or a cycloalkyl chain with 3 to 12 carbon atoms or both residues R' represent together a residue derived from pinacol, in the presence of a second palladium catalyst, and a second base to obtain a product compound of the formula **(I)**

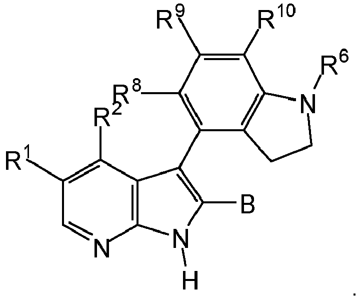

In each of the steps **A1, A3,** and **A4** Sonogashia coupling reaction of compound **1*** and domino cyclization of the resulting coupling compound is performed in the present of the first palladium catalyst, and the base. Optionally, copper catalyst may be used as cocatalyst and is preferred Cu(I) and more preferred CuI.

The first palladium catalyst for C-C coupling reaction is Pd(0) or Pd(II) catalyst, preferred PdCl₂, Pd(PPh₃)₄, Pd(acac)₂, PdCl₂(CH₃CN)₂, PdCl₂(PCy₃)₂, PdCl₂(PPh₃)₂, [(π-ally)PdCl]₂, (SIPr)PdCl₂(TEA). Optionally, further phosphine ligand may be used together with the first palladium catalyst.
A ratio of the palladium catalyst to the starting material is in the range of 0.01 to 20 mol-%, preferred 0.01-10 mol-%, more preferred 0.01-5 mol-%, most preferred 0.01-1 mol-%.
The first base may be an organic base or inorganic bases. The organic base may be tertiary amine such as Et₃N, and DIPEA, DABCO, DBU, pyrrolidine or piperidine. The inorganic base may be K₂CO₃, Cs₂CO₃ or K₃PO₄. A ratio of the first base to the starting material is in the range of 1.0 to 5.0 equivalents, preferred 1.0 to 3.0 equivalents, more preferred 1.0 to 3.0 equivalents, most preferred 1.0 to 1.5 equivalents.
Preferred, this reaction is performed in a polar aprotic solvent such as DMF or DMSO under N₂ atmosphere at a temperature in a range of 80°C to 200°C, preferred 100°C to 180°C, more preferred 100°C to 150°C, most preferred 120°C to 150°C.

In each of the steps **B1, B2, B3,** and **B4,** conversion of trimethylsilyl (TMS) group to iodide group is performed by treating with N-iodosuccinimide (NIS) as an idonation reagent for 15 h at a temperature in a range of 10°C to 35°C, preferred, 15°C to 30°C, more preferred 20°C to 30°C. A polar aprotic solvent such as dichlorormethane or chlororform is used.

In each of the steps **C1, C2, C3,** and **C4** Suzuki coupling reaction of iodinated or brominated compound **4*, 12*, 12b*,** or **12c*** with a compound **5*** as a boronic acid derivative is performed in the present in the second palladium catalyst and the second base.

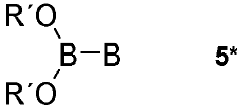

In compound **5*** the two substituents R' are H or an alkyl chain with 1-10 carbon atoms or a cycloalkyl chain with 3 to 12 carbon atoms or both residues R' represent together a residue derived from pinacol. Preferably, the boronic acid derivative may be a boronic acid (R' = -H) or an ester of the boronic acid, e.g. its isopropyl ester (R' = -CH(CH₃)₂), an ester derived from pinacol (R'-R' = -C(CH₃)₂-C(CH₃)₂-).

The second palladium catalyst is Pd(0) or Pd(II) catalyst. The Pd(0) catalyst may be tetrakis(triphenylphosphine)palladium(0) [Pd(PPh₃)₄], tris(dibenzylideneacetone)di-palladium(0) [Pd₂(dba)₃]. Pd(II) catalyst may be dichlorobis(triphenylphosphine)-palladium(II) [Pd(PPh₃)₂Cl₂], palladium(II) acetate and triphenylphosphine or more preferred [1,1'-bis(diphenylphosphino)ferrocene]palladium dichloride. The reaction is preferably carried out in a mixture of a solvent like dioxane, DMF, DME, THF, or isopropanol with water and in the presence of the second base like aqueous sodium bicarbonate or K₃PO₄.

In each of the Steps **D1** and **D2** a nitro (NO₂) group is reduced to a primary amine (NH₂) group with the treatment of a reducing agent. Preferably, Fe, or Pd/H₂ is used as a reducing agent.

In each of the Steps **E1, E2, E3,** and **E4** group is introduced by a coupling reaction with HO-**R⁶** or AG-**R⁶**.

For perfoming the coupling reaction, firstly carboxylic acid or sulfonic acid group of HO-R⁶ is activated *in situ* to promote the coupling reaction with amino group of intermediate compound. If HO-R⁶ is a carboxylic acid, the activating group (AG) of carboxylic acid may be introduced *in situ* reaction. Preferably, the activating group (AG) may be selected from the group consisting of or comprising: halides such as -F, -Br, -CI, -I, anhydride group such as -OCOCH₃, N-oxy-benzotriazol group and N-oxy-succinimide.

Further, a carboxylic acid of HO-**R⁶** is coupled with the amine group of intermediate compound by a well-known amide coupling reaction. Any of the following coupling reagent can be used for amide coupling reaction: BOP, PyBOP, AOP, PyAOP, TBTU, EEDQ, Polyphosphoric Acid (PPA), DPPA, HATU, HOBt, HOAt, DCC, EDCI, BOP-Cl, TFFH, Brop, PyBrop, and CIP.

For perfoming the coupling reaction, also an activated compound AG-R⁶ having activated carboxyl sulfony group can be used.

As above described, the activating group (AG) may be selected from the group consisting of or comprising: halides such as -F, -Br, -CI, -I, anhydride group such as -OCOCH₃, N-oxy-benzotriazol group and N-oxy-succinimide, preferably AG is Cl.

In the Step **A3, A4,** and **E4,** a protecting group (PG) is an amino protecting group. The amine protecting group may be t-butyloxycarbonyl (Boc) or benzyloxycarbonyl (Cbz) and removed under acidic condition, for example, treating with HCl, or TFA.

The structure activity relationship (SAR) of the compounds of the present invention shows covalent inhibitors as cellular potent mutant-selective ErbB inhibitors. The covalent binding mode, obtained for example through the introduction of an acrylic moiety, is crucial for cellular activity.

It was found that the compound of the present invention is useful for the prophylaxis and/or the treatment of cancer having activating mutation of a receptor belonging to the ErbB family of receptor, preferred, the mutation is an insertion within exon 20 of EGFR or within exon 20 of HER2.
The compound of the present invention is a selective inhibitor of mutants of EGFR and Her2, preferred Exon 20 mutations as shown in **Table 3.**

Thus, the compound of the present invention is useful as a medicament or as pharmaceutically active agent. The compound of the present invention is useful for the prophylaxis and/or the treatment of cell proliferative diseases, especially cancer.

Said cancer is selected from breast cancer, colon cancer, prostate cancer, lung cancer, gastric cancer, ovarian cancer, endometrial cancer, renal cancer, hepatocellular cancer, thyroid cancer, uterine cancer, esophagus cancer, squamous cell cancer, leukemia, lymphoma, osteosarcoma, mamma carcinoma, melanoma, glioblastoma and neuroblastoma.

In specific embodiment, the compound is useful for in the the prophylaxis and/or the treatment of non small cell lung cancer (NSCLC) or mamma carcinoma.

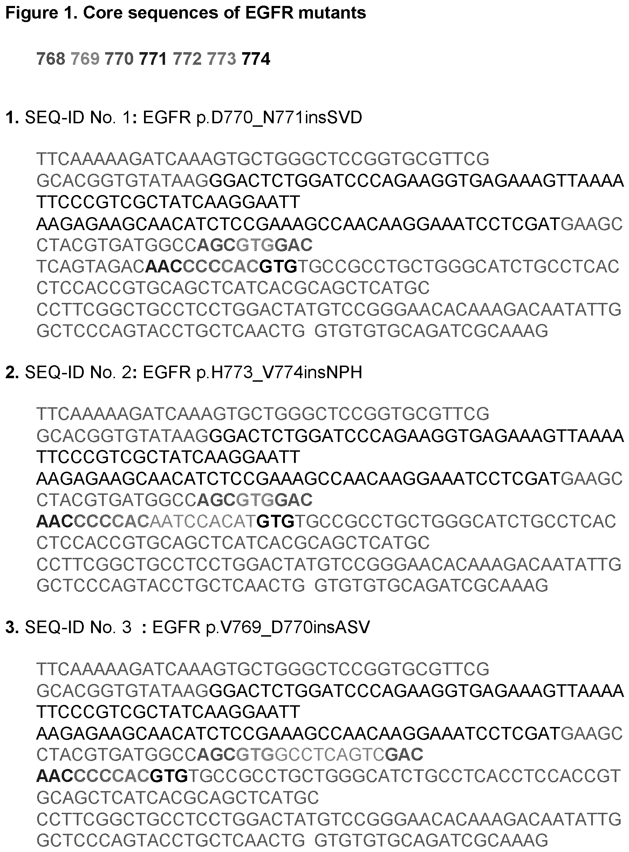

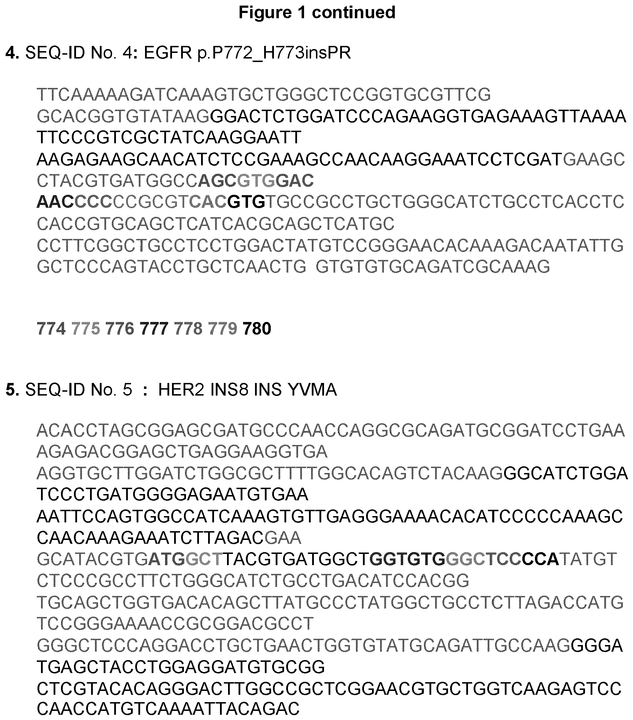

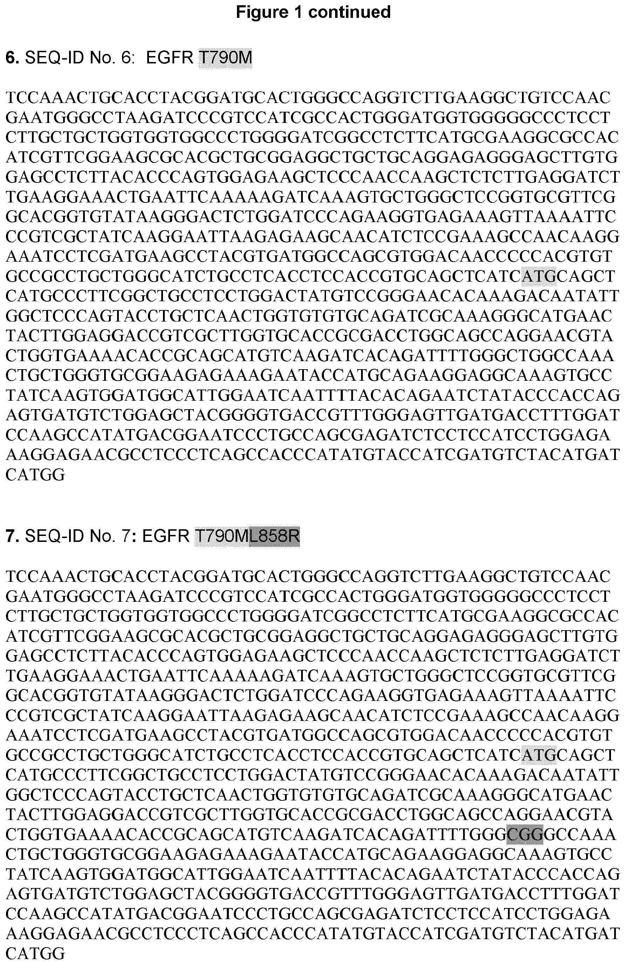

Preferably, the mutation is an insertion within exon 20 of EGFR or within exon 20 of HER2. One aspect herein are the compounds of the present invention for use in the prophylaxis and/or the treatment of cancer caused by or associated with mutations associated with EGFR TKI resistance and in particular caused by or associated with in-frame insertions and/or duplications of 3 to 21 base pairs (bp) between codons 763 and 775 of the Her2 gene or the EGFR (Her1) gene. More preferred, the mutation is selected from the group consisting of Her2 INS8 INS YVMA, EGFR D770_N771 insSVD, EGFR H773_V774insNPH, EGFR V769_D770insASV, EGFR P772_H773insPR, EGFR T790M and EGFR T790ML858R. The important sequences are shown in SEQ-ID No. 1 to SEQ:NO 7 in Figure 1

In an embodiment, the present invention is directed to the compound of in combination with at least one anticancer drug for use in treatment of cancer. The at least one anticancer is anticancer drug inhibting EGFR/HER2 tyrosine kinases, anticancer drug targeting the RAS/RAF/MEK/ERK signal pathway, anticancer drug targeting PI3K/AKT/mTOR signal pathway, and/or anticancer drug targeting JAK/STAT signal pathway.

The anticancer drug inhibting EGFR/HER2 tyrosine kinases is selected from the group consisting of A928605, ABT414, ABT806, AC480, Adgef (gefitinib), AEE788, AF802 (alectinib hydrochloride), Afatinib, Afinitor (everolimus), AFM21, AG1478, AGT2000, AL6802, ALL3, AMG595, Anti-Cripto-1 monoclonal antibodies PRIMA BIOMED, AP23464, AP26113, ARQ197 (tivantinib), ARRY380, ARRY543 (varlitinib tosylate), ASP7487 (linsitinib), ASP8273, AV203, AVL291, AZD4769, AZD9291, B-Peptimetics ANTYRA, BGB324, BIBW2948BS, Bispecific Anti-Her2 Zybodies ZYNGENIA (trastuzumab), Bispecific Anti-Her3 Zybodies ZYNGENIA (cetuximab), Bispecific Antibody Drug Conjugate Program AVIDBIOLOGICS, BL7030, BMS536924, BMS582664 (brivanib alaninate), BMS690514, BMSATI2, BT2111 (trastuzumab), CAB051, Canertinib, Caprelsa (vandetanib), CCX140, CDX110, CetuGEX, Cetuximab AMGEN, Cetuximab BIOXPRESS THERAPEUTICS, Cetuximab HARVEST MOON, Cetuximab ONCOBIOLOGICS, Cetuximab PANACEA BIOTECH, Cetuximab PLANTFORM, Cetuximab ZENOTECH, CGP59326A, Chemofit (gefitinib), CI1033 (canertinib dihydrochloride), CIMAher (nimotuzumab), Citoprot-P, CMAB009 (cetuximab), cMet-EGFR Dual Inhibitor CRYSTALGENOMICS, CO-1686, Cometriq (cabozantinib), Conmana (icotinib hydrochloride), CTP15 (cetuximab), CTX023, CUDC101, CYC202 (seliciclib), DC101, DXL1218, EDP13, EGF816, EGFR Antibody Drug Conjugate AVIDBIOLOGICS, EGFR BiTE antibody MICROMET, EGFR Monoclonal Antibody REVO, EGFR Probody Drug Conjugate CYTOMX, EGFR-Antibody-Targeted Endostatin Payload, EGFR501, EKB569 (pelitinib), EM1-mAb GENMAB, EMD121974 (cilengitide), EMD72000 (matuzumab), Emfib (gefitinib), Epidermal Growth Factor Receptor (EGFR) humanized antibody CHINA PHARMAHUB, Erbitux (cetuximab), Erlobenz (erlotinib), Erlocip (erlotinib), Erlonat NATCO (erlotinib), Erlonat RADIANCE (erlotinib), Erlons (erlotinib hydrochloride), Erlostar (erlotinib), Erloswift (erlotinib), Erlotad (erlotinib), Erlotaz (erlotinib), Erlotinib CYNO PHARMACEUTICALS (erlotinib hydrochloride), Erlotinib hydrochloride HETERO, Erlotinib Hydrochloride MYLAN, Erlotinib hydrochloride TEVA, Erlotinib LABORATORIOS INDUQUIMICA, Erlotinib NAPROD, Erlotinib P2D BIOSCEINCE, Erlotinib SALIUS, Erlotinib SRS PHARMA, Erlotinib UNITED BIOTECH, Etk/Bmx Kinase Inhibitor ASTEX, EZN3920, Fderceptin SHANGHAI FUDAN-ZHANGJIANG, FV225, Geffy (gefitinib), Geficad (gefitinib), Gefitinib CELOGEN, Gefitinib CYNO PHARMACEUTICALS, Gefitinib HETERO, Gefitinib LABORATORIOS INDUQUIMICA, Gefitinib MARKSANS, Gefitinib MISSION VIVACARE, Gefitinib NAPROD, Gefitinib SALIUS, Gefitinib SAVA, Gefitinib SRS PHARMA, Gefitinib UNITED BIOTECH, Gefitoz (gefitinib), GefiTrust (gefitinib), Gefnib (gefitinib), Geftex (gefitinib), Geftib (gefitinib), Gefticip (gefitinib), Geftifos (gefitinib), Geftiget (gefitinib), Geftin (gefitinib), Geftinat NATCO (gefitinib), Geftinat RADIANCE (gefitinib), Geftistar (gefitinib), Genasense (oblimersen sodium), GI3000, Gilotrif (aftinib), GSK2136773, HC15, HD201 (trastuzumab), HE39, HER-1 therapeutic cancer vaccine BIOVEN, HER1 Cancer Vaccine, HER2 BiTE Antibody AMGEN, Herzuma (trastuzumab), HKI357, HM60390, HM61713, HM78136B (poziotinib), HMPL309 (theliatinib), HMPL504 (volitinib), HMPL813 (epitinib), HuMax-EGFr (zalutumumab), HyERB (cetuximab), ICS283, Imatinib Ecker hydrochloride, Imbruvica (ibrutinib), IMC11F8 (necitumumab), IMCA12 (cixutumumab), IMGN289, INC280, INCB7839 (aderbasib), Inlyta (axitinib), Iressa (gefitinib), ISU101 (cetuximab), JNJ26483327, JNJ28871063, JX929, Kabigef (gefitinib), KD019, KD020, KHK2866, KRN633, KRN951 (tivozanib), KSB102, KT6587, Lapatinib AQVIDA, Lapatinib ditosylate, Lapatinib SRS PHARMA, Lefibenz (gefitinib), Lortinib (erlotinib), LY2875358 (emibetuzumab), MabionEGFR (cetuximab), MDP01, MDX214 (CD89 monoclonal antibody), MDX447, Meftinib (gefitinib), MEHD7945A, Menadione TALON, MGCD265, mir-7 mimetic SILENCE, MM121, MM151, MP412, MP470 (amuvatinib), MT062, Novel Tyrosine kinase Inhibitor MEBIOPHARM, NT004, NT113, ORIL003, ORIL007, OSI632, Panitumumab BIOXPRESS THERAPEUTICS (panitumumab), Panitumumab PANPHARMACEUTICALS, PB272 (neratinib), PBI1737, PBI4050, Perjeta (pertuzumab), PF00299804 (dacomitinib), PKI166, PM92102 (kahalalide F), RadioTheraCIM, RAF and HER inhibitors DECIPHERA, RBLX200, RC3095, Reglycosylated Cetuximab FOUNTAIN BIOPHARM, RG3638 (onartuzumab), RG7160 (imgatuzumab), RX1792, S222611, SAB-Y1 SYNTAB, Sai Pu Ting, SAI-EGFR-ECD MICROMET, SAR103168, SC100, Selatinib Ditosilate QILU, Selective EGFR Inhibitors VICHEM, SL175, SL176, SL433, SL461, SL634, SRXMs MAXOCARE, STIA020X, STIA050X, Stivarga (regorafenib), STP523, STP801, Stridessa (gefitinib), Surrobody Drug Conjugate SEA LANE, Sym004, Sym013, TaiXinSheng (nimotuzumab), TAK285, Tarceva (erlotinib hydrochloride), Targretin (bexarotene), TAS2913, TG03 (apricoxib), TGF therapeutic cancer vaccine BIOVEN, TGF-alpha Cancer Vaccine MICROMET, Trastuzumab ZENOTECH, Trisilensa, Trispecific Anti-Her1/Her3 Zybodies ZYNGENIA (cetuximab), Tykerb (lapatinib ditosylate), Tyrogef, Tyrokinin 100 (erlotinib hydrochloride), U31287 (partitumab), Ultragef (gefitinib), VCC395122, VCC868449, Vectibix (panitumumab), VI14442, Xefta (gefitinib), YMB1005, Zefotib (gefitinib), and Zufinib (gefitinib).

The anticancer drug targeting the RAS/RAF/MEK/ERK signal pathway is selected from the group consisting of: Dabrafenib, GSK2118436, LGX818, Vemurafenib, RAF265, RO5126766, Sorafenib, XL281 (Raf inhibitor); ARRY-300, AZD8330, E6201, PD-0325901, RO4987655, Bimetinib (ARRY-162/ MEK162), Cobimetinib (GDC-0973/XL518), Refametinib (BAY86-9766/RDEA119), Pisasertib (AS703026), Selumetinib (AZD6244/ARRY-142886), TAK-733, Trametinib (GSK1120212) (MEK inhibitor), BVD-523, and MK8553 (ERK inhibitor) .

The anticancer drug targeting PI3K/AKT/mTOR signal pathway is selected from the group consisting of : BGT226, BEZ235, GDC-0980, GSK2126458, PF-04691502, PF-05212384/PKI-587, SF1126, XL765/SAR245409, BKM120, BYL719, CAL-101, GDC-0032, GDC-0941, GSK2636771, IPI-145, NVP-BEZ235, PX866, XL147 (PI3K inhibitor); Erucylphosphocholine, GSK2141795, GSK690693, Miltefosine, MK2206, PBI-05204, Perifosine, RX-0201, SR13668, XL-418 (AKT inhibitor), AZD2014, AZD8055, CC-223, Everolimus (RAD001), Ridaforolimus (MK-8669), OSI-027, Sirolimus (Rapamycin), and Temsirolmus (Torisel®, CCI-779) (mTOR inhibitor).

The anticancer drug targeting JAK/STAT signal pathway is JAK kinase inhibitor or STAT inhibitor. JAK kinase inhibitor has inhibitory activity against JAK1, JAK2, and/or JAK3 and is selected from the group consisting of ABT-494, AT9283, atiprimod dihydrochloride, AZD1480, Baricitinib, BMS-911543, CP 690550, Cucurbitacin I, Decernotinib, Filgotinib, Gandotinib, GSK2586184, Itacitinib (INCB039110), INCB018424, INCB047986, INCB052793, Lestaurtinib, Momelotinib (CYT387), NS-018, NSC 33994, Pacritinib, Peficitinib, Ruxolitinib (Jakafi), PF-04965842, SD 1008, Tofacitinib, Upadacitinib, XL019, and WP1066.

STAT inhibitor has inhibitory activity against STATs 1, 2, 3, 4, 5a, 5b, and 6, in particular STAT3 and STAT5b and is selected from the group consisting of AZD9150, Capsaicin, CPA-1, CPA-7, FLLL11, FLLL12, FLLL32, FLLL62, IS3295, JQ1, OPB-111077, OPB-31121, OPB-51602 and pimozide.

### Pharmaceutical Composition

The pharmaceutical compositions according to the present invention comprise at least one compound according to the present invention as an active ingredient together with at least one pharmaceutically acceptable (i.e. non-toxic) carrier, excipient and/or diluent. The pharmaceutical compositions of the present invention can be prepared in a conventional solid or liquid carrier or diluent and a conventional pharmaceutically made adjuvant at suitable dosage level in a known way. The preferred preparations are adapted for oral application. These administration forms include, for example, pills, tablets, film tablets, coated tablets, capsules, powders and deposits.

Optionally, the pharmaceutical compositions according to the present invention comprise further at least one anticancer drug. The at least one anticancer is anticancer drug inhibting EGFR/HER2 tyrosine kinases, anticancer drug targeting the RAS/RAF/MEK/ERK signal pathway, anticancer drug targeting PI3K/AKT/mTOR signal pathway, and/or anticancer drug targeting JAK/STAT signal pathway as defined above.

Furthermore, the present invention also includes pharmaceutical preparations for parenteral application, including dermal, intradermal, intragastral, intracutaneous, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the derivatives according to the general formula (I) or analogues compound thereof or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. anticancer activity or activity against cancer metastases and the like. Suitable dosage forms for sustained release include tablets having layers of varying disintegration rates or controlled release, polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to 1.0 weight %.

The compounds of the present invention are suitable for use in medicine, particulary in human medicine, but also in veterinary medicine. The dosage of the compounds may be determined by a skilled practitioner according to the type and severity of the disorder to be treated.

The compounds of the present invention may be admistered as a monotherapy or together with further active agents, particulary chemotherapeutic agents or antitumor antibodies. Furthermore they may be used in combination with surgery and/or irradiation.

### Description of Figures

**Figure 1****:** core sequences of EGFR mutants EGFR D770_N771insSVD, EGFR H773_V774insNPH, EGFR V769_D770insASV, EGFR P772_H773insPR, EGFR T790M and EGFR T790ML858R and HER mutant Her2 INS8 INS YVMA.

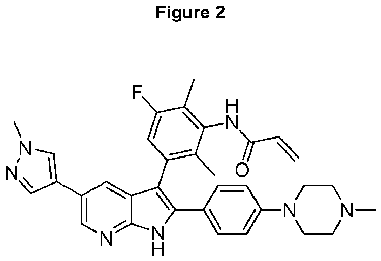

**Figure 2****:** shows a representative example of a compound according to the invention.

### Preparation of compounds

### General Information:

All reactions involving air- or moisture-sensitive reagents or intermediates were carried out in flame-dried glassware under an argon atmosphere. Dry solvents (THF, toluene, MeOH, DMF, DCM) were used as commercially available. ¹H-NMR and ¹³C-NMR were recorded on a Bruker DRX400 (400 MHz). Multiplicities are indicated as: br s (broadened singlet), s (singlet), d (doublet), t (triplet), q (quartet), quin (quintet), m (multiplet); and coupling constants (J) are given in Hertz (Hz). HPLC - electrospray mass spectra (HPLC ES-MS) were obtained using Waters Acquity Performance Liquid Chromatography (UPLC) equipped SQ 3100 Mass detector spectrometer. Column: Acquity UPLC BEH C18 1.7um, 2.1x50mm. Flow: 0.5ml/min. Eluents: A: H₂O with 0.05% formic acid and B: ACN with 0.05% TFA. All chemicals and solvents were purchased from commercial sources like Sigma-Aldrich, Fluka, TCI, Acros Organics, ABCR, Alfa Aesar, Enamine, VWR, Combi-Blocks, Apollo Scientific, Aquilla Pharmatech, Ark Pharm, D-L Chiral Chemicals, ChemBridge, Renno Tech, Accela, KeyOrganics, Pharmablock and Chem Impex. Unless otherwise noted, all commercially available compounds were used as received without further purifications.

### Abbreviations used in the description of the chemistry and in the Examples that follow are:

ACN (acetonitrile); br (broad); CDCl₃ (deuterated chloroform); cHex (cyclohexane); DCM (dichloromethane); DIPEA (di-iso-propylethylamine); DMF (dimethylformamide); DMSO (dimethyl sulfoxide); eq. (equivalent); ES (electrospray); EtOAc (ethyl acetate); EtOH (ethanol); HATU (*O*-(7-azabenzotriazol-1-yl)-*N*,*N*,*N*',*N*'-tetramethyluronium hexafluorophosphate); HCI (hydrochloric acid); HOAc (acetic acid); H₂O (water); K₂CO₃ (potassium carbonate); KOH (potassium hydroxide); MeOH (methanol); MS (mass spectrometry); Mwt (molecular weight); NaHCO₃ (sodium hydrogencarbonate); NH₃ (ammonia); NH₄Cl (ammonium chloride); NIS (*N*-lodosuccinimide); NMP (N-methyl-2-pyrrolidon); NMR (nuclear magnetic resonance); Pd(dppf)Cl₂ ([1,1'-bis(diphenylphosphino)ferrocene]dichloro palladium(II) complex with dichloromethane); iPrOH (iso-propanol); PyBroP (Bromotripyrrolidinophosphonium hexafluorophosphate); RP (reversed-phase); RT (room temperature); sat. aq. (saturated aqueous); SiO₂ (silica gel); TFA (trifluoroacetic acid); THF (tetrahydrofurane); TMS: Trimethylsilyl.

### Preparative Examples

### Example 1:

### N-(2-methyl-5-(5-(1-methyl-1H-pyrazol-4-yl)-2-(4-(4-methylpiperazin-1-yl)phenyl)-1H-pyrrolo[2,3-b]pyridin-3-yl)phenyl)acrylamide 2,2,2-trifluoroacetate (A6)

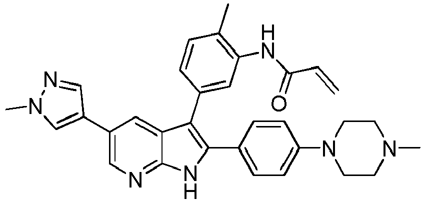

### Step 1: 5-bromo-3-(4-methyl-3-nitrophenyl)-2-(trimethylsilyl)-1H-pyrrolo[2,3-b]pyridine (A1)

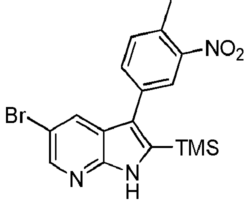

In a pressure tube, 5-bromo-3-iodopyridin-2-amine (1g, 3.3mmol, 1eq) and trimethyl[2-(4-methyl-3-nitrophenyl)ethynyl]silane (1.03g, 4.2mmol, 1.25eq) were dissolved in DMF (20mL). Next, triethylenediamine (0.64g, 5.7mmol, 1.7eq) and bis(triphenylphosphine)palladium (II) dichloride (0.23g, 0.33mmol, 0.1eq) were added, the tube was purged with argon, and then heated at 145°C for 24h. The resulting mixture was diluted with saturated aqueous NaHCO₃ solution, and extracted with EtOAc (3x50mL). The organic layer was washed with saturated aqueous NaCl solution, dried over Na₂SO₄, and concentrated under reduced pressure. The crude product was purified by column chromatography (hexane:EtOAc, 6:4) to afford the pure product **A1** as an orange solid (0.8g, Y:62%). ¹H NMR (300 MHz, DMSO-*d*₆) δ 11.80 (s, 1H), 8.23 (s, 1H), 7.70 (s, 2H), 7.59 (dd, *J* = 9.9, 1.7 Hz, 2H), 2.03 (s, 3H), 0.00 (s, 9H). MS (ES) C₁₇H₁₈BrN₃O₂Si requires: 404, found: 405 (M+H)⁺.

### Step 2: 5-bromo-2-iodo-3-(4-methyl-3-nitrophenyl)-1H-pyrrolo[2,3-b]pyridine (A2)

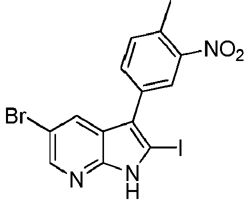

5-Bromo-3-(4-methyl-3-nitrophenyl)-2-(trimethylsilyl)-1H-pyrrolo[2,3-b]pyridine **(A1)** (0.8g, 2mmol, 1eq) and NIS (0.8g, 3.4mmol, 1.8eq) were dissolved in dry DCM (30mL). The reaction mixture was stirred for 4h at room temperature, and was quenched with saturated aqueous Na₂S₂O₃ solution. The desired product was extracted with DCM (3x100mL), the organic phase was washed with saturated aqueous NaHCO₃ solution, dried over Na₂SO₄, and solvent were removed in vacuo. The crude material **A2** as an orange solid (0.6g, Y:66%) was immediately used without purification in the next step. MS (ES) C₁₄H₉BrIN₃O₂ requires: 458, found: 459 (M+H)⁺.

### Step 3: 5-bromo-3-(4-methyl-3-nitrophenyl)-2-(4-(4-methylpiperazin-1-yl)phenyl)-1H-pyrrolo[2,3-b]pyridine (A3)

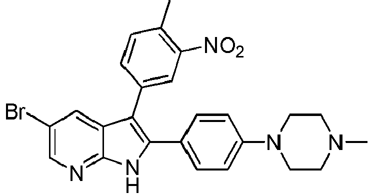

5-Bromo-2-iodo-3-(4-methyl-3-nitrophenyl)-1H-pyrrolo[2,3-b]pyridine **(A2)** (600mg, 1.3mmol, 1eq), [4-(4-methylpiperazin-1-yl)phenyl]boronic acid (288mg, 1.3mmol, 1eq), and Na₂CO₃ (278mg, 2.6mmol, 2eq) were dissolved in the mixture of solvents: THF 6mL/MeOH 0.6mL/ H₂O 0.6mL (1:0.1:0.1 vol), degassed under vacuum, and purged with argon. Next, Pd(PPh₃)₂Cl₂ (184mg, 0.26mmol, 0.2eq) was added, and the reaction mixture heated at 80°C for 24h. The resulting mixture was cooled, the desired product was extracted with DCM (3x45mL). The organic phase was dried over Na₂SO₄, and concentrated under reduced pressure. The crude product was purified by column chromatography DCM:MeOH, 8:2) to afford the product **A3** as a brown solid (500mg, Y:75%). MS (ES) C₂₅H₂₄BrN₅O₂ requires: 506, found: 507 (M+H)⁺.

### Step 4: 5-(5-bromo-2-(4-(4-methylpiperazin-1-yl)phenyl)-1H-pyrrolo[2,3-b]pyridin-3-yl)-2-methylaniline (A4)

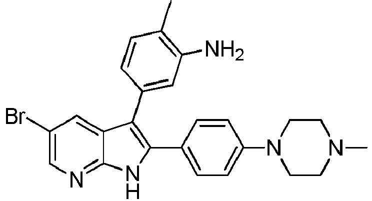

A suspension of 1-{4-[5-bromo-3-(4-methyl-3-nitrophenyl)-1H-pyrrolo[2,3-b]pyridin-2-yl]phenyl}-4-methylpiperazine **(A3)** (500mg, 1mmol, 1eq) and iron (276mg, 4.9mmol, 5eq) in a mixture of EtOH (5mL) and saturated aqueous NH₄Cl solution (0.5mL) was stirred for 3h at 80°C. Then, the reaction mixture was filtered through a pad of silica gel, washed with DCM (200mL), next AcOEt (200mL). The solvents were evaporated under pressure to obtain the desired product **A4** as a yellow solid (300mg, Y:63%). ¹H NMR (300 MHz, DMSO-*d*₆) δ 12.31 (s, 1H), 8.27 (s, 2H), 7.84 (d, *J* = 1.6 Hz, 2H), 7.77-7.61 (m, 4H), 7.47 (s, 1H), 7.41 (s, 2H), 3.81-3.75 (m, 4H), 2.48-2.44 (m, 4H), 2.13 (d, 6H). MS (ES) C₂₅H₂₆BrN₅ requires: 476, found: 477 (M+H)⁺.

### Step 5: N-(5-(5-bromo-2-(4-(4-methylpiperazin-1-yl)phenyl)-1H-pyrrolo[2,3-b]pyridin-3-yl)-2-methylphenyl)acrylamide (A5)

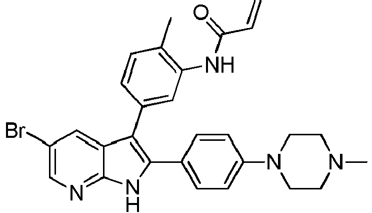

5-{5-Bromo-2-[4-(4-methylpiperazin-1-yl)phenyl]-1 H-pyrrolo[2,3-b]pyridin-3-yl}-2-methylaniline **(A4)** (300mg, 0.6mmol, 1eq) was dissolved in THF (10mL) at -10°C. Next, DIPEA (1.1mL, 6mmol, 10eq) was dropwise added, and the mixture was stirred for 5min at -10°C. A solution of acryloyl chloride (27µL, 0.6mmol, 1eq) in THF (10mL) was dropwise added, and the reaction mixture was slowly warmed to room temperature. After 30min, LCMS showed full conversion. The reaction mixture was diluted with water (10mL), and the desired product was extracted with DCM (3x10mL). The organic phase was dried over Na₂SO₄, and concentrated under reduced pressure. The crude product was purified by column chromatography (hexane:EtOAc, 0-50% of AcOEt) to yield the material that was triturated with DCM to obtain 120mg (Y: 36%) of the pure product **A5** as a creamy solid. ¹H NMR (300 MHz, DMSO-d6) δ 12.18 (s, 1H), 9.53 (s, 1H), 8.26 (d, J = 2.2 Hz, 1H), 7.94 (d, J = 2.2 Hz, 1H), 7.59 (s, 1H), 7.39 (d, J = 8.7 Hz, 2H), 7.22 (d, J = 7.9 Hz, 1H), 6.98 (d, J = 7.7 Hz, 1H), 6.92 (d, J = 8.9 Hz, 2H), 6.55 (dd, J = 17.0, 10.3 Hz, 1H), 6.24 (dd, J = 17.0, 2.1 Hz, 1H), 5.75 (d, J = 9.4 Hz, 1H), 3.22 - 3.13 (m, 4H), 2.46 - 2.38 (m, 4H), 2.23 (d, J = 10.9 Hz, 6H). MS (ES) C₂₈H₂₈BrN₅O requires: 530, found: 531 (M+H)⁺.

### Step 6: N-(2-methyl-5-(5-(1-methyl-1H-pyrazol-4-yl)-2-(4-(4-methylpiperazin-1-yl)phenyl)-1H-pyrrolo[2,3-b]pyridin-3-yl)phenyl)acrylamide 2,2,2-trifluoroacetate (A6)

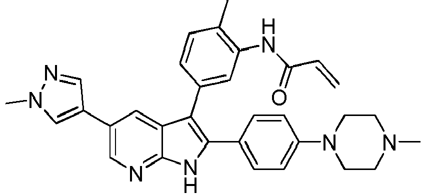

A mixture of N-(5-(5-bromo-2-(4-(4-methylpiperazin-1-yl)phenyl)-1H-pyrrolo[2,3-b]pyridin-3-yl)-2-methylphenyl)acrylamide **(A5)** (37.0mg, 0.07mmol, 1.0eq.), K₃PO₄ (29.6mg, 0.139mmol, 2.0eq.), 1-methyl-4-(4,4,5,5-tetramethyl-1,3,2-dioxaborolan-2-yl)-1H-pyrazole (18.9mg, 0.09mmol, 1.3eq.) and [1,1'-Bis(diphenylphosphino)ferrocene]-palladium dichloride dichloromethane adduct (5.7mg, 0.007mmol, 0.1eq.) in dioxane/water (4:1, 1.9mL) was degassed by bubbling nitrogen through it for a few minutes. The mixture was then heated in the microwave at 130°C for 2h. The resulting reaction mixture was filtered through a syringe filter and the filtrate was directly used for reversed phase HPLC (C18 column) separation (water + 0.1% TFA/ACN + 0.1% TFA = 95:5 to 65:35, 30 min). The desired fractions were combined and lyophilized to yield the title compound **A6** (3.0mg, 0.004mmol, 6%) as a yellow solid. ¹H NMR (300 MHz, DMSO-d6) δ 11.93 (s, 1H), 9.62 (br s, 1H), 9.52 (s, 1H), 8.47 (d, J = 2.0 Hz, 1H), 8.15 (s, 1H), 8.01 (d, J = 2.0 Hz, 1H), 7.89 (d, J = 0.9 Hz, 1H), 7.66 (s, 1H), 7.43 (d, J = 9.0 Hz, 2H), 7.20 (d, J = 7.9 Hz, 1H), 6.98 (d, J = 9.0 Hz, 2H), 6.57 (dd, J = 10.1 Hz, J = 17.0 Hz, 1H), 6.24 (dd, J = 2.0 Hz, J = 17.0 Hz, 1H), 5.74 (dd, J = 2.0 Hz, J = 10.1 Hz, 1H), 3.91 (d, J = 13.3 Hz, 2H), 3.84 (s, 3H), 3.50 (d, J = 12.1 Hz, 2H), 3.13 (m, 2H), 2.97 (t, J = 11.7 Hz, 2H), 2.85 (d, J = 3.2 Hz, 3H), 2.25 (s, 3H). MS (ES) C₃₂H₃₃N₇O requires: 531, found: 532 (M+H)⁺.

The Examples in the following table were prepared according to the procedure described for **A6** (Example 1).

| **Example** | **Name** | **Mwt** | **[M+H]⁺** |
|---|---|---|---|
| 2 | 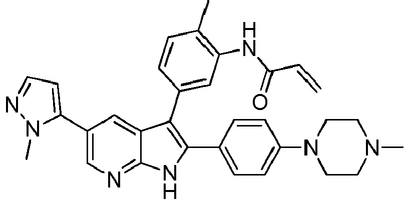 | 531 | 532 |
| 3 | 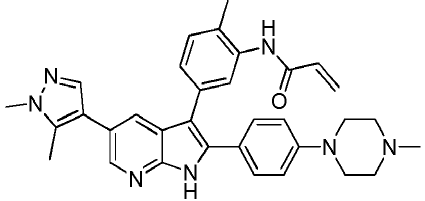 | 545 | 546 |
| 4 | 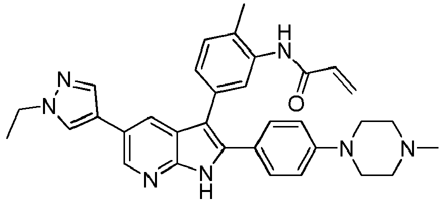 | 545 | 546 |
| 5 | 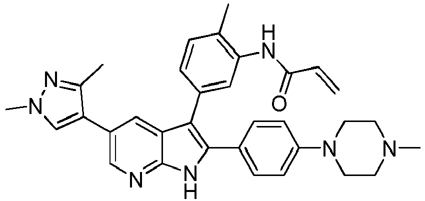 | 545 | 546 |
| 6 | 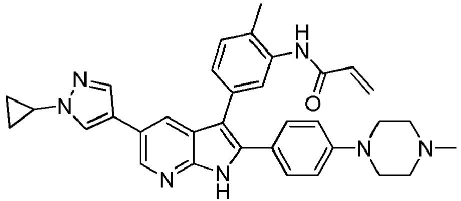 | 557 | 558 |
| 7 | 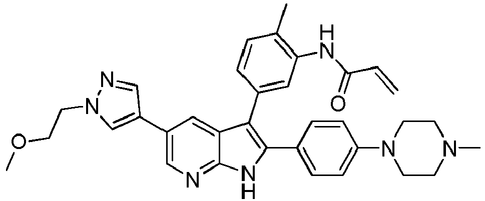 | 575 | 576 |
| 8 | 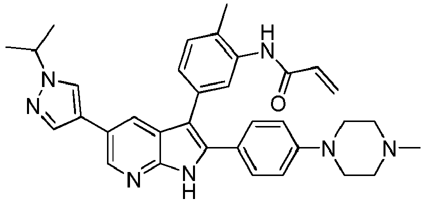 | 559 | 560 |
| 9 | 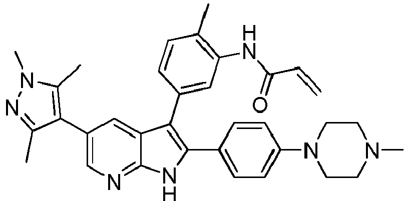 | 559 | 560 |
| 10 | 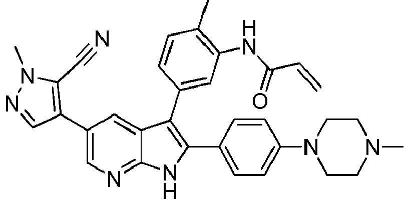 | 556 | 557 |
| 11 | 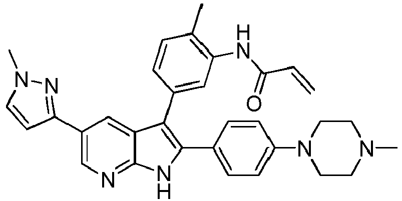 | 531 | 532 |
| 12 | 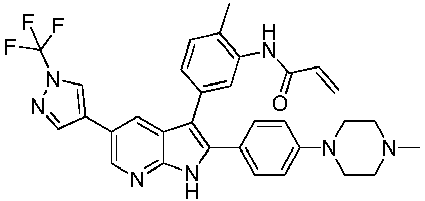 | 585 | 586 |
| 13 | 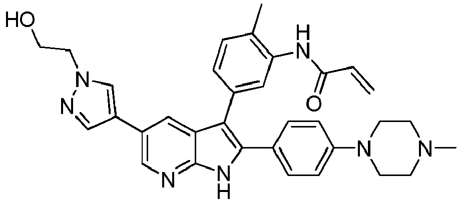 | 561 | 562 |
| 14 | 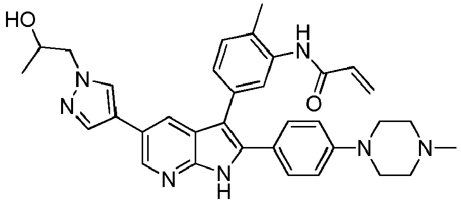 | 575 | 576 |
| 15 | 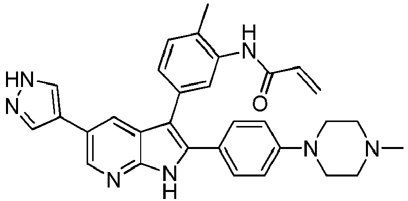 | 517 | 518 |
| 16 | 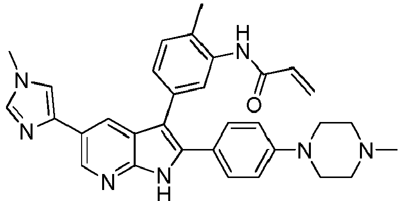 | 531 | 532 |
| 17 | 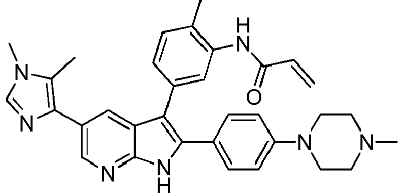 | 545 | 546 |
| 18 | 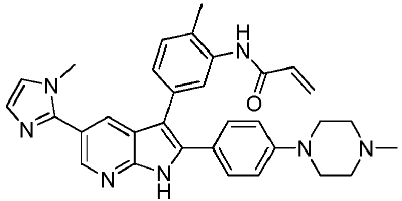 | 531 | 532 |
| 19 | 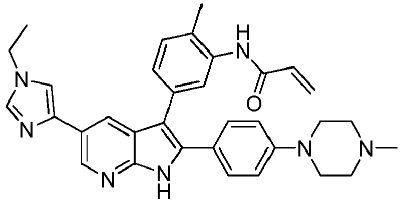 | 545 | 546 |

The Examples in the following table were prepared from 5-bromo-4-ethyl-3-iodopyridin-2-amine, trimethyl((4-methyl-3-nitrophenyl)ethynyl)silane and 4-(4-methylpiperazin-1-yl)phenylboronic acid according to the procedure described for **A6** (Example 1).

| **Example** | **Name** | **Mwt** | **[M+H]⁺** |
|---|---|---|---|
| 20 | 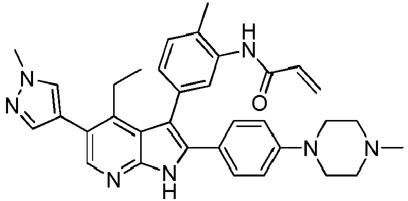 | 559 | 560 |
| 21 | 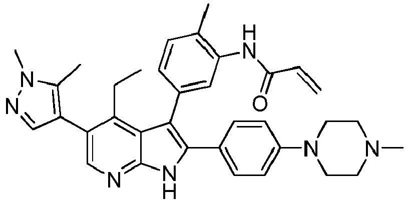 | 573 | 574 |

The Examples in the following table were prepared from 5-bromo-4-chloro-3-iodopyridin-2-amine, trimethyl((4-methyl-3-nitrophenyl)ethynyl)silane and 4-(4-methylpiperazin-1-yl)phenylboronic acid according to the procedure described for **A6** (Example 1).

| **Example** | **Name** | **Mwt** | **[M+H]⁺** |
|---|---|---|---|
| 22 | 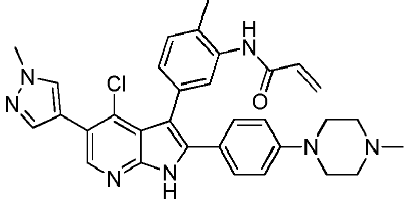 | 566 | 567 |
| 23 | 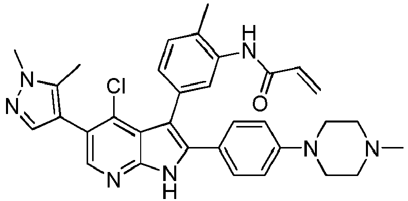 | 579 | 580 |
| 24 | 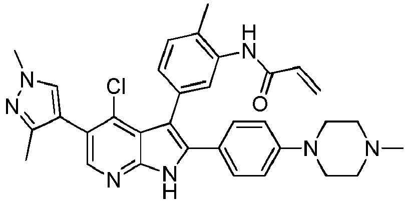 | 579 | 580 |
| 25 | 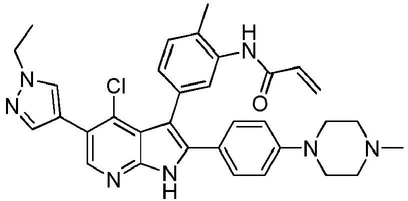 | 579 | 580 |
| 26 | 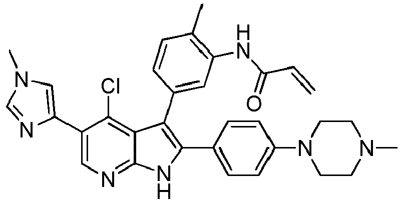 | 565 | 566 |
| 27 | 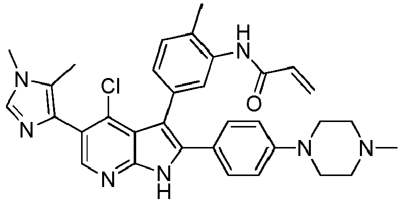 | 579 | 580 |
| 28 | 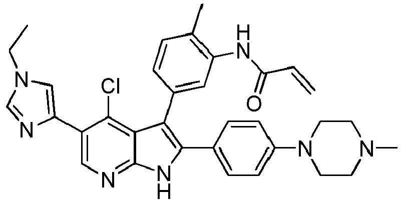 | 579 | 580 |

The Examples in the following table were prepared from 5-bromo-3-iodo-4-methoxypyridin-2-amine, trimethyl((4-methyl-3-nitrophenyl)ethynyl)silane and 4-(4-methylpiperazin-1-yl)phenylboronic acid according to the procedure described for **A6** (Example 1).

| **Example** | **Name** | **Mwt** | **[M+H]⁺** |
|---|---|---|---|
| 29 | 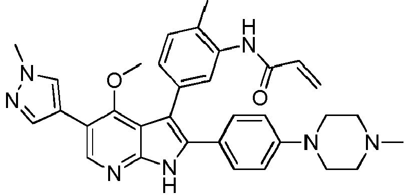 | 561 | 562 |
| 30 | 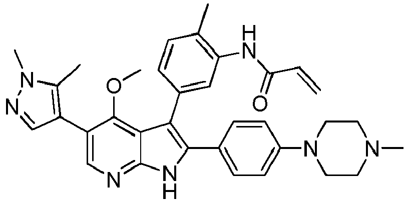 | 575 | 576 |
| 31 | 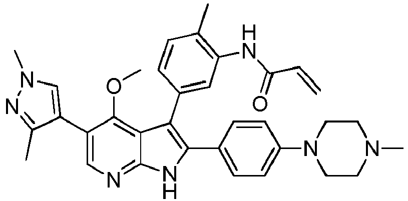 | 575 | 576 |
| 32 | 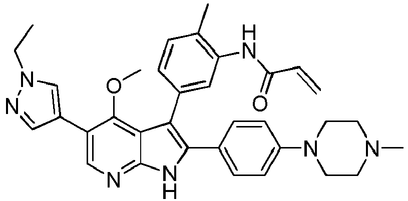 | 575 | 576 |
| 33 | 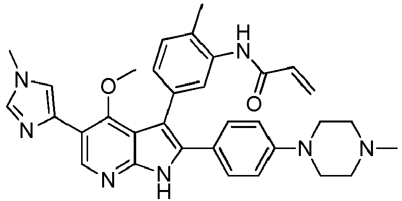 | 561 | 562 |
| 34 | 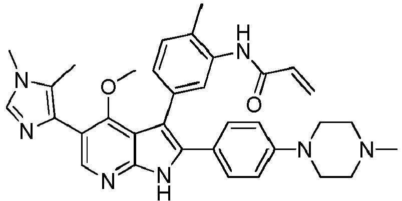 | 575 | 576 |
| 35 | 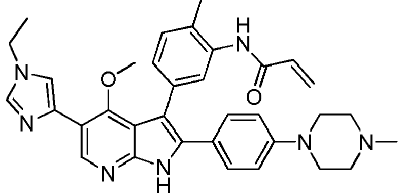 | 575 | 576 |

The Examples in the following table were prepared from 5-bromo-3-iodo-4-methylpyridin-2-amine, trimethyl((4-methyl-3-nitrophenyl)ethynyl)silane and 4-(4-methylpiperazin-1-yl)phenylboronic acid according to the procedure described for **A6** (Example 1).

| **Example** | **Name** | **Mwt** | **[M+H]⁺** |
|---|---|---|---|
| 36 | 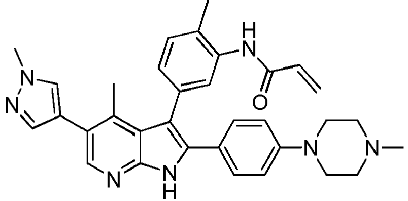 | 545 | 546 |
| 37 | 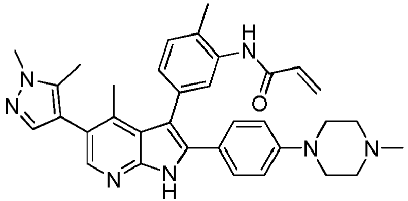 | 559 | 560 |
| 38 | 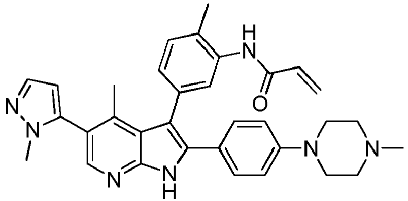 | 545 | 546 |
| 39 | 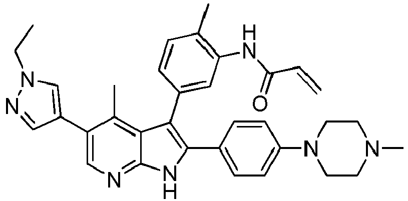 | 559 | 560 |
| 40 | 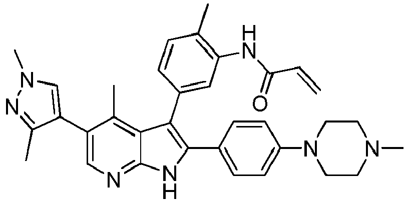 | 559 | 560 |
| 41 | 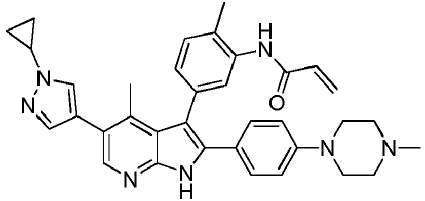 | 571 | 572 |
| 42 | 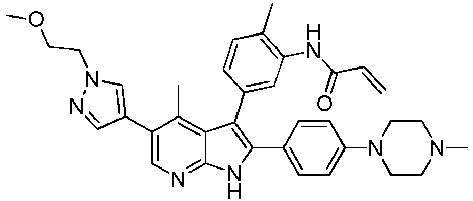 | 589 | 590 |
| 43 | 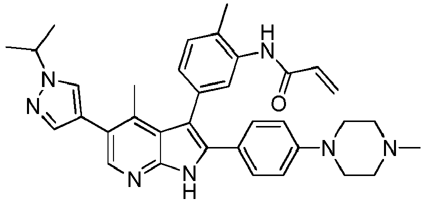 | 573 | 574 |
| 44 | 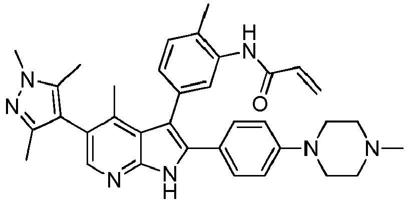 | 573 | 574 |
| 45 | 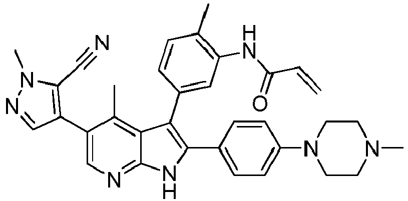 | 570 | 571 |
| 46 | 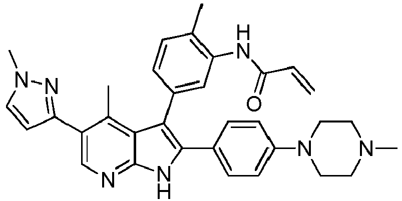 | 545 | 546 |
| 47 | 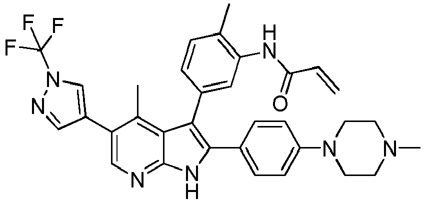 | 599 | 600 |
| 48 | 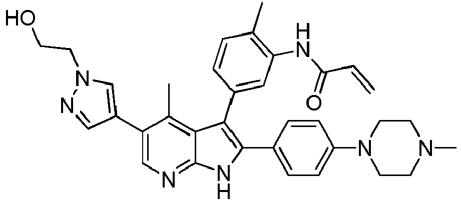 | 575 | 576 |
| 49 | 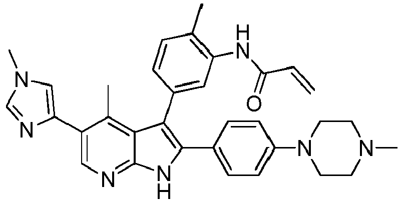 | 545 | 546 |
| 50 | 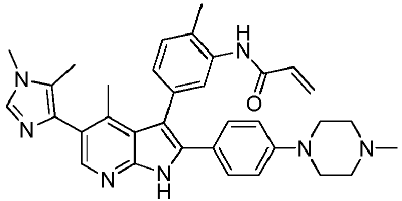 | 559 | 560 |

The Examples in the following table were prepared from 5-bromo-3-iodopyridin-2-amine, ((2,4-dimethyl-3-nitrophenyl)ethynyl)trimethylsilane and 4-(4-methylpiperazin-1-yl)phenylboronic acid according to the procedure described for **A6** (Example 1).

| **Example** | **Name** | **Mwt** | **[M+H]⁺** |
|---|---|---|---|
| 51 | 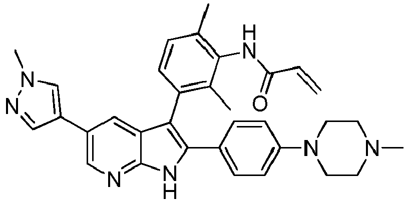 | 545 | 546 |
| 52 | 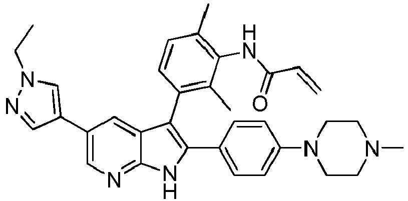 | 559 | 560 |
| 53 | 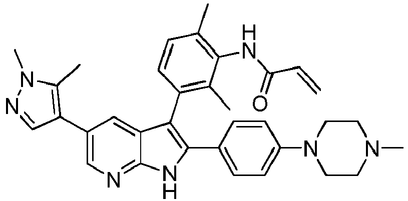 | 559 | 560 |
| 54 | 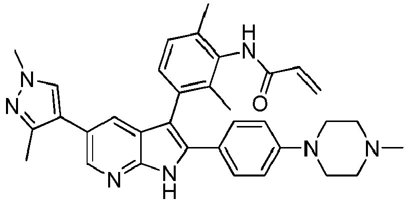 | 559 | 560 |

The Examples in the following table were prepared from 5-bromo-3-iodopyridin-2-amine, ((3-fluoro-4-methyl-5-nitrophenyl)ethynyl)trimethylsilane and 4-(4-methylpiperazin-1-yl)phenylboronic acid according to the procedure described for **A6** (Example 1).

| **Example** | **Name** | **Mwt** | **[M+H]⁺** |
|---|---|---|---|
| 55 | 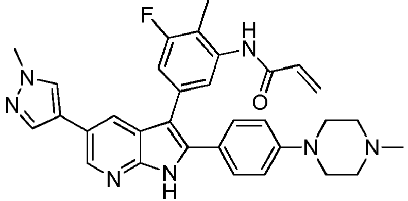 | 549 | 550 |
| 56 | 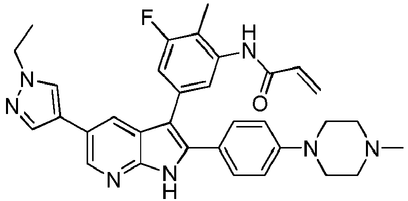 | 563 | 564 |
| 57 | 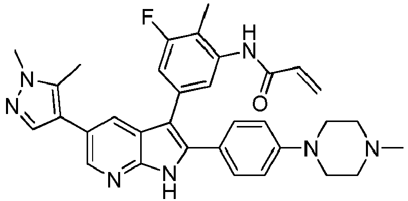 | 563 | 564 |
| 58 | 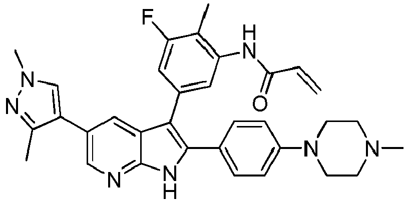 | 563 | 564 |

The Examples in the following table were prepared from 5-bromo-3-iodopyridin-2-amine, ((5-fluoro-2,4-dimethyl-3-nitrophenyl)ethynyl)trimethylsilane and 4-(4-methylpiperazin-1-yl)phenylboronic acid according to the procedure described for **A6** (Example 1).

| **Example** | **Name** | **Mwt** | **[M+H]⁺** |
|---|---|---|---|
| 59 | 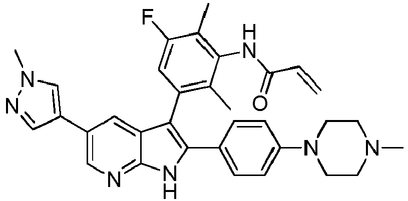 | 563 | 564 |
| 60 | 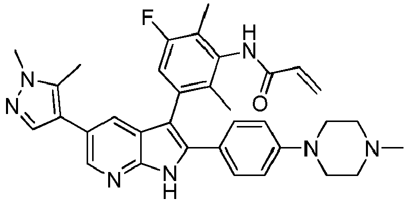 | 577 | 578 |

The Examples in the following table were prepared from 5-bromo-3-iodopyridin-2-amine, tert-butyl 6-((trimethylsilyl)ethynyl)indoline-1-carboxylate and 4-(4-methylpiperazin-1-yl)phenylboronic acid according to the procedure described for **A6** (Example 1). Instead of the reduction in Step 4 of Example 1 for these Examples the protecting group tert-butyloxycarbonyl (*Boc*) was removed under standard conditions using TFA in DCM at ambient temperature.

| **Example** | **Name** | **Mwt** | **[M+H]⁺** |
|---|---|---|---|
| 61 | 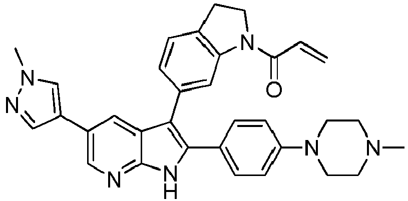 | 543 | 544 |
| 62 | 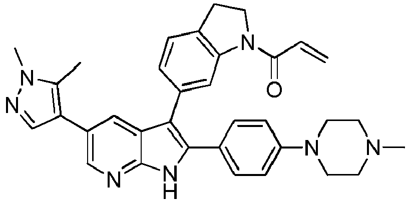 | 557 | 558 |

The Examples in the following table were prepared from 5-bromo-3-iodopyridin-2-amine, tert-butyl 4-((trimethylsilyl)ethynyl)indoline-1-carboxylate and 4-(4-methylpiperazin-1-yl)phenylboronic acid according to the procedure described for **A6** (Example 1). Instead of the reduction in Step 4 of Example 1 for these Examples the protecting group tert-butyloxycarbonyl (*Boc*) was removed under standard conditions using TFA in DCM at ambient temperature.

| **Example** | **Name** | **Mwt** | **[M+H]⁺** |
|---|---|---|---|
| 63 | 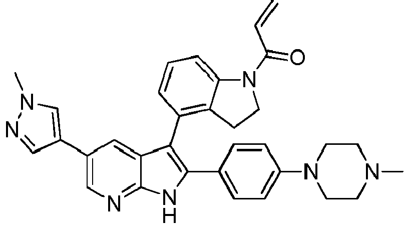 | 543 | 544 |
| 64 | 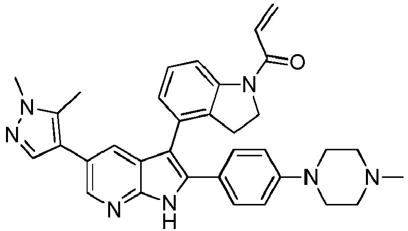 | 557 | 558 |

The Examples in the following table were prepared from 5-bromo-3-iodopyridin-2-amine, ((2-methoxy-4-methyl-3-nitrophenyl)ethynyl)trimethylsilane and 4-(4-methylpiperazin-1-yl)phenylboronic acid according to the procedure described for **A6** (Example 1).

| **Example** | **Name** | **Mwt** | **[M+H]⁺** |
|---|---|---|---|
| 65 | 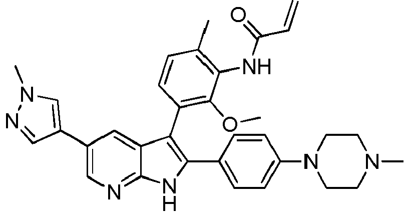 | 561 | 562 |
| 66 | 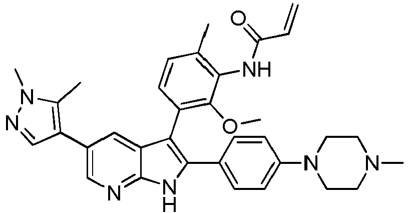 | 575 | 576 |

The Examples in the following table were prepared from 5-bromo-3-iodopyridin-2-amine, tert-butyl methyl(3-((trimethylsilyl)ethynyl)phenyl)carbamate and and 4-(4-methylpiperazin-1-yl)phenylboronic acid according to the procedure described for **A6** (Example 1).

| **Example** | **Name** | **Mwt** | **[M+H]⁺** |
|---|---|---|---|
| 67 | 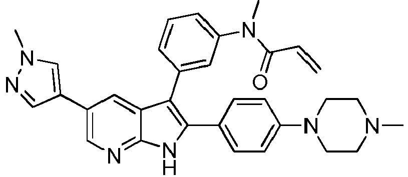 | 531 | 532 |
| 68 | 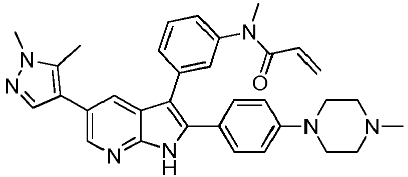 | 545 | 546 |

The Examples in the following table were prepared from 5-bromo-3-iodopyridin-2-amine, trimethyl((4-methyl-3-nitrophenyl)ethynyl)silane and (*R*)-1,2-dimethyl-4-(4-(4,4,5,5-tetramethyl-1,3,2-dioxaborolan-2-yl)phenyl)piperazine according to the procedure described for **A6** (Example 1).

| **Example** | **Name** | **Mwt** | **[M+H]⁺** |
|---|---|---|---|
| 69 | 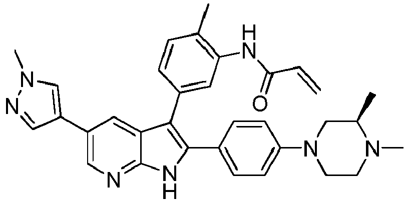 | 545 | 546 |
| 70 | 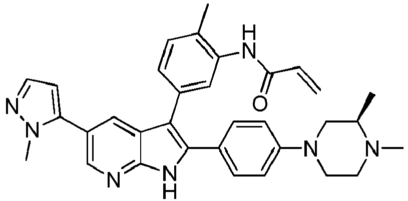 | 545 | 546 |
| 71 | 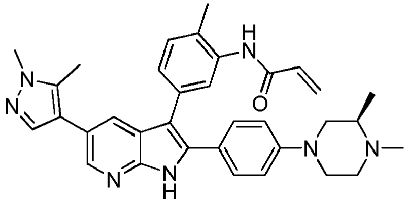 | 559 | 560 |
| 72 | 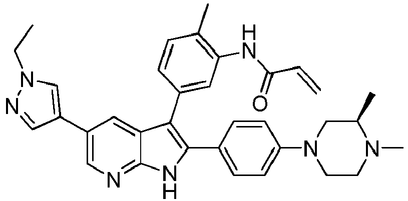 | 559 | 560 |
| 73 | 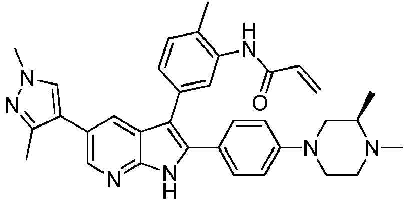 | 559 | 560 |
| 74 | 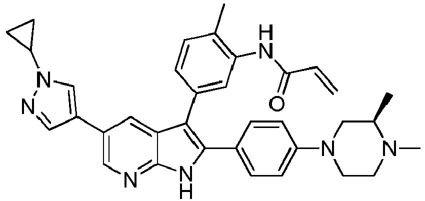 | 571 | 572 |
| 75 | 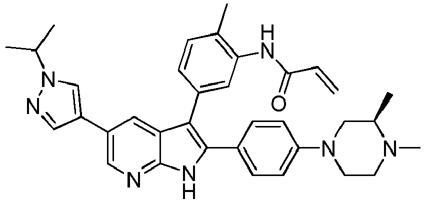 | 573 | 574 |
| 76 | 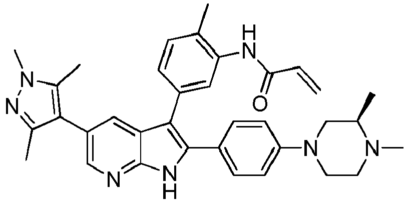 | 573 | 574 |
| 77 | 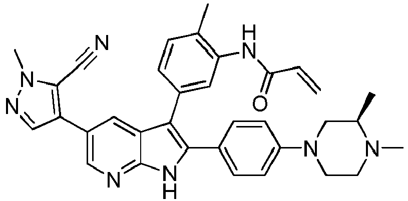 | 570 | 571 |
| 78 | 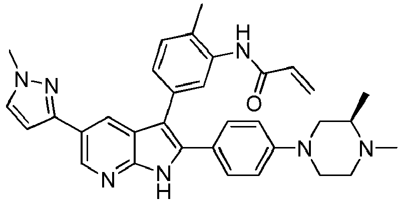 | 545 | 546 |
| 79 | 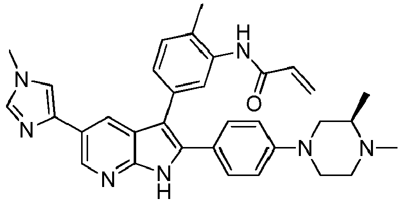 | 545 | 546 |

The Examples in the following table were prepared from 5-bromo-3-iodo-4-methylpyridin-2-amine, trimethyl((4-methyl-3-nitrophenyl)ethynyl)silane and (*R*)-1,2-dimethyl-4-(4-(4,4,5,5-tetramethyl-1,3,2-dioxaborolan-2-yl)phenyl)piperazine according to the procedure described for **A6** (Example 1).

| **Example** | **Name** | **Mwt** | **[M+H]⁺** |
|---|---|---|---|
| 80 | 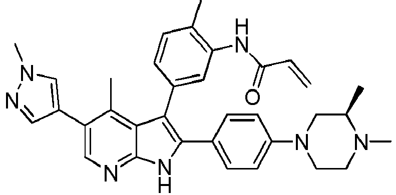 | 559 | 560 |
| 81 | 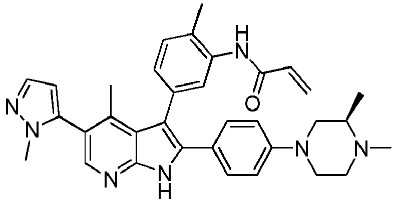 | 559 | 560 |
| 82 | 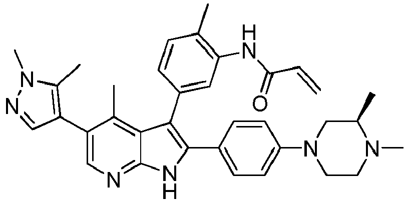 | 573 | 574 |
| 83 | 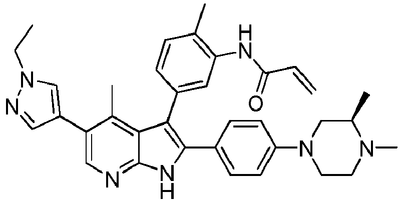 | 573 | 574 |
| 84 | 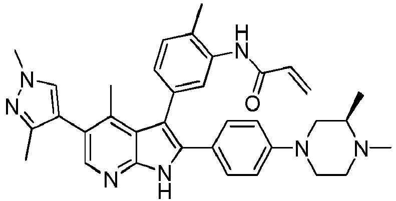 | 573 | 574 |
| 85 | 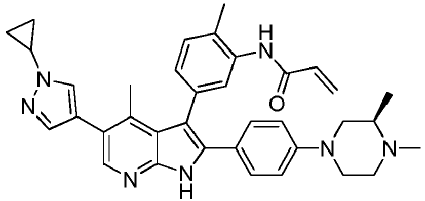 | 585 | 586 |
| 86 | 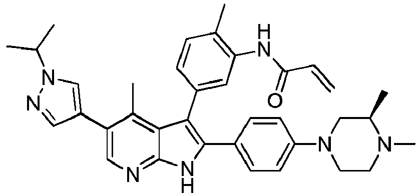 | 587 | 588 |
| 87 | 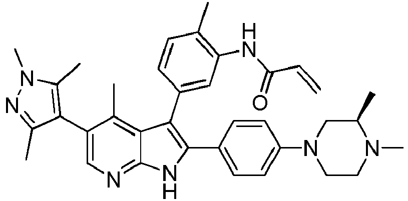 | 587 | 588 |
| 88 | 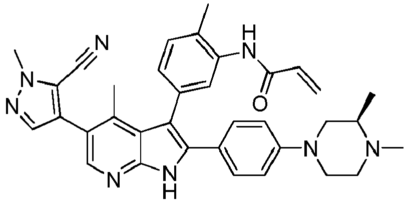 | 584 | 585 |
| 89 | 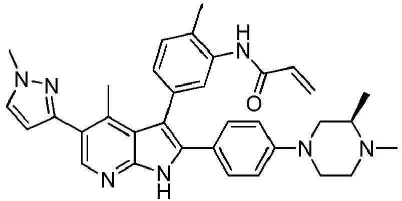 | 559 | 560 |
| 90 | 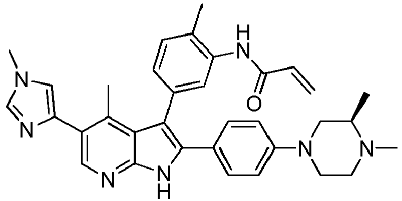 | 559 | 560 |

The Examples in the following table were prepared from 5-bromo-3-iodo-pyridin-2-amine, trimethyl((4-methyl-3-nitrophenyl)ethynyl)silane and (*S*)-1,2-dimethyl-4-(4-(4,4,5,5-tetramethyl-1,3,2-dioxaborolan-2-yl)phenyl)piperazine according to the procedure described for **A6** (Example 1).

| **Example** | **Name** | **Mwt** | **[M+H]⁺** |
|---|---|---|---|
| 91 | 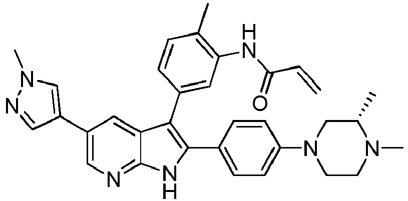 | 545 | 546 |
| 92 | 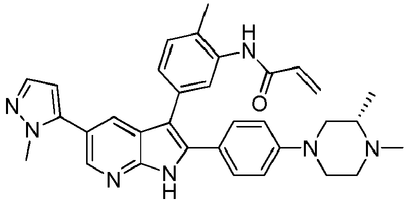 | 545 | 546 |
| 93 | 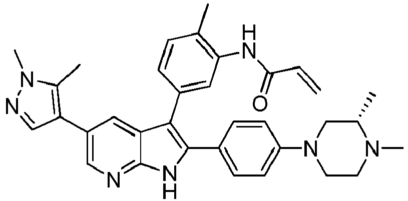 | 559 | 560 |
| 94 | 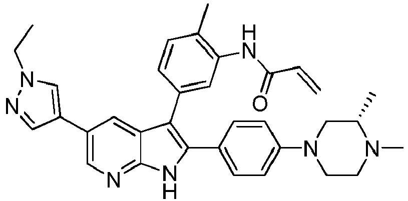 | 559 | 560 |
| 95 | 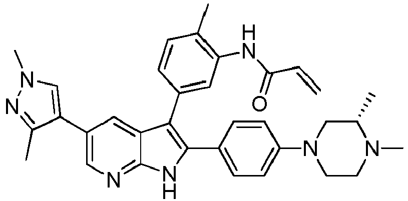 | 559 | 560 |
| 96 | 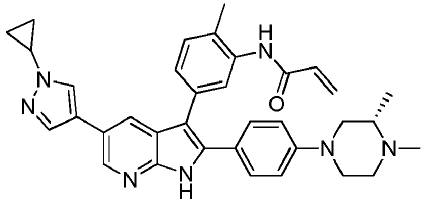 | 571 | 572 |
| 97 | 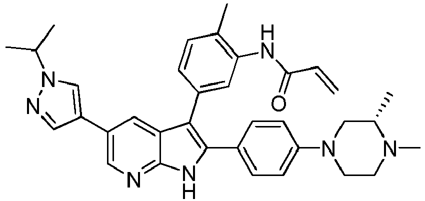 | 573 | 574 |
| 98 | 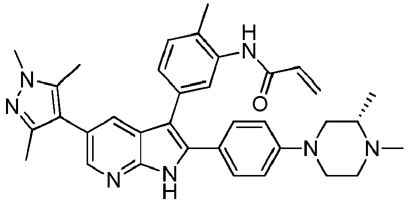 | 573 | 574 |
| 99 | 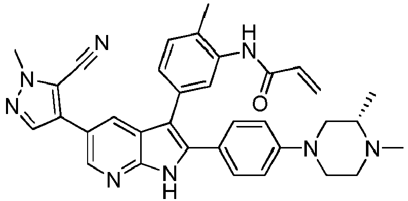 | 570 | 571 |
| 100 | 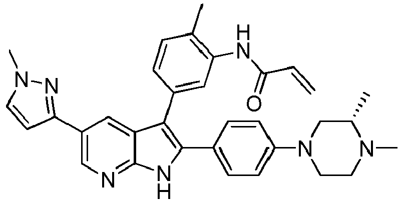 | 545 | 546 |
| 101 | 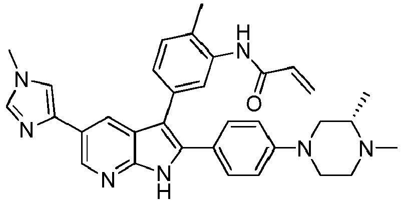 | 545 | 546 |

The Examples in the following table were prepared from 5-bromo-3-iodo-4-methylpyridin-2-amine, trimethyl((4-methyl-3-nitrophenyl)ethynyl)silane and (*S*)-1,2-dimethyl-4-(4-(4,4,5,5-tetramethyl-1,3,2-dioxaborolan-2-yl)phenyl)piperazine according to the procedure described for **A6** (Example 1).

| **Example** | **Name** | **Mwt** | **[M+H]⁺** |
|---|---|---|---|
| 102 | 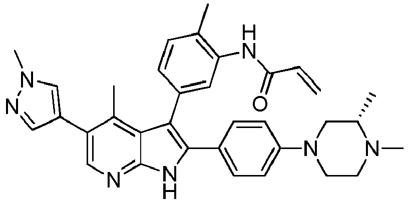 | 559 | 560 |
| 103 | 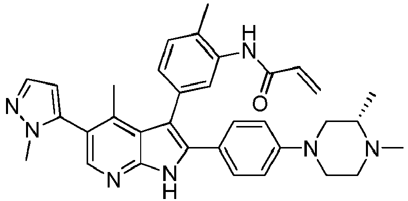 | 559 | 560 |
| 104 | 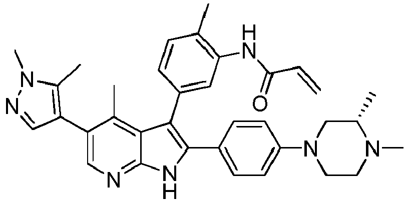 | 573 | 574 |
| 105 | 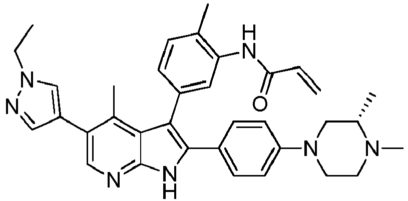 | 573 | 574 |
| 106 | 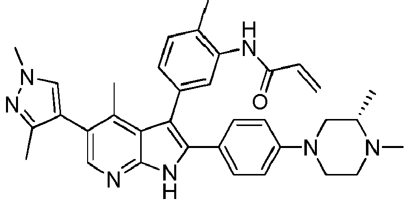 | 573 | 574 |
| 107 | 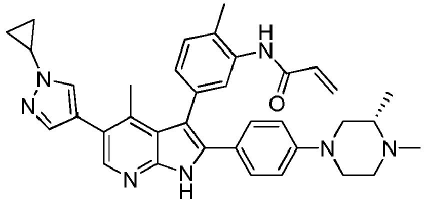 | 585 | 586 |
| 108 | 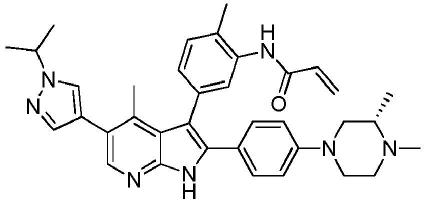 | 587 | 588 |
| 109 | 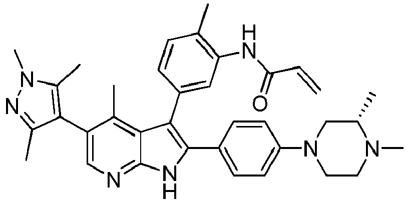 | 587 | 588 |
| 110 | 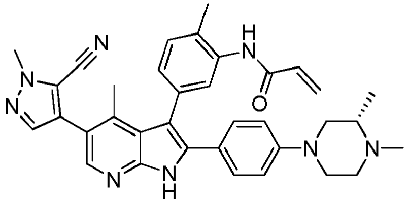 | 584 | 585 |
| 111 | 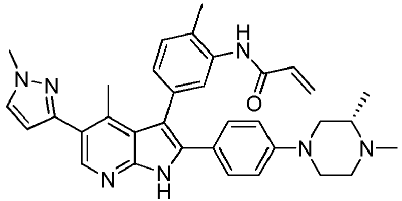 | 559 | 560 |
| 112 | 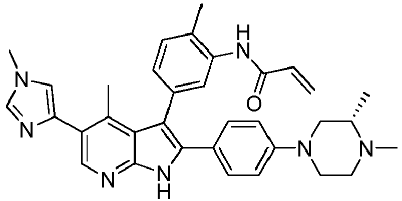 | 559 | 560 |

The Examples in the following table were prepared from 5-bromo-3-iodo-pyridin-2-amine, trimethyl((4-methyl-3-nitrophenyl)ethynyl)silane and N1,N1,N2-trimethyl-N2-(4-(4,4,5,5-tetramethyl-1,3,2-dioxaborolan-2-yl)phenyl)ethane-1,2-diamine according to the procedure described for **A6** (Example 1).

| **Example** | **Name** | **Mwt** | **[M+H]⁺** |
|---|---|---|---|
| 113 | 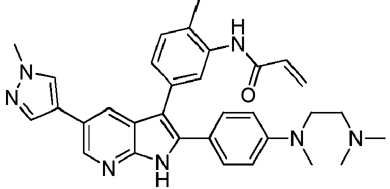 | 533 | 534 |
| 114 | 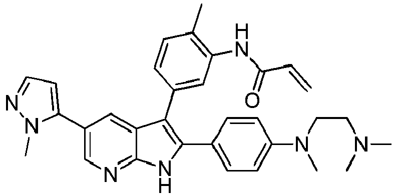 | 533 | 534 |
| 115 | 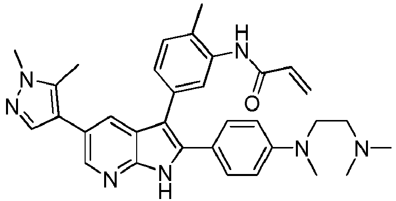 | 547 | 548 |
| 116 | 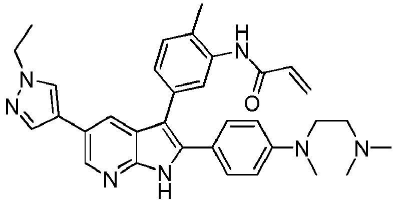 | 547 | 548 |
| 117 | 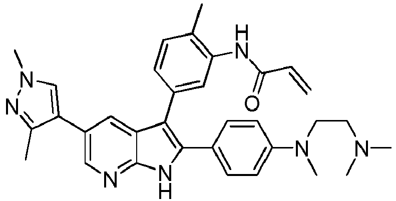 | 547 | 548 |
| 118 | 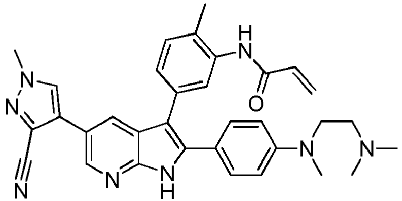 | 558 | 559 |
| 119 | 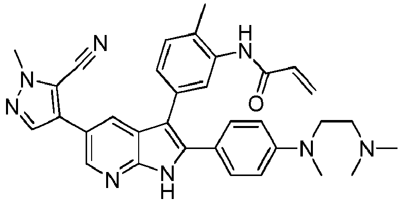 | 558 | 559 |
| 120 | 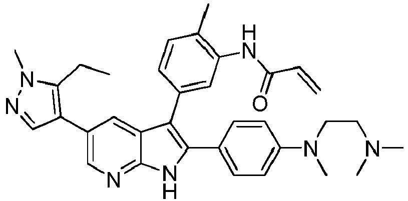 | 561 | 562 |

### Biological Assays

The exemplified compounds described herein were tested for activity and were found to have an IC₅₀ value less than 10 uM, particularly less than 500 nM, in one of the following assays:

### 1. Measurement of HER2 INS YVMA kinase activity

This protocol describes how the Lance Kinase Activity Assay was performed to determine IC₅₀ values of compounds of general formula (I) against HER2 INS YVMA. The principle behind this enzymatic assay is based upon the phosphorylation of the Ulight-peptide substrate. It is detected by using a specific EU-labeled anti-phospho peptide antibody. The binding of the Eu labeled anti-phospho peptide antibody to the phosphorylated ULight labeled peptide gives rise to a FRET-signal.

Binding of an inhibitor to the kinase prevents phosphorylation of the Ulight-substrate, resulting in a loss of FRET. In table 2 is summarized the relevant information for the LANCE assay.

**Table 2: Reagents, stock concentrations and final assay concentrations for Her2 INS YVMA.**

| Reagents | Stock concentration | Working concentration | Final assay concentration | Supplier |
|---|---|---|---|---|
| ULight™-Poly GT substrate | 10 µM | 250 nM | 100 nM | PerkinElmer |
| Eu-Anti-PT66 Antibody (AB) | 3125 nM | 4nM | 2 nM | PerkinElmer |
| Her2 ins YVMA | 41,83 µM | 2,5 nM | 1 nM | DPF |
| ATP | 100 mM | 8 µM | 1,6 µM | Sigma |

The compounds of general formula (I) summarized in Table 3 were serial diluted from a 10 mM DMSO stock solution 1:3 over 8 steps in a total volume of 20 µl.

For every sample, 8 µl of kinase-substrate mix was transferred into a suitable assay plate (e.g. Corning #3673). Compound was added via pintool transfer (10nl/well) using a Biomek FX robot (BeckmanCoulter). Reaction was started by addition of 2µl ATP working solution and mixed using variomag teleshaker (Thermo Fischer Scientific). After 1 h incubation at room temperature the reaction was stopped with 10µl detection mix containing the Eu-labeled phosphospecific antibody and 10mM EDTA. After a second incubation period of 1 h at room temperature the FRET signal was measured at 340 nm excitation, 665 nm and 615 nm emission (for the ULight-substrate and Eu-AB, respectively) with an Envision spectrophotometer (Perkin Elmer, Waltham, MA, USA) with 50 µs delay and 300 µs integration time. IC₅₀ values were determined from the sigmoidal dose response curves with the software Quattro Workflow (Quattro GmbH, Munich, Germany).

### 2. Measurement of cellular activity

The CellTiter-Glo Luminescent Cell Viability Assay (Promega) is a homogeneous method of determining the number of viable cells in culture. It is based on quantification of ATP, indicating the presence of metabolically active cells. Cells are seeded on day 1 at cell numbers that assure assay linearity and optimal signal intensity. After incubation for 24h in humidified chambers at 37°C and 5% CO₂, compounds in DMSO are added at different concentrations. Cells are further incubated for 72 h at 37°C and 5% CO₂. Cells treated with the compound vehicle DMSO are used as positive controls and cells treated with 10 µM Staurosporine serve as negative controls. At day 5 the CellTiter Glo Reagent is prepared according to the instructions of the kit (Promega Inc.): Reagent is mixed 1:1 with cell culture medium. Thereon, mixture and assay plates are equilibrated at room temperature for 20 min. Equal volumes of the reagent-medium-mixture is added to the volume of culture medium present in each well. The plates are mixed at ∼200 rpm for 2 minutes on an orbital shaker. The microplates are then incubated at room temperature for 10 minutes for stabilization of the luminescent signal. Following incubation the luminescence is recorded on a Victor microplate reader (Perkin Elmer) using a 200 ms integration time. The data is then analyzed with Excel using the XLFIT Plugin (dose response Fit 205) for IC₅₀-determination. As quality control the Z'-factor is calculated from 16 positive and negative control values. Only assay results showing a Z'-factor ≥ 0.5 are used for further analysis.

Table 3 shows activity data in the biochemical EGFR Exon 20 InsNPG and Her2 Exon 20 INSYVMA Lance assays as well in the cellular EGFR Exon 20 INSNPH Ba/F3 CellTiter-Glo, Her2 Exon 20 INSYVMA Ba/F3 CellTiter-Glo and Cuto-17 CellTiter-Glo assays. Inhibition is indicated as IC₅₀ [nM] ("-" = not measured). Compounds having an activity designated as "A" provided an IC₅₀ ≤ 50nM; compounds having an activity designated as "B" provided an 50nM < IC₅₀ ≤ 100nM; compounds having an activity designated as "C" provided an 100nM < IC₅₀ ≤ 500nM; compounds having an activity designated as "D" provided an 500nM < IC₅₀ ≤ 1000nM; compounds having an activity designated as "E" provided an 1000nM < IC₅₀ ≤ 10000nM; and compounds having an activity designated as "F" provided an IC₅₀ > 10000nM.

**Table 3:**

| **Example** | **EGFR Exon 20 InsNPG Lance IC₅₀ [nM]** | **Her2 Exon 20 InsYVMA Lance IC₅₀ [nM]** | **EGFR Exon 20 InsNPH Ba/F3 CTG IC₅₀ [nM]** | **Her2 Exon 20 InsYVMA Ba/F3 CTG IC₅₀ [nM]** | **Cuto-17 CTG IC₅₀ [nM]** |
|---|---|---|---|---|---|
| **1** | - | A | A | C | C |
| **2** | - | A | B | C | C |
| **3** | - | A | A | A | A |
| **4** | - | A | A | B | C |
| **5** | - | A | A | C | C |
| **6** | - | A | A | A | C |
| **7** | - | A | A | C | C |

Table 4 shows activity data in the biochemical EGFR T790ML858R Lance, EGFR L858R Lance, EGFR (del746-750) Lance and EGFR wt Lance assays. Inhibition is indicated as IC₅₀ [nM] ("-" = not measured). Compounds having an activity designated as "A" provided an IC₅₀ ≤ 50nM; compounds having an activity designated as "B" provided an 50nM < IC₅₀ ≤ 100nM; compounds having an activity designated as "C" provided an 100nM < IC₅₀ ≤ 500nM; compounds having an activity designated as "D" provided an 500nM < IC₅₀ ≤ 1000nM; compounds having an activity designated as "E" provided an 1000nM < IC₅₀ ≤ 10000nM; and compounds having an activity designated as "F" provided an IC₅₀ > 10000nM.

**Table 4:**

| **Example** | **EGFR T790M L858R Lance IC₅₀ / [nM]** | **EGFR L858R Lance IC₅₀ [nM]** | **EGFR (del746-750) Lance IC₅₀ [nM]** | **EGFR wt Lance IC₅₀ [nM]** |
|---|---|---|---|---|
| **1** | A | - | - | A |
| **2** | A | - | - | A |
| **3** | A | - | - | A |
| **4** | A | - | - | A |
| **5** | A | A | A | A |
| **6** | A | A | A | A |
| **7** | A | - | - | A |

Table 5 shows activity data in the cellular H1975 CellTiter-Glo, A431 CellTiter-Glo, EGFR L858RT790M Ba/F3 CellTiter-Glo, EGFR L858R Ba/F3 CellTiter-Glo and EGFR vIII Ba/F3 CellTiter-Glo assays. Inhibition is indicated as IC₅₀ [nM] ("-" = not measured). Compounds having an activity designated as "A" provided an IC₅₀ ≤ 50nM; compounds having an activity designated as "B" provided an 50nM < IC₅₀ ≤ 100nM; compounds having an activity designated as "C" provided an 100nM < IC₅₀ ≤ 500nM; compounds having an activity designated as "D" provided an 500nM < IC₅₀ ≤ 1000nM; compounds having an activity designated as "E" provided an 1000nM < IC₅₀ ≤ 10000nM; and compounds having an activity designated as "F" provided an IC₅₀ > 10000nM.

**Table 5:**

| **Example** | **H1975 CTG IC₅₀ [nM]** | **A431 CTG IC₅₀ [nM]** | **EGFR L858R T790M Ba/F3 CTG IC₅₀ [nM]** | **EGFR L858R Ba/F3 CTG IC₅₀ [nM]** | **EGFR vIII Ba/F3 CTG IC₅₀ [nM]** |
|---|---|---|---|---|---|
| **1** | C | E | - | - | A |
| **2** | C | E | - | - | A |
| **3** | B | D | - | - | A |
| **4** | C | D | - | - | A |
| **5** | D | E | - | - | B |
| **6** | C | E | A | A | A |
| **7** | C | E | - | - | A |

The compounds 8 to 112 show similar activity in comparison to compounds 1 to 7 so that compounds 1 to 7 are regarded as selected representative examples of all 112 compounds explicitly disclosed herein.

### SEQUENCE LIST

SEQ-ID No. 1: EGFR p.D770_N771insSVD

ttcaaaaaga tcaaagtgct gggctccggt gcgttcggca cggtgtataa gggactctgg
atcccagaag gtgagaaagt taaaattccc gtcgctatca aggaattaag agaagcaaca
tctccgaaag ccaacaagga aatcctcgat gaagcctacg tgatggccag cgtggactca
gtagacaacc cccacgtgtg ccgcctgctg ggcatctgcc tcacctccac cgtgcagctc
atcacgcagc tcatgccctt cggctgcctc ctggactatg tccgggaaca caaagacaat
attggctccc agtacctgct caactggtgt gtgcagatcg caaag

SEQ-ID No. 2: EGFR p.H773_V774insNPH

ttcaaaaaga tcaaagtgct gggctccggt gcgttcggca cggtgtataa gggactctgg
atcccagaag gtgagaaagt taaaattccc gtcgctatca aggaattaag agaagcaaca
tctccgaaag ccaacaagga aatcctcgat gaagcctacg tgatggccag cgtggacaac
ccccacaatc cacatgtgtg ccgcctgctg ggcatctgcc tcacctccac cgtgcagctc
atcacgcagc tcatgccctt cggctgcctc ctggactatg tccgggaaca caaagacaat
attggctccc agtacctgct caactggtgt gtgcagatcg caaag

SEQ-ID No. 3: EGFR p.V769_D770insASV

ttcaaaaaga tcaaagtgct gggctccggt gcgttcggca cggtgtataa gggactctgg
atcccagaag gtgagaaagt taaaattccc gtcgctatca aggaattaag agaagcaaca
tctccgaaag ccaacaagga aatcctcgat gaagcctacg tgatggccag cgtggcctca
gtcgacaacc cccacgtgtg ccgcctgctg ggcatctgcc tcacctccac cgtgcagctc
atcacgcagc tcatgccctt cggctgcctc ctggactatg tccgggaaca caaagacaat
attggctccc agtacctgct caactggtgt gtgcagatcg caaag

SEQ-ID No. 4: EGFR p.P772_H773insPR

ttcaaaaaga tcaaagtgct gggctccggt gcgttcggca cggtgtataa gggactctgg
atcccagaag gtgagaaagt taaaattccc gtcgctatca aggaattaag agaagcaaca
tctccgaaag ccaacaagga aatcctcgat gaagcctacg tgatggccag cgtggacaac
cccccgcgtc acgtgtgccg cctgctgggc atctgcctca cctccaccgt gcagctcatc
acgcagctca tgcccttcgg ctgcctcctg gactatgtcc gggaacacaa agacaatatt
ggctcccagt acctgctcaa ctggtgtgtg cagatcgcaa ag

SEQ-ID No. 5:  HER2 INS8 INS YVMA

acacctagcg gagcgatgcc caaccaggcg cagatgcgga tcctgaaaga gacggagctg
aggaaggtga aggtgcttgg atctggcgct tttggcacag tctacaaggg catctggatc
cctgatgggg agaatgtgaa aattccagtg gccatcaaag tgttgaggga aaacacatcc
cccaaagcca acaaagaaat cttagacgaa gcatacgtga tggcttacgt gatggctggt
gtgggctccc catatgtctc ccgccttctg ggcatctgcc tgacatccac ggtgcagctg
gtgacacagc ttatgcccta tggctgcctc ttagaccatg tccgggaaaa ccgcggacgc
ctgggctccc aggacctgct gaactggtgt atgcagattg ccaaggggat gagctacctg
gaggatgtgc ggctcgtaca cagggacttg gccgctcgga acgtgctggt caagagtccc
aaccatgtca aaattacaga c

6. SEQ-ID No. 6: EGFR T790M

tccaaactgc acctacggat gcactgggcc aggtcttgaa ggctgtccaa cgaatgggcc
taagatcccg tccatcgcca ctgggatggt gggggccctc ctcttgctgc tggtggtggc
cctggggatc ggcctcttca tgcgaaggcg ccacatcgtt cggaagcgca cgctgcggag
gctgctgcag gagagggagc ttgtggagcc tcttacaccc agtggagaag ctcccaacca
agctctcttg aggatcttga aggaaactga attcaaaaag atcaaagtgc tgggctccgg
tgcgttcggc acggtgtata agggactctg gatcccagaa ggtgagaaag ttaaaattcc
cgtcgctatc aaggaattaa gagaagcaac atctccgaaa gccaacaagg aaatcctcga
tgaagcctac gtgatggcca gcgtggacaa cccccacgtg tgccgcctgc tgggcatctg
cctcacctcc accgtgcagc tcatcatgca gctcatgccc ttcggctgcc tcctggacta
tgtccgggaa cacaaagaca atattggctc ccagtacctg ctcaactggt gtgtgcagat
cgcaaagggc atgaactact tggaggaccg tcgcttggtg caccgcgacc tggcagccag
gaacgtactg gtgaaaacac cgcagcatgt caagatcaca gattttgggc tggccaaact
gctgggtgcg gaagagaaag aataccatgc agaaggaggc aaagtgccta tcaagtggat
ggcattggaa tcaattttac acagaatcta tacccaccag agtgatgtct ggagctacgg
ggtgaccgtt tgggagttga tgacctttgg atccaagcca tatgacggaa tccctgccag
cgagatctcc tccatcctgg agaaaggaga acgcctccct cagccaccca tatgtaccat
cgatgtctac atgatcatgg

7. SEQ-ID No. 7: EGFR T790ML858R

tccaaactgc acctacggat gcactgggcc aggtcttgaa ggctgtccaa cgaatgggcc
taagatcccg tccatcgcca ctgggatggt gggggccctc ctcttgctgc tggtggtggc
cctggggatc ggcctcttca tgcgaaggcg ccacatcgtt cggaagcgca cgctgcggag
gctgctgcag gagagggagc ttgtggagcc tcttacaccc agtggagaag ctcccaacca
agctctcttg aggatcttga aggaaactga attcaaaaag atcaaagtgc tgggctccgg
tgcgttcggc acggtgtata agggactctg gatcccagaa ggtgagaaag ttaaaattcc
cgtcgctatc aaggaattaa gagaagcaac atctccgaaa gccaacaagg aaatcctcga
tgaagcctac gtgatggcca gcgtggacaa cccccacgtg tgccgcctgc tgggcatctg
cctcacctcc accgtgcagc tcatcatgca gctcatgccc ttcggctgcc tcctggacta
tgtccgggaa cacaaagaca atattggctc ccagtacctg ctcaactggt gtgtgcagat
cgcaaagggc atgaactact tggaggaccg tcgcttggtg caccgcgacc tggcagccag
gaacgtactg gtgaaaacac cgcagcatgt caagatcaca gattttgggc gggccaaact
gctgggtgcg gaagagaaag aataccatgc agaaggaggc aaagtgccta tcaagtggat
ggcattggaa tcaattttac acagaatcta tacccaccag agtgatgtct ggagctacgg
ggtgaccgtt tgggagttga tgacctttgg atccaagcca tatgacggaa tccctgccag
cgagatctcc tccatcctgg agaaaggaga acgcctccct cagccaccca tatgtaccat
cgatgtctac atgatcatgg

## Claims

1. A compound of the formula (I) 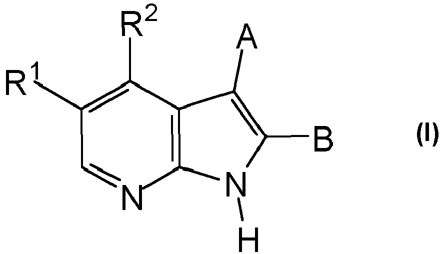 wherein
**A** represents 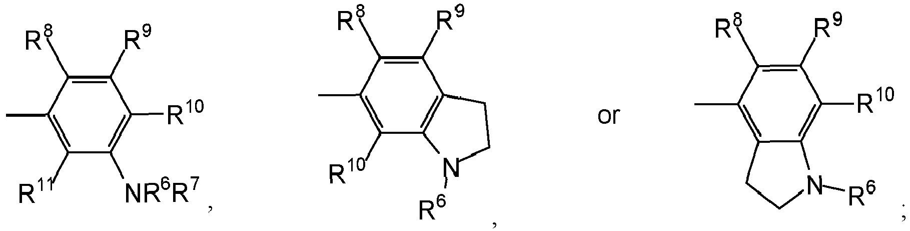
**B** represents 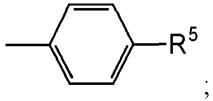
**R¹** represents 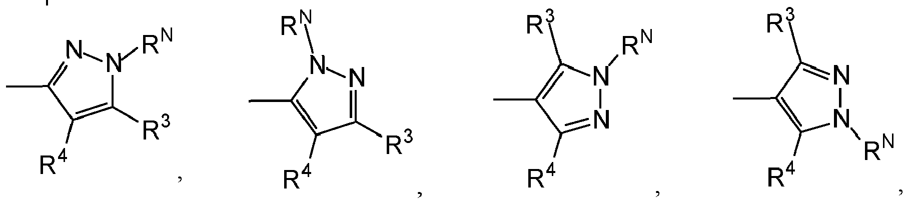 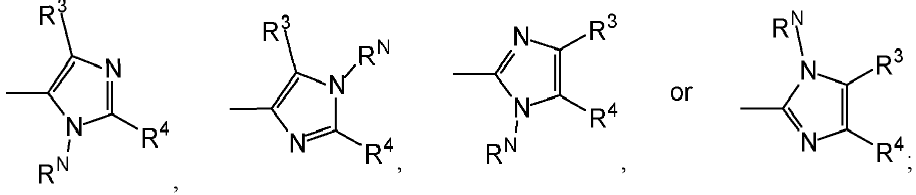
**R²** represents -H, -F, -CI, -Br, -CN, -CH₃, -C₂H₅, -C₃H₇, -OCH₃, -OC₂H₅, or -OC₃H₇;
**R³** and **R⁴** represent independently of each other -H, -F, -CI, -CN, -CF₃, -OCH₃, -OC₂H₅, -CH₂OCH₃, -CH₃, -C₂H₅, or -C₃H₇;
**R⁵** represents 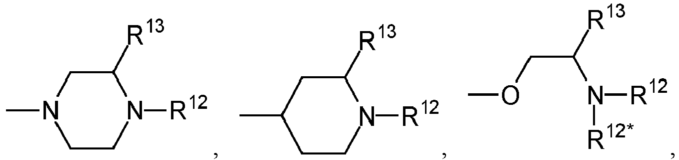 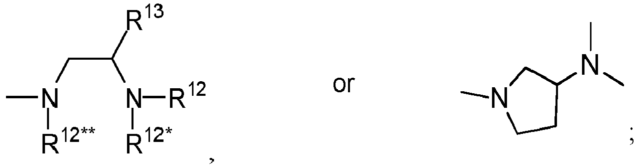
**R^{N}** represent -H, -CH₃, -CF₃, -C₂H₅, -C₃H₇, -CH(CH₃)₂, -CH₂CH(CH₃)₂, -C₄H₉, -cyclo-C₃H₅, -cyclo-C₄H₇, -CH₂CH₂OH, -CH₂CH(CH₃)OH, -CH₂CH₂OCH₃, -CH₂CH₂CH₂OH, -CH₂CH₂CH₂OCH₃, or -CH₂CH₂N(CH₃)₂;
**R⁶** represents 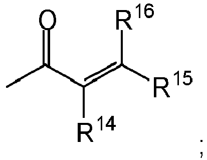
**R⁷** represents -H, -CH₃, or -C₂H₅;
**R⁸** - **R¹¹** represent independently of each other -H, -F, -CI, -CN, -OCH₃, -OC₂H₅, -CH₃, -CF₃, or -C₂H₅;
**R¹²**, **R^{12*}**, **R**^{**12****}, and **R¹³** represent independently of each other -H, -CH₃, or -C₂H₅;
**R¹⁴**, **R¹⁵,** and **R¹⁶** represent independently of each other -H, -CH₃, -CF₃, -CN, -NO₂, -COCH₃, or -COC₂H₅;
or an enantiomer, a mixture of enantiomers, a diastereomer, a mixture of diastereomers, a tautomer, a hydrate, a solvate, or pharmaceutically acceptable salts thereof.

2. The compound according to Claim 1, wherein B represents 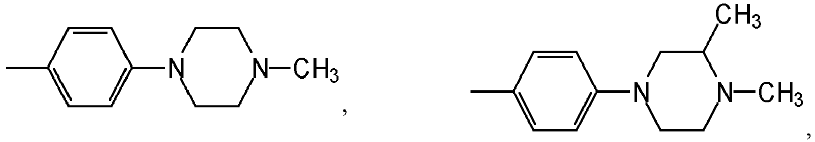 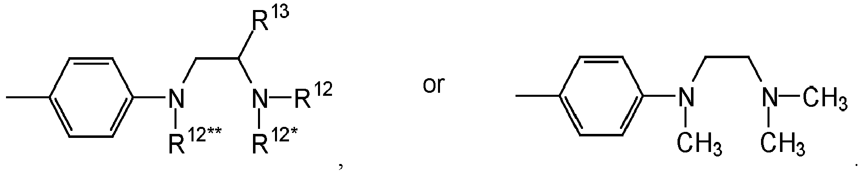 and **R¹²**, **R^{12*}**, **R^{12**}**, and **R¹³** have the meanings as defined in Claim 1.

3. The compound according to Claim 1 or 2, wherein A represents 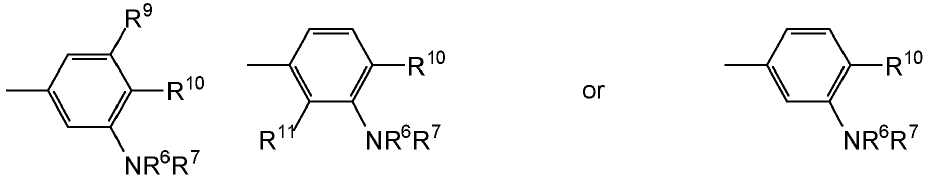 and **R⁶, R⁷** and **R⁹**- **R¹¹** have the meanings as defined in Claim 1.

4. The compound according to any one of the Claims 1 - 3, wherein **R¹** represents 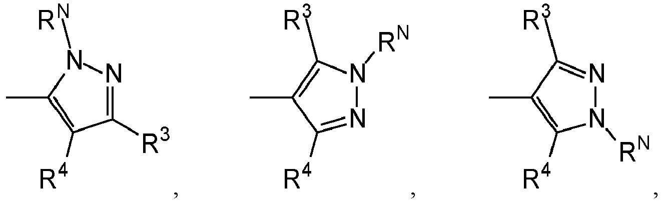 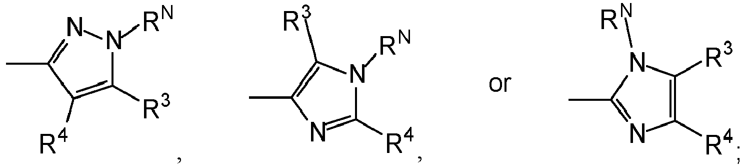 **R³**, **R⁴**, and **R^{N}** have the meanings as defined in Claim 1.

5. The compound according to any one of the Claims 1 - 4, wherein **R⁶** represents

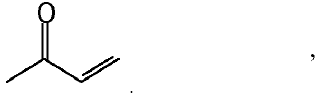

6. The compound according to Claim 1 selected from the group consisting of:
N-(2-methyl-5-(5-(1-methyl-1H-pyrazol-4-yl)-2-(4-(4-methylpiperazin-1-yl)phenyl)-1H-pyrrolo[2,3-b]pyridin-3-yl)phenyl)acrylamide,
N-(2-methyl-5-(5-(1-methyl-1H-pyrazol-5-yl)-2-(4-(4-methylpiperazin-1-yl)phenyl)-1H-pyrrolo[2,3-b]pyridin-3-yl)phenyl)acrylamide,
N-(5-(5-(1,5-dimethyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(5-(1-ethyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(5-(1,3-dimethyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(5-(1-cyclopropyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(5-(1-(2-methoxyethyl)-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(5-(1-isopropyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(2-methyl-5-(2-(4-(4-methylpiperazin-1-yl)phenyl)-5-(1,3,5-trimethyl-1H-pyrazol-4-yl)-1H-pyrrolo[2,3-b]pyridin-3-yl)phenyl)acrylamide,
N-(5-(5-(5-cyano-1-methyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(2-methyl-5-(5-(1-methyl-1H-pyrazol-3-yl)-2-(4-(4-methylpiperazin-1-yl)phenyl)-1H-pyrrolo[2,3-b]pyridin-3-yl)phenyl)acrylamide,
N-(2-methyl-5-(2-(4-(4-methylpiperazin-1-yl)phenyl)-5-(1-(trifluoromethyl)-1H-pyrazol-4-yl)-1H-pyrrolo[2,3-b]pyridin-3-yl)phenyl)acrylamide,
N-(5-(5-(1-(2-hydroxyethyl)-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(5-(1-(2-hydroxypropyl)-1H-pyrazol-4-yl)-2-(4-(4-methylpiperazin-1-yl)phenyl)-1H-pyrrolo[2,3-b]pyridin-3-yl)-2-methylphenyl)acrylamide,
N-(2-methyl-5-(2-(4-(4-methylpiperazin-1-yl)phenyl)-5-(1H-pyrazol-4-yl)-1H-pyrrolo[2,3-b]pyridin-3-yl)phenyl)acrylamide,
N-(2-methyl-5-(5-(1-methyl-1H-imidazol-4-yl)-2-(4-(4-methylpiperazin-1-yl)phenyl)-1H-pyrrolo[2,3-b]pyridin-3-yl)phenyl)acrylamide,
N-(5-(5-(1,5-dimethyl-1H-imidazol-4-yl)-2-(4-(4-methylpiperazin-1-yl)phenyl)-1H-pyrrolo[2,3-b]pyridin-3-yl)-2-methylphenyl)acrylamide,
N-(2-methyl-5-(5-(1-methyl-1H-imidazol-2-yl)-2-(4-(4-methylpiperazin-1-yl)phenyl)-1H-pyrrolo[2,3-b]pyridin-3-yl)phenyl)acrylamide,
N-(5-(5-(1-ethyl-1H-imidazol-4-yl)-2-(4-(4-methylpiperazin-1-yl)phenyl)-1H-pyrrolo[2,3-b]pyridin-3-yl)-2-methylphenyl)acrylamide,
N-(5-(4-ethyl-5-(1-methyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(5-(1,5-dimethyl-1H-pyrazol-4-yl)-4-ethyl-2-(4-(4-methylpiperazin-1-yl)phenyl)-1H-pyrrolo[2,3-b]pyridin-3-yl)-2-methylphenyl)acrylamide,
N-(5-(4-chloro-5-(1-methyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(4-chloro-5-(1,5-dimethyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(4-chloro-5-(1,3-dimethyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(4-chloro-5-(1-ethyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(4-chloro-5-(1-methyl-1H-imidazol-4-yl)-2-(4-(4-methylpiperazin-1-yl)phenyl)-1H-pyrrolo[2,3-b]pyridin-3-yl)-2-methylphenyl)acrylamide,
N-(5-(4-chloro-5-(1,5-dimethyl-1H-imidazol-4-yl)-2-(4-(4-methylpiperazin-1-yl)phenyl)-1H-pyrrolo[2,3-b]pyridin-3-yl)-2-methylphenyl)acrylamide,
N-(5-(4-chloro-5-(1-ethyl-1H-imidazol-4-yl)-2-(4-(4-methylpiperazin-1-yl)phenyl)-1H-pyrrolo[2,3-b]pyridin-3-yl)-2-methylphenyl)acrylamide,
N-(5-(4-methoxy-5-(1-methyl-1H-pyrazol-4-yl)-2-(4-(4-methylpiperazin-1-yl)phenyl)-1H-pyrrolo[2,3-b]pyridin-3-yl)-2-methylphenyl)acrylamide,
N-(5-(5-(1,5-dimethyl-1H-pyrazol-4-yl)-4-methoxy-2-(4-(4-methylpiperazin-1-yl)phenyl)-1H-pyrrolo[2,3-b]pyridin-3-yl)-2-methylphenyl)acrylamide,
N-(5-(5-(1,3-dimethyl-1H-pyrazol-4-yl)-4-methoxy-2-(4-(4-methylpiperazin-1-yl)phenyl)-1H-pyrrolo[2,3-b]pyridin-3-yl)-2-methylphenyl)acrylamide,
N-(5-(5-(1-ethyl-1H-pyrazol-4-yl)-4-methoxy-2-(4-(4-methylpiperazin-1-yl)phenyl)-1H-pyrrolo[2,3-b]pyridin-3-yl)-2-methylphenyl)acrylamide,
N-(5-(4-methoxy-5-(1-methyl-1H-imidazol-4-yl)-2-(4-(4-methylpiperazin-1-yl)phenyl)-1H-pyrrolo[2,3-b]pyridin-3-yl)-2-methylphenyl)acrylamide,
N-(5-(5-(1,5-dimethyl-1H-imidazol-4-yl)-4-methoxy-2-(4-(4-methylpiperazin-1-yl)phenyl)-1H-pyrrolo[2,3-b]pyridin-3-yl)-2-methylphenyl)acrylamide,
N-(5-(5-(1-ethyl-1H-imidazol-4-yl)-4-methoxy-2-(4-(4-methylpiperazin-1-yl)phenyl)-1H-pyrrolo[2,3-b]pyridin-3-yl)-2-methylphenyl)acrylamide,
N-(2-methyl-5-(4-methyl-5-(1-methyl-1H-pyrazol-4-yl)-2-(4-(4-methylpiperazin-1-yl)phenyl)-1 H-pyrrolo[2,3-b]pyridin-3-yl)phenyl)acrylamide,
N-(5-(5-(1,5-dimethyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide,
N-(2-methyl-5-(4-methyl-5-(1-methyl-1H-pyrazol-5-yl)-2-(4-(4-methylpiperazin-1-yl)phenyl)-1H-pyrrolo[2,3-b]pyridin-3-yl)phenyl)acrylamide,
N-(5-(5-(1-ethyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide,
N-(5-(5-(1,3-dimethyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide,
N-(5-(5-(1-cyclopropyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide,
N-(5-(5-(1-(2-methoxyethyl)-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide,
N-(5-(5-(1-isopropyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide,
N-(2-methyl-5-(4-methyl-2-(4-(4-methylpiperazin-1-yl)phenyl)-5-(1,3,5-trimethyl-1H-pyrazol-4-yl)-1H-pyrrolo[2,3-b]pyridin-3-yl)phenyl)acrylamide,
N-(5-(5-(5-cyano-1-methyl-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide,
N-(2-methyl-5-(4-methyl-5-(1-methyl-1H-pyrazol-3-yl)-2-(4-(4-methylpiperazin-1-yl)phenyl)-1H-pyrrolo[2,3-b]pyridin-3-yl)phenyl)acrylamide,
N-(2-methyl-5-(4-methyl-2-(4-(4-methylpiperazin-1-yl)phenyl)-5-(1-(trifluoromethyl)-1H-pyrazol-4-yl)-1H-pyrrolo[2,3-b]pyridin-3-yl)phenyl)acrylamide,
N-(5-(5-(1-(2-hydroxyethyl)-1H-pyrazol-4-yl)-4-methyl-2-(4-(4-methylpiperazin-1-yl)phenyl)-1H-pyrrolo[2,3-b]pyridin-3-yl)-2-methylphenyl)acrylamide,
N-(2-methyl-5-(4-methyl-5-(1-methyl-1H-imidazol-4-yl)-2-(4-(4-methylpiperazin-1-yl)phenyl)-1H-pyrrolo[2,3-b]pyridin-3-yl)phenyl)acrylamide,
N-(5-(5-(1,5-dimethyl-1H-imidazol-4-yl)-4-methyl-2-(4-(4-methylpiperazin-1-yl)phenyl)-1H-pyrrolo[2,3-b]pyridin-3-yl)-2-methylphenyl)acrylamide,
N-(2,6-dimethyl-3-(5-(1-methyl-1H-pyrazol-4-yl)-2-(4-(4-methylpiperazin-1-yl)phenyl)-1H-pyrrolo[2,3-b]pyridin-3-yl)phenyl)acrylamide,
N-(3-(5-(1-ethyl-1H-pyrazol-4-yl)-2-(4-(4-methylpiperazin-1-yl)phenyl)-1H-pyrrolo[2,3-b]pyridin-3-yl)-2,6-dimethylphenyl)acrylamide,
N-(3-(5-(1,5-dimethyl-1H-pyrazol-4-yl)-2-(4-(4-methylpiperazin-1-yl)phenyl)-1H-pyrrolo[2,3-b]pyridin-3-yl)-2,6-dimethylphenyl)acrylamide,
N-(3-(5-(1,3-dimethyl-1H-pyrazol-4-yl)-2-(4-(4-methylpiperazin-1-yl)phenyl)-1H-pyrrolo[2,3-b]pyridin-3-yl)-2,6-dimethylphenyl)acrylamide,
N-(3-fluoro-2-methyl-5-(5-(1-methyl-1H-pyrazol-4-yl)-2-(4-(4-methylpiperazin-1-yl)phenyl)-1H-pyrrolo[2,3-b]pyridin-3-yl)phenyl)acrylamide,
N-(5-(5-(1-ethyl-1H-pyrazol-4-yl)-2-(4-(4-methylpiperazin-1-yl)phenyl)-1H-pyrrolo[2,3-b]pyridin-3-yl)-3-fluoro-2-methylphenyl)acrylamide,
N-(5-(5-(1,5-dimethyl-1H-pyrazol-4-yl)-2-(4-(4-methylpiperazin-1-yl)phenyl)-1H-pyrrolo[2,3-b]pyridin-3-yl)-3-fluoro-2-methylphenyl)acrylamide,
N-(5-(5-(1,3-dimethyl-1H-pyrazol-4-yl)-2-(4-(4-methylpiperazin-1-yl)phenyl)-1H-pyrrolo[2,3-b]pyridin-3-yl)-3-fluoro-2-methylphenyl)acrylamide, N-(3-fluoro-2,6-dimethyl-5-(5-(1-methyl-1H-pyrazol-4-yl)-2-(4-(4-methylpiperazin-1-yl)phenyl)-1H-pyrrolo[2,3-b]pyridin-3-yl)phenyl)acrylamide,
N-(3-(5-(1,5-dimethyl-1H-pyrazol-4-yl)-2-(4-(4-methylpiperazin-1-yl)phenyl)-1H-pyrrolo[2,3-b]pyridin-3-yl)-5-fluoro-2,6-dimethylphenyl)acrylamide,
1-(6-(5-(1-methyl-1H-pyrazol-4-yl)-2-(4-(4-methylpiperazin-1-yl)phenyl)-1H-pyrrolo[2,3-b]pyridin-3-yl)indolin-1-yl)prop-2-en-1-one,
1-(6-(5-(1,5-dimethyl-1H-pyrazol-4-yl)-2-(4-(4-methylpiperazin-1-yl)phenyl)-1H-pyrrolo[2,3-b]pyridin-3-yl)indolin-1-yl)prop-2-en-1-one,
1-(4-(5-(1-methyl-1H-pyrazol-4-yl)-2-(4-(4-methylpiperazin-1-yl)phenyl)-1H-pyrrolo[2,3-b]pyridin-3-yl)indolin-1-yl)prop-2-en-1-one,
1-(4-(5-(1,5-dimethyl-1H-pyrazol-4-yl)-2-(4-(4-methylpiperazin-1-yl)phenyl)-1H-pyrrolo[2,3-b]pyridin-3-yl)indolin-1-yl)prop-2-en-1-one,
N-(2-methoxy-6-methyl-3-(5-(1-methyl-1H-pyrazol-4-yl)-2-(4-(4-methylpiperazin-1-yl)phenyl)-1H-pyrrolo[2,3-b]pyridin-3-yl)phenyl)acrylamide,
N-(3-(5-(1,5-dimethyl-1H-pyrazol-4-yl)-2-(4-(4-methylpiperazin-1-yl)phenyl)-1H-pyrrolo[2,3-b]pyridin-3-yl)-2-methoxy-6-methylphenyl)acrylamide,
N-methyl-N-(3-(5-(1-methyl-1H-pyrazol-4-yl)-2-(4-(4-methylpiperazin-1-yl)phenyl)-1H-pyrrolo[2,3-b]pyridin-3-yl)phenyl)acrylamide,
N-(3-(5-(1,5-dimethyl-1H-pyrazol-4-yl)-2-(4-(4-methylpiperazin-1-yl)phenyl)-1H-pyrrolo[2,3-b]pyridin-3-yl)phenyl)-N-methylacrylamide,
(R)-N-(5-(2-(4-(3,4-dimethylpiperazin-1-yl)phenyl)-5-(1-methyl-1H-pyrazol-4-yl)-1H-pyrrolo[2,3-b]pyridin-3-yl)-2-methylphenyl)acrylamide,
(R)-N-(5-(2-(4-(3,4-dimethylpiperazin-1-yl)phenyl)-5-(1-methyl-1H-pyrazol-5-yl)-1H-pyrrolo[2,3-b]pyridin-3-yl)-2-methylphenyl)acrylamide,
(R)-N-(5-(5-(1,5-dimethyl-1H-pyrazol-4-yl)-2-(4-(3,4-dimethylpiperazin-1-yl)phenyl)-1H-pyrrolo[2,3-b]pyridin-3-yl)-2-methylphenyl)acrylamide,
(R)-N-(5-(2-(4-(3,4-dimethylpiperazin-1-yl)phenyl)-5-(1-ethyl-1H-pyrazol-4-yl)-1H-pyrrolo[2, 3-b]pyridin-3-yl)-2-methylphenyl)acrylamide,
(R)-N-(5-(5-(1,3-dimethyl-1H-pyrazol-4-yl)-2-(4-(3,4-dimethylpiperazin-1-yl)phenyl)-1H-pyrrolo[2,3-b]pyridin-3-yl)-2-methylphenyl)acrylamide,
(R)-N-(5-(5-(1-cyclopropyl-1H-pyrazol-4-yl)-2-(4-(3,4-dimethylpiperazin-1-yl)phenyl)-1H-pyrrolo[2,3-b]pyridin-3-yl)-2-methylphenyl)acrylamide,
(R)-N-(5-(2-(4-(3,4-dimethylpiperazin-1-yl)phenyl)-5-(1-isopropyl-1H-pyrazol-4-yl)-1H-pyrrolo[2,3-b]pyridin-3-yl)-2-methylphenyl)acrylamide,
(R)-N-(5-(2-(4-(3,4-dimethylpiperazin-1-yl)phenyl)-5-(1,3,5-trimethyl-1H-pyrazol-4-yl)-1H-pyrrolo[2,3-b]pyridin-3-yl)-2-methylphenyl)acrylamide,
(R)-N-(5-(5-(5-cyano-1-methyl-1H-pyrazol-4-yl)-2-(4-(3,4-dimethylpiperazin-1-yl)phenyl)-1H-pyrrolo[2,3-b]pyridin-3-yl)-2-methylphenyl)acrylamide,
(R)-N-(5-(2-(4-(3,4-dimethylpiperazin-1-yl)phenyl)-5-(1-methyl-1H-pyrazol-3-yl)-1H-pyrrolo[2,3-b]pyridin-3-yl)-2-methylphenyl)acrylamide,
(R)-N-(5-(2-(4-(3,4-dimethylpiperazin-1-yl)phenyl)-5-(1-methyl-1H-imidazol-4-yl)-1H-pyrrolo[2,3-b]pyridin-3-yl)-2-methylphenyl)acrylamide,
(R)-N-(5-(2-(4-(3,4-dimethylpiperazin-1-yl)phenyl)-4-methyl-5-(1-methyl-1H-pyrazol-4-yl)-1H-pyrrolo[2,3-b]pyridin-3-yl)-2-methylphenyl)acrylamide,
(R)-N-(5-(2-(4-(3,4-dimethylpiperazin-1-yl)phenyl)-4-methyl-5-(1-methyl-1H-pyrazol-5-yl)-1H-pyrrolo[2,3-b]pyridin-3-yl)-2-methylphenyl)acrylamide,
(R)-N-(5-(5-(1,5-dimethyl-1H-pyrazol-4-yl)-2-(4-(3,4-dimethylpiperazin-1-yl)phenyl)-4-methyl-1H-pyrrolo[2,3-b]pyridin-3-yl)-2-methylphenyl)acrylamide,
(R)-N-(5-(2-(4-(3,4-dimethylpiperazin-1-yl)phenyl)-5-(1-ethyl-1H-pyrazol-4-yl)-4-methyl-1H-pyrrolo[2,3-b]pyridin-3-yl)-2-methylphenyl)acrylamide,
(R)-N-(5-(5-(1,3-dimethyl-1H-pyrazol-4-yl)-2-(4-(3,4-dimethylpiperazin-1-yl)phenyl)-4-methyl-1H-pyrrolo[2,3-b]pyridin-3-yl)-2-methylphenyl)acrylamide,
(R)-N-(5-(5-(1-cyclopropyl-1H-pyrazol-4-yl)-2-(4-(3,4-dimethylpiperazin-1-yl)phenyl)-4-methyl-1H-pyrrolo[2,3-b]pyridin-3-yl)-2-methylphenyl)acrylamide,
(R)-N-(5-(2-(4-(3,4-dimethylpiperazin-1-yl)phenyl)-5-(1-isopropyl-1H-pyrazol-4-yl)-4-methyl-1H-pyrrolo[2,3-b]pyridin-3-yl)-2-methylphenyl)acrylamide,
(R)-N-(5-(2-(4-(3,4-dimethylpiperazin-1-yl)phenyl)-4-methyl-5-(1,3,5-trimethyl-1H-pyrazol-4-yl)-1H-pyrrolo[2,3-b]pyridin-3-yl)-2-methylphenyl)acrylamide,
(R)-N-(5-(5-(5-cyano-1-methyl-1H-pyrazol-4-yl)-2-(4-(3,4-dimethylpiperazin-1-yl)phenyl)-4-methyl-1H-pyrrolo[2,3-b]pyridin-3-yl)-2-methylphenyl)acrylamide,
(R)-N-(5-(2-(4-(3,4-dimethylpiperazin-1-yl)phenyl)-4-methyl-5-(1-methyl-1H-pyrazol-3-yl)-1H-pyrrolo[2,3-b]pyridin-3-yl)-2-methylphenyl)acrylamide,
(R)-N-(5-(2-(4-(3,4-dimethylpiperazin-1-yl)phenyl)-4-methyl-5-(1-methyl-1H-imidazol-4-yl)-1H-pyrrolo[2,3-b]pyridin-3-yl)-2-methylphenyl)acrylamide,
(S)-N-(5-(2-(4-(3,4-dimethylpiperazin-1-yl)phenyl)-5-(1-methyl-1H-pyrazol-4-yl)-1H-pyrrolo[2,3-b]pyridin-3-yl)-2-methylphenyl)acrylamide,
(S)-N-(5-(2-(4-(3,4-dimethylpiperazin-1-yl)phenyl)-5-(1-methyl-1H-pyrazol-5-yl)-1H-pyrrolo[2,3-b]pyridin-3-yl)-2-methylphenyl)acrylamide,
(S)-N-(5-(5-(1,5-dimethyl-1H-pyrazol-4-yl)-2-(4-(3,4-dimethylpiperazin-1-yl)phenyl)-1H-pyrrolo[2,3-b]pyridin-3-yl)-2-methylphenyl)acrylamide,
(S)-N-(5-(2-(4-(3,4-dimethylpiperazin-1-yl)phenyl)-5-(1-ethyl-1H-pyrazol-4-yl)-1H-pyrrolo[2,3-b]pyridin-3-yl)-2-methylphenyl)acrylamide,
(S)-N-(5-(5-(1,3-dimethyl-1H-pyrazol-4-yl)-2-(4-(3,4-dimethylpiperazin-1-yl)phenyl)-1H-pyrrolo[2,3-b]pyridin-3-yl)-2-methylphenyl)acrylamide,
(S)-N-(5-(5-(1-cyclopropyl-1H-pyrazol-4-yl)-2-(4-(3,4-dimethylpiperazin-1-yl)phenyl)-1H-pyrrolo[2,3-b]pyridin-3-yl)-2-methylphenyl)acrylamide, (S)-N-(5-(2-(4-(3,4-dimethylpiperazin-1-yl)phenyl)-5-(1-isopropyl-1H-pyrazol-4-yl)-1H-pyrrolo[2,3-b]pyridin-3-yl)-2-methylphenyl)acrylamide,
(S)-N-(5-(2-(4-(3,4-dimethylpiperazin-1-yl)phenyl)-5-(1,3,5-trimethyl-1H-pyrazol-4-yl)-1H-pyrrolo[2,3-b]pyridin-3-yl)-2-methylphenyl)acrylamide,
(S)-N-(5-(5-(5-cyano-1-methyl-1H-pyrazol-4-yl)-2-(4-(3,4-dimethylpiperazin-1-yl)phenyl)-1H-pyrrolo[2,3-b]pyridin-3-yl)-2-methylphenyl)acrylamide,
(S)-N-(5-(2-(4-(3,4-dimethylpiperazin-1-yl)phenyl)-5-(1-methyl-1H-pyrazol-3-yl)-1H-pyrrolo[2,3-b]pyridin-3-yl)-2-methylphenyl)acrylamide,
(S)-N-(5-(2-(4-(3,4-dimethylpiperazin-1-yl)phenyl)-5-(1-methyl-1H-imidazol-4-yl)-1H-pyrrolo[2,3-b]pyridin-3-yl)-2-methylphenyl)acrylamide,
(S)-N-(5-(2-(4-(3,4-dimethylpiperazin-1-yl)phenyl)-4-methyl-5-(1-methyl-1H-pyrazol-4-yl)-1H-pyrrolo[2,3-b]pyridin-3-yl)-2-methylphenyl)acrylamide,
(S)-N-(5-(2-(4-(3,4-dimethylpiperazin-1-yl)phenyl)-4-methyl-5-(1-methyl-1H-pyrazol-5-yl)-1H-pyrrolo[2,3-b]pyridin-3-yl)-2-methylphenyl)acrylamide,
(S)-N-(5-(5-(1,5-dimethyl-1H-pyrazol-4-yl)-2-(4-(3,4-dimethylpiperazin-1-yl)phenyl)-4-methyl-1H-pyrrolo[2,3-b]pyridin-3-yl)-2-methylphenyl)acrylamide,
(S)-N-(5-(2-(4-(3,4-dimethylpiperazin-1-yl)phenyl)-5-(1-ethyl-1H-pyrazol-4-yl)-4-methyl-1H-pyrrolo[2,3-b]pyridin-3-yl)-2-methylphenyl)acrylamide,
(S)-N-(5-(5-(1,3-dimethyl-1H-pyrazol-4-yl)-2-(4-(3,4-dimethylpiperazin-1-yl)phenyl)-4-methyl-1H-pyrrolo[2,3-b]pyridin-3-yl)-2-methylphenyl)acrylamide,
(S)-N-(5-(5-(1-cyclopropyl-1H-pyrazol-4-yl)-2-(4-(3,4-dimethylpiperazin-1-yl)phenyl)-4-methyl-1H-pyrrolo[2,3-b]pyridin-3-yl)-2-methylphenyl)acrylamide,
(S)-N-(5-(2-(4-(3,4-dimethylpiperazin-1-yl)phenyl)-5-(1-isopropyl-1H-pyrazol-4-yl)-4-methyl-1H-pyrrolo[2,3-b]pyridin-3-yl)-2-methylphenyl)acrylamide,
(S)-N-(5-(2-(4-(3,4-dimethylpiperazin-1-yl)phenyl)-4-methyl-5-(1,3,5-trimethyl-1H-pyrazol-4-yl)-1H-pyrrolo[2,3-b]pyridin-3-yl)-2-methylphenyl)acrylamide,
(S)-N-(5-(5-(5-cyano-1-methyl-1H-pyrazol-4-yl)-2-(4-(3,4-dimethylpiperazin-1-yl)phenyl)-4-methyl-1H-pyrrolo[2,3-b]pyridin-3-yl)-2-methylphenyl)acrylamide,
(S)-N-(5-(2-(4-(3,4-dimethylpiperazin-1-yl)phenyl)-4-methyl-5-(1 -methyl-1H-pyrazol-3-yl)-1H-pyrrolo[2,3-b]pyridin-3-yl)-2-methylphenyl)acrylamide,
(S)-N-(5-(2-(4-(3,4-dimethylpiperazin-1-yl)phenyl)-4-methyl-5-(1 -methyl-1H-imidazol-4-yl)-1H-pyrrolo[2,3-b]pyridin-3-yl)-2-methylphenyl)acrylamide,
N-(5-(2-(4-((2-(dimethylamino)ethyl)(methyl)amino)phenyl)-5-(1-methyl-1H-pyrazol-4-yl)-1H-pyrrolo[2,3-b]pyridin-3-yl)-2-methylphenyl)acrylamide,
N-(5-(2-(4-((2-(dimethylamino)ethyl)(methyl)amino)phenyl)-5-(1-methyl-1H-pyrazol-5-yl)-1H-pyrrolo[2,3-b]pyridin-3-yl)-2-methylphenyl)acrylamide,
N-(5-(5-(1,5-dimethyl-1H-pyrazol-4-yl)-2-(4-((2-(dimethylamino)ethyl)(methyl)amino)phenyl)-1H-pyrrolo[2,3-b]pyridin-3-yl)-2-methylphenyl)acrylamide,
N-(5-(2-(4-((2-(dimethylamino)ethyl)(methyl)amino)phenyl)-5-(1-ethyl-1H-pyrazol-4-yl)-1H-pyrrolo[2,3-b]pyridin-3-yl)-2-methylphenyl)acrylamide,
N-(5-(5-(1,3-dimethyl-1H-pyrazol-4-yl)-2-(4-((2-(dimethylamino)ethyl)(methyl)amino)phenyl)-1H-pyrrolo[2,3-b]pyridin-3-yl)-2-methylphenyl)acrylamide,
N-(5-(5-(3-cyano-1-methyl-1H-pyrazol-4-yl)-2-(4-((2-(dimethylamino)ethyl)(methyl)amino)phenyl)-1H-pyrrolo[2,3-b]pyridin-3-yl)-2-methylphenyl)acrylamide,
N-(5-(5-(5-cyano-1-methyl-1H-pyrazol-4-yl)-2-(4-((2-(dimethylamino)ethyl)(methyl)amino)phenyl)-1H-pyrrolo[2,3-b]pyridin-3-yl)-2-methylphenyl)acrylamide,
N-(5-(2-(4-((2-(dimethylamino)ethyl)(methyl)amino)phenyl)-5-(5-ethyl-1-methyl-1 H-pyrazol-4-yl)-1H-pyrrolo[2,3-b]pyridin-3-yl)-2-methylphenyl)acrylamide
or an enantiomer, a mixture of enantiomers, a diastereomer, a mixture of diastereomers, a tautomer, a hydrate, a solvate of the above mentioned compounds, or pharmaceutically acceptable salts thereof.

7. A compound according to any one of claims 1 - 6 as selective inhibitor of Exon 20 mutations of EGFR and Her2.

8. A compound according to any one of claims 1 - 6 for use as a medicament.

9. A compound according to any one of claims 1 - 6 for use in the treatment of cancer or for use in the treatment of cancer, wherein the cancer has an activating mutation of a receptor belonging to an ErbB family of receptors.

10. The compound for use according to claim 9, wherein the activating mutation of the receptor is an insertion within exon 20 of epidermal growth factor receptor (EGFR) or within exon 20 of human epidermal growth factor receptor (HER) or wherein the activating mutation of the receptor is selected from the group consisting of Her2 A775_G776insYVMA, EGFR D770_N771insSVD, EGFR H773_V774insNPH, EGFR V769_D770insASV, EGFR P772_H773insPR, EGFR T790M and EGFR T790ML858R.

11. The compound for use according to claim 9, wherein the cancer is selected from the group consisting of breast cancer, colon cancer, prostate cancer, lung cancer, gastric cancer, ovarian cancer, renal cancer, hepatocellular cancer, thyroid cancer, uterine cancer, esophagus cancer, squamous cell cancer, leukemia, lymphoma, osteosarcoma, melanoma, glioblastoma and neuroblastoma.

12. The compound for use according to any one of claims 9 - 11, wherein the cancer is non-small cell lung cancer or mamma carcinoma.

13. A compound according to claim 1 - 12 in combination with at least one anticancer drug for use in treatment of cancer.

14. A method for producing a compound of the formula (**I**), comprising:
**Step A1:** perfoming a first cross coupling reaction of pyridine compound **1*** with alkyne compound **2a*** 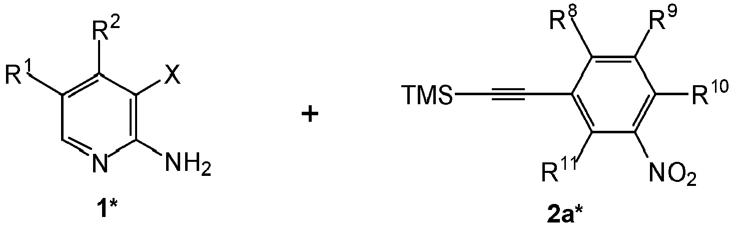 wherein the substituents **R¹**, **R²** and **R⁸** - **R¹¹** have the meanings as defined in claim 1 and X is a leaving group and represents Cl, Br, I, or OTf,
to obtain a compound **3*** 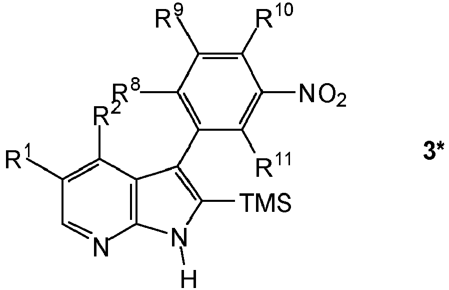 in the presence of a first palladium catalyst, and a first base;
**Step B1:** converting a trimethylsilyl group of the compound **3*** to a halide like an iodide to obtain a compound **4*** 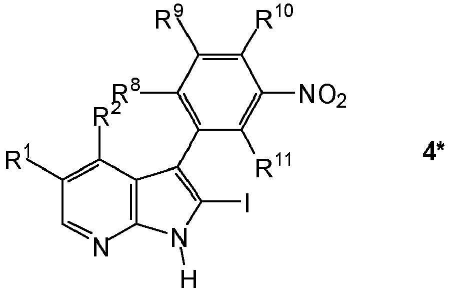
**Step C1:** perfoming a second cross coupling reaction of **4*** with a compound **5*** 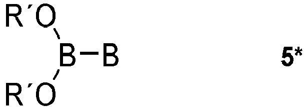 wherein R' is H or an alkyl chain with 1-10 carbon atoms or a cycloalkyl chain with 3 to 12 carbon atoms or both residues R' represent together a residue derived from pinacol,
in the presence of a second palladium catalyst, and a second base to obtain a compound **6*** 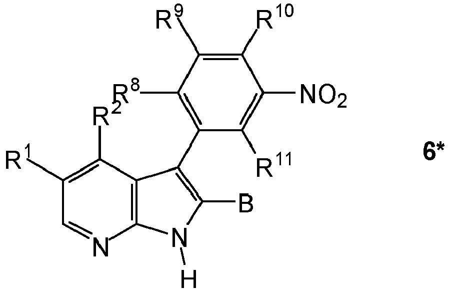
**Step D1:** reducing nitro group of the compound **6*** to a primary amine group to obtain a compound **7***; and
**Step E1:** perfoming a coupling reaction of the compound **7*** 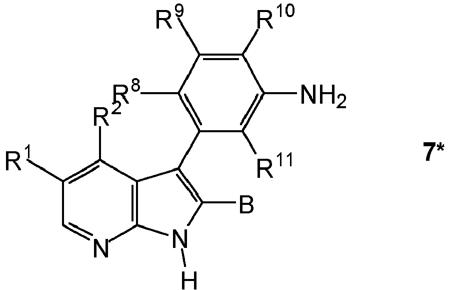 with a compound HO-**R⁶** or AG-**R⁶**, wherein the substituent **R⁶** has the meanings as defined in claim 1 and AG is an activating group of a carboxylic acid,
to obtain a product compound of the general formula (**I**) 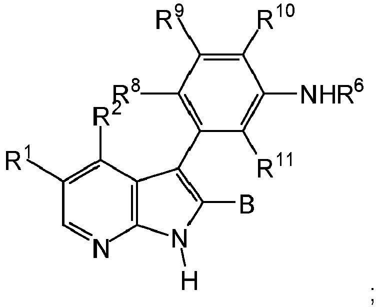
a method for producing a compound of the formula (**I**), comprising:
**Step A1:** perfoming a first cross coupling reaction of pyridine compound **1*** with alkyne compound **2a*** 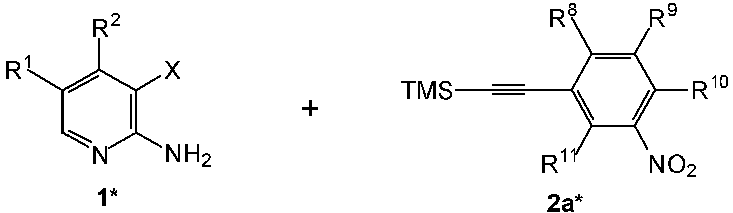 wherein the substituents **R¹**, **R²** and **R⁸** - **R¹¹** have the meanings as defined in claim 1 and X is a leaving group and represents Cl, Br, I, or OTf, to obtain a compound **3*** 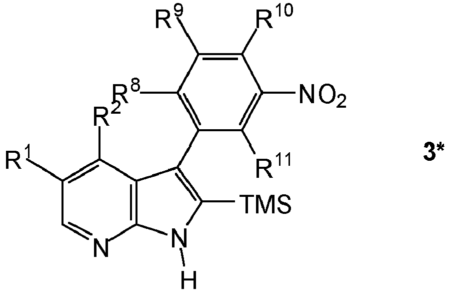 in the presence of a first palladium catalyst, and a first base;
**Step D2:** reducing nitro group of the compound **3*** to a primary amine group to obtain a compound **10*** 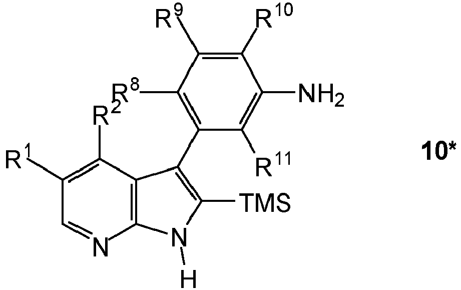
**Step E2:** perfoming a coupling reaction of the compound **10*** with a compound HO-**R⁶** or AG-**R⁶**, wherein the substituent **R⁶** has the meanings as defined in claim 1 and AG is an activating group of a carboxylic acid,
to obtain a compound **11*** 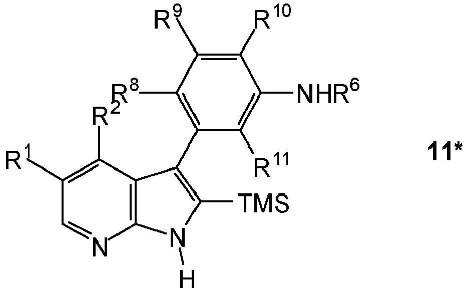
**Step B2:** converting a trimethylsilyl group of the compound **11*** to a halide like an iodide to obtain a compound **12*** 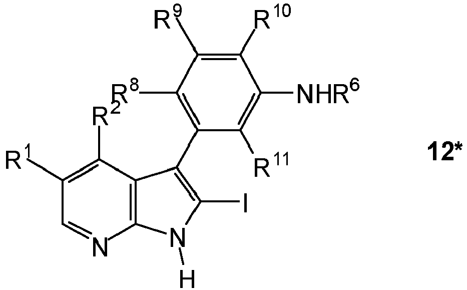
**Step C2:** perfoming a second cross coupling reaction of the compound **12*** with a compound **5*** in the presence of a second palladium catalyst, and a second base to obtain a product compound of the formula (I) 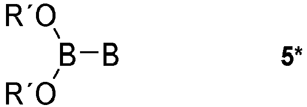 wherein R' is H or an alkyl chain with 1-10 carbon atoms or a cycloalkyl chain with 3 to 12 carbon atoms or both residues R' represent together a residue derived from pinacol, 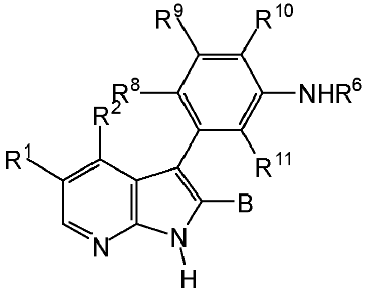
a method for producing a compound of the formula (**I**), comprising:
**Step A3:**
i) perfoming a first cross coupling reaction of pyridine compound **1*** with alkyne compound **2b*** 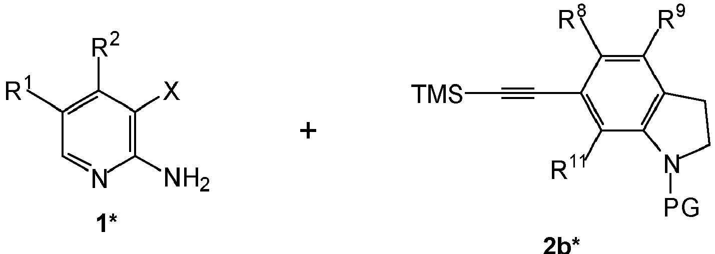 wherein the substituents **R¹**, **R², R⁸, R⁹, R¹¹,** and **R^{a}** - **R^{d}** have the meanings as defined in claim 1 and X is a leaving group and represents Cl, Br, I, or OTf and PG is an amino protecting group,
in the presence of a first palladium catalyst, and a first base; and
ii) removing a protecting group PG of a resulting compound after the step i) to obtain a compound **3b*** 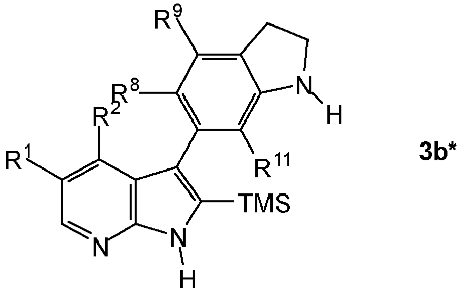
**Step E3:** perfoming a coupling reaction of the compound **3b*** with a compound HO-**R⁶** or AG-**R⁶**, wherein the substituent **R⁶** has the meanings as defined in claim 1 and AG is an activating group of a carboxylic acid,
to obtain a compound **11b*** 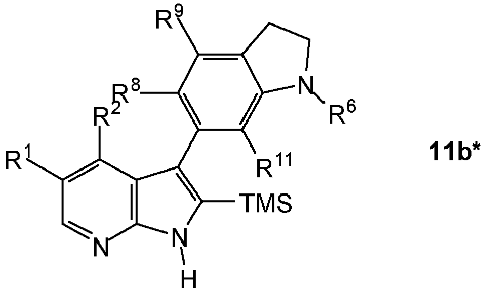
**Step B3:** converting a trimethylsilyl group of the compound **11b*** to a halide like an iodide to obtain a compound **12b*** 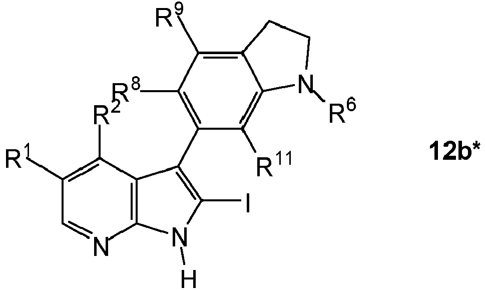
**Step C3:** perfoming a second cross coupling reaction of the compound **12b*** with a compound **5*** 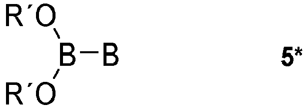 wherein R' is H or an alkyl chain with 1-10 carbon atoms or a cycloalkyl chain with 3 to 12 carbon atoms or both residues R' represent together a residue derived from pinacol,
in the presence of a second palladium catalyst, and a second base to obtain a product compound of the formula (**I**) 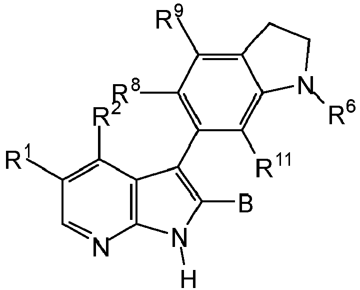
a method for producing a compound of the formula (**I**), comprising:
**Step A4:**
i) perfoming a first cross coupling reaction of pyridine compound **1*** with alkyne compound **2c*** 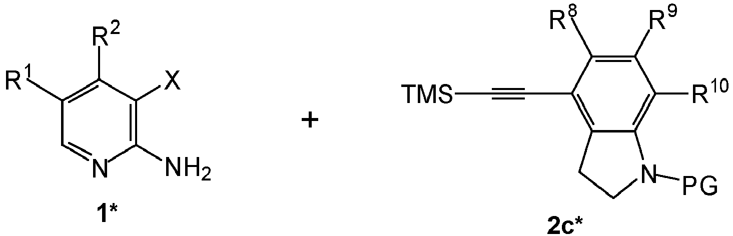 wherein the substituents **R¹**, **R²**, **R⁸**, **R⁹**, **R¹¹**, and **R^{a}** - **R^{d}** have the meanings as defined in claim 1 and X is a leaving group and represents Cl, Br, I, or OTf and PG is an amino protecting group,
in the presence of a first palladium catalyst, and a first base; and
ii) removing a protecting group PG of a resulting compound after the step i) to obtain a compound **3c*** 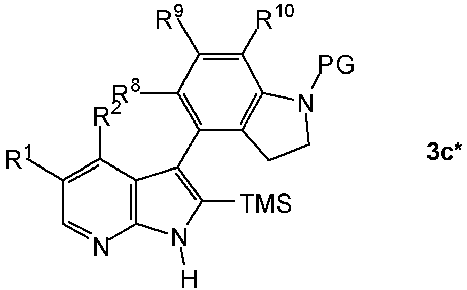
**Step E4:** perfoming a coupling reaction of the compound **3c*** with a compound HO-**R⁶** or AG-**R⁶**, wherein the substituent **R⁶** has the meanings as defined in claim 1 and AG is an activating group of a carboxylic acid,
to obtain a compound **11c*** 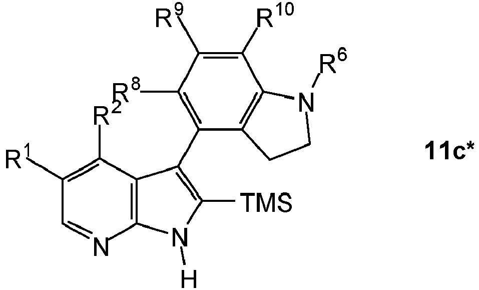
**Step B4:** converting a trimethylsilyl group of the compound **11c*** to a halide like an iodide to obtain a compound **12c*** 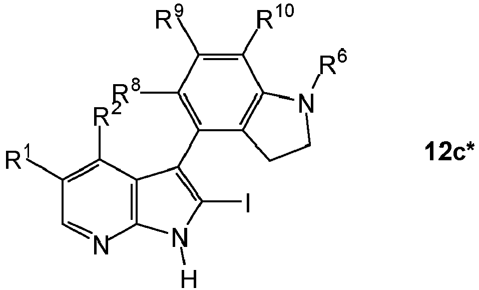
**Step C4:** perfoming a second cross coupling reaction of the compound **12c*** with a compound **5*** 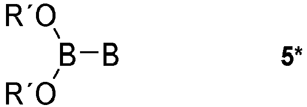 wherein R' is H or an alkyl chain with 1-10 carbon atoms or a cycloalkyl chain with 3 to 12 carbon atoms or both residues R' represent together a residue derived from pinacol,
in the presence of a second palladium catalyst, and a second base to obtain a product compound of the formula (**I**)


SEQUENCE LISTING

<110>  PearlRiver Bio GmbH

<120>  2,3,5-Substituted pyrrolo[2,3-b]pyridines as ErbB modulators useful
for treating cancer

<130>  PRB-P04233EP

<160>  7

<170>  PatentIn version 3.5

<210>  1
<211>  345
<212>  DNA
<213>  Artificial Sequence

<220>
<223>  EGFR p.D770_N771insSVD

<400>  1
ttcaaaaaga tcaaagtgct gggctccggt gcgttcggca cggtgtataa gggactctgg      60

atcccagaag gtgagaaagt taaaattccc gtcgctatca aggaattaag agaagcaaca     120

tctccgaaag ccaacaagga aatcctcgat gaagcctacg tgatggccag cgtggactca     180

gtagacaacc cccacgtgtg ccgcctgctg ggcatctgcc tcacctccac cgtgcagctc     240

atcacgcagc tcatgccctt cggctgcctc ctggactatg tccgggaaca caaagacaat     300

attggctccc agtacctgct caactggtgt gtgcagatcg caaag                     345


<210>  2
<211>  345
<212>  DNA
<213>  Artificial Sequence

<220>
<223>  EGFR p.H773_V774insNPH

<400>  2
ttcaaaaaga tcaaagtgct gggctccggt gcgttcggca cggtgtataa gggactctgg      60

atcccagaag gtgagaaagt taaaattccc gtcgctatca aggaattaag agaagcaaca     120

tctccgaaag ccaacaagga aatcctcgat gaagcctacg tgatggccag cgtggacaac     180

ccccacaatc cacatgtgtg ccgcctgctg ggcatctgcc tcacctccac cgtgcagctc     240

atcacgcagc tcatgccctt cggctgcctc ctggactatg tccgggaaca caaagacaat     300

attggctccc agtacctgct caactggtgt gtgcagatcg caaag                     345


<210>  3
<211>  345
<212>  DNA
<213>  Artificial Sequence

<220>

<223>  EGFR p.V769_D770insASV

<400>  3
ttcaaaaaga tcaaagtgct gggctccggt gcgttcggca cggtgtataa gggactctgg      60

atcccagaag gtgagaaagt taaaattccc gtcgctatca aggaattaag agaagcaaca     120

tctccgaaag ccaacaagga aatcctcgat gaagcctacg tgatggccag cgtggcctca     180

gtcgacaacc cccacgtgtg ccgcctgctg ggcatctgcc tcacctccac cgtgcagctc     240

atcacgcagc tcatgccctt cggctgcctc ctggactatg tccgggaaca caaagacaat     300

attggctccc agtacctgct caactggtgt gtgcagatcg caaag                     345


<210>  4
<211>  342
<212>  DNA
<213>  Artificial Sequence

<220>
<223>  EGFR p.P772_H773insPR

<400>  4
ttcaaaaaga tcaaagtgct gggctccggt gcgttcggca cggtgtataa gggactctgg      60

atcccagaag gtgagaaagt taaaattccc gtcgctatca aggaattaag agaagcaaca     120

tctccgaaag ccaacaagga aatcctcgat gaagcctacg tgatggccag cgtggacaac     180

cccccgcgtc acgtgtgccg cctgctgggc atctgcctca cctccaccgt gcagctcatc     240

acgcagctca tgcccttcgg ctgcctcctg gactatgtcc gggaacacaa agacaatatt     300

ggctcccagt acctgctcaa ctggtgtgtg cagatcgcaa ag                        342


<210>  5
<211>  501
<212>  DNA
<213>  Artificial Sequence

<220>
<223>  HER2 INS8 INS YVMA

<400>  5
acacctagcg gagcgatgcc caaccaggcg cagatgcgga tcctgaaaga gacggagctg      60

aggaaggtga aggtgcttgg atctggcgct tttggcacag tctacaaggg catctggatc     120

cctgatgggg agaatgtgaa aattccagtg gccatcaaag tgttgaggga aaacacatcc     180

cccaaagcca acaaagaaat cttagacgaa gcatacgtga tggcttacgt gatggctggt     240

gtgggctccc catatgtctc ccgccttctg ggcatctgcc tgacatccac ggtgcagctg     300

gtgacacagc ttatgcccta tggctgcctc ttagaccatg tccgggaaaa ccgcggacgc     360

ctgggctccc aggacctgct gaactggtgt atgcagattg ccaaggggat gagctacctg     420

gaggatgtgc ggctcgtaca cagggacttg gccgctcgga acgtgctggt caagagtccc     480

aaccatgtca aaattacaga c 501

<210> 6
<211> 980
<212> DNA
<213> Artificial Sequence

<220>
<223> EGFR T790M

<400> 6
tccaaactgc acctacggat gcactgggcc aggtcttgaa ggctgtccaa cgaatgggcc 60

taagatcccg tccatcgcca ctgggatggt gggggccctc ctcttgctgc tggtggtggc 120

cctggggatc ggcctcttca tgcgaaggcg ccacatcgtt cggaagcgca cgctgcggag 180

gctgctgcag gagagggagc ttgtggagcc tcttacaccc agtggagaag ctcccaacca 240

agctctcttg aggatcttga aggaaactga attcaaaaag atcaaagtgc tgggctccgg 300

tgcgttcggc acggtgtata agggactctg gatcccagaa ggtgagaaag ttaaaattcc 360

cgtcgctatc aaggaattaa gagaagcaac atctccgaaa gccaacaagg aaatcctcga 420

tgaagcctac gtgatggcca gcgtggacaa cccccacgtg tgccgcctgc tgggcatctg 480

cctcacctcc accgtgcagc tcatcatgca gctcatgccc ttcggctgcc tcctggacta 540

tgtccgggaa cacaaagaca atattggctc ccagtacctg ctcaactggt gtgtgcagat 600

cgcaaagggc atgaactact tggaggaccg tcgcttggtg caccgcgacc tggcagccag 660

gaacgtactg gtgaaaacac cgcagcatgt caagatcaca gattttgggc tggccaaact 720

gctgggtgcg gaagagaaag aataccatgc agaaggaggc aaagtgccta tcaagtggat 780

ggcattggaa tcaattttac acagaatcta tacccaccag agtgatgtct ggagctacgg 840

ggtgaccgtt tgggagttga tgacctttgg atccaagcca tatgacggaa tccctgccag 900

cgagatctcc tccatcctgg agaaaggaga acgcctccct cagccaccca tatgtaccat 960

cgatgtctac atgatcatgg 980

<210> 7
<211> 980
<212> DNA
<213> Artificial Sequence

<220>
<223> EGFR T790ML858R

<400> 7
tccaaactgc acctacggat gcactgggcc aggtcttgaa ggctgtccaa cgaatgggcc 60

taagatcccg tccatcgcca ctgggatggt gggggccctc ctcttgctgc tggtggtggc 120

cctggggatc ggcctcttca tgcgaaggcg ccacatcgtt cggaagcgca cgctgcggag 180

gctgctgcag gagagggagc ttgtggagcc tcttacaccc agtggagaag ctcccaacca 240

agctctcttg aggatcttga aggaaactga attcaaaaag atcaaagtgc tgggctccgg 300

tgcgttcggc acggtgtata agggactctg gatcccagaa ggtgagaaag ttaaaattcc 360

cgtcgctatc aaggaattaa gagaagcaac atctccgaaa gccaacaagg aaatcctcga 420

tgaagcctac gtgatggcca gcgtggacaa cccccacgtg tgccgcctgc tgggcatctg 480

cctcacctcc accgtgcagc tcatcatgca gctcatgccc ttcggctgcc tcctggacta 540

tgtccgggaa cacaaagaca atattggctc ccagtacctg ctcaactggt gtgtgcagat 600

cgcaaagggc atgaactact tggaggaccg tcgcttggtg caccgcgacc tggcagccag 660

gaacgtactg gtgaaaacac cgcagcatgt caagatcaca gattttgggc gggccaaact 720

gctgggtgcg gaagagaaag aataccatgc agaaggaggc aaagtgccta tcaagtggat 780

ggcattggaa tcaattttac acagaatcta tacccaccag agtgatgtct ggagctacgg 840

ggtgaccgtt tgggagttga tgacctttgg atccaagcca tatgacggaa tccctgccag 900

cgagatctcc tccatcctgg agaaaggaga acgcctccct cagccaccca tatgtaccat 960

cgatgtctac atgatcatgg 980